(19) Europäisches Patentamt
European Patent Office
Office européen des brevets

(11) EP 4 093 787 B1

(12) **EUROPEAN PATENT SPECIFICATION**

(45) Date of publication and mention of the grant of the patent:
**11.10.2023 Bulletin 2023/41**

(21) Application number: **21701739.1**

(22) Date of filing: **21.01.2021**

(51) International Patent Classification (IPC):
***C08G 18/08*** *(2006.01)* ***C08G 18/10*** *(2006.01)*
***C08G 18/12*** *(2006.01)* ***C08G 18/28*** *(2006.01)*
***C08G 18/30*** *(2006.01)* ***C08G 18/32*** *(2006.01)*
***C08G 18/34*** *(2006.01)* ***C08G 18/48*** *(2006.01)*
***C08G 18/66*** *(2006.01)* ***C08G 18/73*** *(2006.01)*
***C08G 18/75*** *(2006.01)* ***C08G 18/79*** *(2006.01)*
***C09D 175/08*** *(2006.01)* ***C08G 18/02*** *(2006.01)*
***C07D 203/10*** *(2006.01)* ***C08G 85/00*** *(2006.01)*

(52) Cooperative Patent Classification (CPC):
(C-Sets available)
**C07D 203/10; C08F 220/1804; C08G 18/027; C08G 18/0823; C08G 18/0866; C08G 18/12; C08G 18/227; C08G 18/246; C08G 18/282; C08G 18/2825; C08G 18/283; C08G 18/2865; C08G 18/2875; C08G 18/302; C08G 18/3228;**

(Cont.)

(86) International application number:
**PCT/EP2021/051378**

(87) International publication number:
**WO 2021/148558 (29.07.2021 Gazette 2021/30)**

(54) **(AZIRIDINYL HYDROXY)-FUNCTIONAL ORGANIC COMPOUNDS**

(AZIRIDINYL HYDROXY)-FUNKTIONELLE ORGANISCHE VERBINDUNGEN

COMPOSÉS ORGANIQUES À FONCTION AZIRIDINYL HYDROXY

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**22.01.2020 EP 20153159**
**22.01.2020 EP 20153154**
**22.01.2020 EP 20153239**
**22.01.2020 EP 20153240**
**22.01.2020 EP 20153242**
**22.01.2020 EP 20153245**
**22.01.2020 EP 20153246**
**22.01.2020 EP 20153249**
**22.01.2020 EP 20153250**
**22.01.2020 EP 20153251**
**22.01.2020 EP 20153253**
**24.01.2020 EP 20153630**
**24.01.2020 EP 20153628**
**24.07.2020 EP 20187717**

(43) Date of publication of application:
**30.11.2022 Bulletin 2022/48**

(73) Proprietor: **Covestro (Netherlands) B.V.**
**6167 RD Geleen (NL)**

(72) Inventors:
- **OVERBEEK, Gerardus, Cornelis**
  **6100 AA Echt (NL)**
- **STALS, Patrick, Johannes, Maria**
  **6100 AA Echt (NL)**
- **VAN DER ZWAAG, Daan**
  **6100 AA Echt (NL)**
- **BÜCKMANN, Alfred, Jean, Paul**
  **6100 AA Echt (NL)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 093 787 B1

(74) Representative: **Levpat**
**c/o Covestro AG**
**Gebäude K12**
**51365 Leverkusen (DE)**

(56) References cited:
**EP-A1- 1 865 014** **EP-A2- 0 758 662**
**WO-A1-2015/066868** **US-A- 3 329 674**
**US-A1- 2007 298 006**

(52) Cooperative Patent Classification (CPC): (Cont.)
**C08G 18/3231; C08G 18/3275; C08G 18/348; C08G 18/3842; C08G 18/44; C08G 18/4808; C08G 18/4825; C08G 18/4854; C08G 18/4862; C08G 18/6692; C08G 18/6715; C08G 18/73; C08G 18/755; C08G 18/758; C08G 18/765; C08G 18/792; C08G 18/798; C09D 133/02; C09D 175/04; C09D 175/08**

C-Sets
**C08G 18/12, C08G 18/283;**
**C08G 18/12, C08G 18/302;**
**C08G 18/12, C08G 18/3231;**
C08F 220/1804, C08F 220/14, C08F 220/06

## Description

The invention relates to particular (aziridinyl hydroxy)-functional organic component. The invention further relates to liquid compositions comprising said (aziridinyl hydroxy)-functional organic component. The invention further relates to coating compositions comprising said (aziridinyl hydroxy)-functional organic component. The invention further relates to liquid coating compositions comprising said (aziridinyl hydroxy)-functional organic component. The invention further relates to a kit-of-parts comprising in one of its parts a liquid composition comprising a particular (aziridinyl hydroxy)-functional organic component. The invention further relates to cured forms of said liquid compositions, coating compositions, and liquid coating compositions. The invention further relates to articles comprising the (aziridinyl hydroxy)-functional organic component and/or the liquid compositions and/or the liquid coating compositions and/or their cured forms. The invention further relates to various uses of the (aziridinyl hydroxy)-functional organic component and/or said liquid compositions, and/or said liquid coating compositions, and/or said kit-of-parts and/or said cured forms.

Several organic compounds bearing aziridine ring(s) (a 3-membered ring system composed of one nitrogen and two carbon atoms) are known in the art. Aziridines -which are three-membered cyclic amines (azacyclopropanes)- are an example of organic compounds bearing the aziridine ring. The ring-strain associated with these molecules makes them extremely reactive compounds and attractive for various industrial applications. However, several organic compounds bearing aziridine ring(s) exhibit an unfavourable genotoxic profile, that is to mean that several organic compounds bearing aziridine ring(s) exhibit positive induced genotoxicity -as this term is defined and determined in the specification-. For example, the trimethylolpropane tris(2-methyl-1-aziridinepropionate (CAS No.: 64265-57-2; available by DSM as 'Crosslinker CX-100') and the pentaerythritol tris[3-(1-aziridinyl)propionate (CAS No.: 57116-45-7; available by Ichemco as 'XAMA®7'), as well as the pentaerythritol tris (3-(1-aziridinyl) propionate (CAS No.: 57116-45-7, available by LLC Aziridines as PZ-33), are polyfunctional aziridine crosslinkers that are used for crosslinking carboxylic acid functional polymers. However, the trimethylolpropane tris(2-methyl-1-aziridinepropionate) has an unfavourable genotoxic profile since it exhibits positive induced genotoxicity. As a result, the use of organic compounds bearing aziridine ring(s) is restricted given the desire and the need -imposed by various national regulatory regimes- for various industries to improve on the safety, health and environmental profile of their products, e.g. adhesives, inks and coatings, and also of the raw materials , e.g. compounds bearing the aziridine ring, used for preparing said products, e.g. adhesives, inks and coatings.

In genetics, genotoxicity describes the property of chemical or physical agents that cause any type of damage to the DNA within a cell, causing mutations that may for example, lead to cancer. The damage to the DNA may not always lead to a transmittable mutation. Genotoxicity must not be confused with mutagenicity; all mutagenic substances are genotoxic while not all genotoxic substances are mutagenic. Mutagenicity refers to the induction of permanent and transmissible DNA changes (as DNA composition or chromosome structure), which are retained in somatic cell division and passed onto progeny in germ cells.

The US 2007/298006 A1 (equivalent to WO 2006/115547 A2) to Dendritic Nanotechnologies, PLLC disclosed dendritic polymers with enhanced amplification and interior functionality. The example 12 of the US 2007/298006 A1 disclosed a compound bearing an aziridine ring. This compound was the only compound bearing an aziridine ring that was disclosed in the US 2007/298006 A1. The compound of Example 12 of the US 2007/298006 A1 was obtained as a clear colourless oil, had a molecular weight of 588 Da, and it was used as a synthesis intermediate (via the aziridine ring-opening reaction) in the preparation of dendritic polymers. The US 2007/298006 A1 did not disclose (aziridinyl hydroxy)-functional organic compounds having a molecular weight of at least 600 and at most 10000 Da. The US 2007/298006 A1 was silent as to (aziridinyl hydroxy)-functional organic compounds (and its liquid compositions) that would combine no genotoxicity and have good, preferably very good, more preferably excellent, crosslinking efficiency.

The US 3329674 to Thiokol Chemical Corporation disclosed low molecular weight aziridinyl compounds. The molecular weight of these aziridinyl compounds was well below 600 Da (see examples 1-7; the molecular weight of these compounds varied from 304 to 526). The US 3329674 did not disclose (aziridinyl hydroxy)-functional organic compounds having a molecular weight of at least 600 and at most 10000 Da. The US 3329674 was silent as to (aziridinyl hydroxy)-functional organic compounds (and its liquid compositions) that would combine no genotoxicity and have good, preferably very good, more preferably excellent, crosslinking efficiency.

The EP 1865014 A1 to 3M Innovative Properties Company disclosed compositions comprising a prepolymer as component (A) wherein the prepolymer comprised aziridino groups. On page 8-14 (compounds 1-10), the EP 1865014 A1 disclosed compounds bearing aziridine groups. However, these compounds did not have -at least- a hydroxyl group connected to the β-carbon atom as to the nitrogen atom of the aziridine group. Moreover, the compounds 1, 2, 3, 4, 9, 10 had an ester group connected to the aforementioned β-carbon atom while the compounds 5, 6, 7, 8 methyl group connected to the aforementioned β-carbon atom. Thus, the EP 1865014 A1 disclosed very different compounds bearing aziridine groups. The EP 1865014 A1 did not only disclose very different compounds bearing aziridinyl groups but it also did not disclose (aziridinyl hydroxy)-functional organic compounds having a molecular weight of at least 600 and at most 10000 Da. The EP 1865014 A1 was silent as to (aziridinyl hydroxy)-functional organic compounds (and its liquid compositions) that would combine no genotoxicity and have good, preferably very good, more preferably excellent, crosslinking efficiency.

The WO 2015/066868 A1 to 3M Innovative Properties Company disclosed a fluoropolymer coating composition comprising an aqueous liquid medium, fluoropolymer particles dispersed in the aqueous liquid medium, and at least one aziridine compound. However, the aziridine compounds did not have -at least- a hydroxyl group connected to the β-carbon atom as to the nitrogen atom of the aziridine group. Thus, the WO 2015/066868 A1 disclosed very different compounds bearing aziridine groups. Therefore, the WO 2015/066868 A1 did not only disclose very different compounds bearing aziridinyl groups, but it also did not disclose (aziridinyl hydroxy)-functional organic compounds having a molecular weight of at least 600 and at most 10000 Da. The WO 2015/066868 A1 was silent as to (aziridinyl hydroxy)-functional organic compounds (and its liquid compositions) that would combine no genotoxicity and have good, preferably very good, more preferably excellent, crosslinking efficiency.

The EP 0758662 A2 to Rockwell International Corporation disclosed curable resin systems comprising an epoxy resin, a co-reactant selected from N-alkyl and N-aryl substituted aziridines and a catalyst to promote cure at ambient temperature. In its example 3, the EP 0758662 A2 disclosed the reaction of PY 306 epoxy resin with 2-methylaziridine to afford an aziridine-endcapped organic compound as a viscous liquid. According to the EP 0758662 A2, the PY 306 epoxy resin is a bisphenol F epoxy supplied by Ciba Geigy (cf. EP 0758662 A2, from cl. 5, l. 59 to cl. 6, !.1). This aziridine-endcapped organic compound was obtained as a viscous liquid and had a molecular weight of 426.25 Da (this is worked out from the stoichiometry of the reactants disclosed in the 1$^{st}$ sentence of Example 3 of the EP 0758662 A2), and it was used as a synthesis intermediate. The EP 0758662 A2 did not disclose (aziridinyl hydroxy)-functional organic compounds having a molecular weight of at least 600 and at most 10000 Da. The EP 0758662 A2 was silent as to (aziridinyl hydroxy)-functional organic compounds (and its liquid compositions) that would combine no genotoxicity and have good, preferably very good, more preferably excellent, crosslinking efficiency.

In order to enable broader use of organic compounds bearing aziridine ring(s) which are generally attractive for a variety of potential industrial applications without compromising on the safety, health and environmental aspects of processes and products where organic compounds bearing aziridine ring(s) are or may be employed, there is the desire to provide for organic compounds bearing aziridine ring(s) that are not genotoxic and have good, preferably very good, more preferably excellent, crosslinking efficiency.

However, the above desire still represents an unmet need since the solution to such a problem is particularly challenging given their rather unfavourable genotoxic profile and the common prejudice against their use in industrial applications because of their known unfavourable genotoxic profile.

Therefore, it is the object of the invention to provide for a solution to the above-mentioned unmet need. In other words, it is the object of the invention to provide for organic compounds bearing aziridine ring(s) that are not genotoxic and have good, preferably very good, more preferably excellent, crosslinking efficiency.

This object was surprisingly achieved by specific (aziridinyl hydroxy)-functional organic compounds [that form the (aziridinyl hydroxy)-functional organic component(abbreviated as 'AZ-component')] as described in the claims and as disclosed in the specification.

More particularly, the AZ-component (and a liquid composition comprising the AZ-component) are not genotoxic and have good, preferably very good, more preferably excellent, crosslinking efficiency.

The AZ-component (and a liquid composition comprising the AZ-component) constitutes a significant technological advancement for several industries where the use of highly reactive reagents such as compounds bearing aziridine ring(s), is desired or is on-going. The reason being the AZ-component offers concrete opportunities for the industries not only to pursue new synthetic or application pathways for new and innovative products and processes but also to significantly improve on the safety, health and environmental profile of their operations, products and processes.

Broadly in accordance with the invention, there is provided an AZ-component as described in the claims and as disclosed in the specification.

Broadly in accordance with the invention, there are provided liquid composition as described in the claims and as disclosed in the specification.

Broadly in accordance with the invention, there are provided kits-of-parts as described in the claims and as disclosed in the specification.

Broadly in accordance with the invention, there are provided cured forms as described in the claims and as disclosed in the specification.

Broadly in accordance with the invention, there are provided articles as described in the claims and as disclosed in the specification.

Broadly in accordance with the invention, there is provided a use of any one or any combination of the following:

i) an AZ-component according to any one of A1 to A17 and as disclosed in the entire specification including the claims;
ii) a liquid composition according to any one of A18 to A33 and as disclosed in the entire specification including the claims;

as a crosslinker.

Broadly in accordance with the invention, there is provided a use of any one or any combination of the following:

i) an AZ-component according to any one of A1 to A17 and as disclosed in the entire specification including the claims;
ii) a liquid composition according to any one of A18 to A33 and as disclosed in the entire specification including the claims;
iii) a kit-of-parts according to A34 and as disclosed in the entire specification including the claims;
iv) a cured form according to any of A35 to A36 and as disclosed in the entire specification including the claims;
v) an article according to any one of A37 to A38 and as disclosed in the entire specification, including the claims;

in coatings, paints, inks, varnishes, lubricants, adhesives, additive manufacturing, 3D-printing, textiles, waxes, fuels, photography, plastics, medical devices, and in the preparation of medical compositions.

**A1a** Broadly in accordance with the invention there is provided an (aziridinyl hydroxy)-functional organic component (AZ-component) according to the claim 1.

The following paragraphs A1 to A40 constitute certain explicit preferments of the AZ-component according to A1a, as well as certain further explicit aspects of the invention of the AZ-component according to A1a. More specifically, the preferments of the AZ-component according to A1a include but are not limited to preferments A1 to A17, while the aspects of the invention of the AZ-component according to A1a include but are not limited to aspects A18 to A40. Many other variations, combinations or embodiments of the invention will be apparent to those skilled in the art and such variations, combinations and embodiments are contemplated within the scope of the claimed invention. The antecedent basis for certain terms shown in the preferments and in the aspects can be found in preceding preferments or aspects. Any reference to components includes their preferments and preferred ranges as disclosed in the entire specification, including the claims.

**A1** The (aziridinyl hydroxy)-functional organic component (AZ-component) according to A1a or any combination derived from the disclosure in section 1 and the entire specification, wherein the (aziridinyl hydroxy)-functional organic component (AZ-component) is selected from the group consisting of i) to vi): i) (aziridinyl hydroxy)-functional organic compound AZ1 of Formula A1 having only two aziridine rings (AZ1-compound), ii) (aziridinyl hydroxy)-functional organic compound AZ2 of Formula A2 having only three aziridine rings (AZ2-compound), iii) (aziridinyl hydroxy)-functional organic compound AZ3 of Formula A3 having only four aziridine rings (AZ3-compound), iv) (aziridinyl hydroxy)-functional organic compound AZ4 of Formula A4 having only five aziridine rings (AZ4-compound), v) (aziridinyl hydroxy)-functional organic compound AZ5 of Formula A5 having only six aziridine rings (AZ5-compound), and vi) mixtures thereof, preferably the AZ-component is selected from the group consisting of i) to iv): i) (aziridinyl hydroxy)-functional organic compound AZ1 of Formula A1 having only two aziridine rings (AZ1-compound), ii) (aziridinyl hydroxy)-functional organic compound AZ2 of Formula A2 having only three aziridine rings (AZ2-compound), iii) (aziridinyl hydroxy)-functional organic compound AZ3 of Formula A3 having only four aziridine rings (AZ3-compound), and iv) mixtures thereof,

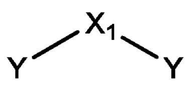

Formula A1

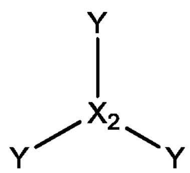

Formula A2

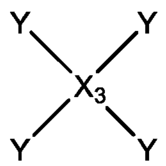

Formula A3

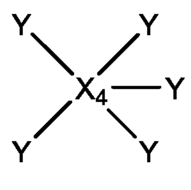

Formula A4

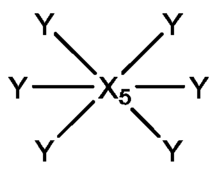

Formula A5

wherein

$X_1$ is a bivalent aliphatic organic radical or a bivalent aromatic organic radical, preferably $X_1$ is a bivalent aliphatic organic radical;

$X_2$ is a trivalent aliphatic organic radical or a trivalent aromatic organic radical, preferably $X_2$ is a trivalent aliphatic organic radical;
$X_3$ is a quadrivalent aliphatic organic radical or a quadrivalent aromatic organic radical, preferably $X_3$ is a quadrivalent aliphatic organic radical;
$X_4$ is a pentavalent aliphatic organic radical or a pentavalent aromatic organic radical, preferably $X_4$ is a pentavalent aliphatic organic radical;
$X_5$ is a hexavalent aliphatic organic radical or a hexavalent aromatic organic radical, preferably $X_5$ is a hexavalent aliphatic organic radical;
and wherein each of the $X_1$ to $X_5$ consists of a collection of atoms covalently connected in a configuration that comprises -preferably consists of- linear and/or branched and/or ring structures, which collection of atoms is selected from the group consisting of i) to x): i) carbon and hydrogen atoms, ii) carbon, hydrogen and oxygen atoms, iii) carbon, hydrogen and nitrogen atoms, iv) carbon, hydrogen and sulphur atoms, v) carbon, hydrogen, oxygen and nitrogen atoms, vi) carbon, hydrogen, nitrogen and sulphur atoms, vii) carbon, hydrogen, oxygen and sulphur atoms, viii) carbon, hydrogen, oxygen, nitrogen and sulphur, atoms, ix) carbon, hydrogen and silicon atoms, and x) carbon, hydrogen, oxygen and silicon atoms and xi) any combination of ix) and/or x) with any one or all of the iii) to viii),
and wherein each of the $X_1$ to $X_5$ has carbon atoms and hydrogen atoms,
and wherein each of the $X_1$ to $X_5$ has optionally oxygen atoms and/or nitrogen atoms and/or sulphur atoms and/or silicon atoms,
and wherein the $X_1$, and/or the $X_2$ and/or the $X_3$ and/or the $X_4$ and/or the $X_5$ may optionally comprise an ionic functional group, preferably the $X_1$, and/or the $X_2$ and/or the $X_3$ and/or the $X_4$ and/or the $X_5$ does not comprise an ionic functional group,
and wherein
Y is a monovalent organic radical selected from the group consisting of: i) monovalent (aziridinyl hydroxyisopropyl) organic radical of Formula B1, ii) monovalent (aziridinyl hydroxycyclohexane) organic radical of Formula B2, and iii) monovalent (aziridinyl hydroxycyclohexane) organic radical of Formula B3, preferably Y is a monovalent (aziridinyl hydroxyisopropyl) organic radical of Formula B1,

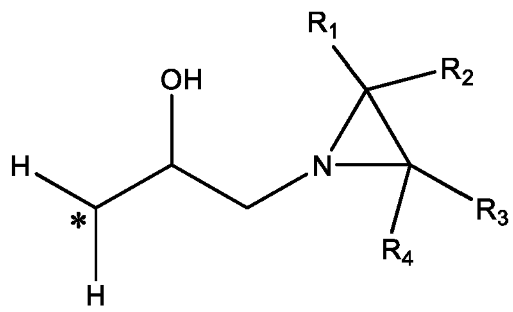

Formula B1

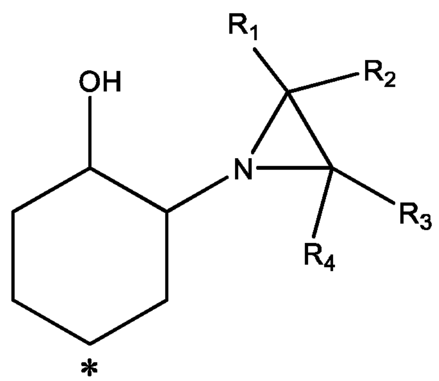

Formula B2

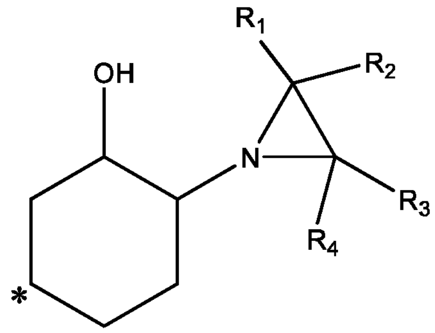

Formula B3

and wherein

$R_1$ is selected from the group consisting of hydrogen and methyl; and

$R_2$ is selected from the group consisting of hydrogen, methyl, and $C_2$-$C_5$ alkyl; and

$R_3$ is selected from the group consisting of methyl, and $C_2$-$C_4$ alkyl; and

$R_4$ is selected from the group consisting of hydrogen, methyl, and $C_2$-$C_4$ alkyl;

and wherein

the Y in each of the compounds A1 to A5 may be the same or different to each other and wherein each of the single covalent bonds between the Y and each one of the $X_1$ to $X_5$ is selected from the group consisting of carbon-carbon single bond and carbon-oxygen single bond preferably each of the single covalent bonds between the Y and each one of the $X_1$ to $X_5$ is carbon-oxygen single bond,

and wherein

each of the AZ1- to AZ5-compounds has a molecular weight determined via MALDI-TOF MS according to the description, of at least 600 and at most 10000, preferably at least 600 and at most 8000, more preferably at least 600 and at most 7000, even more preferably at least 600 and at most 6000, for example at least 600 and at most 5500, for example at least 600 and at most 5000, for example at least 600 and at most 4000, for example at least 600 and at most 3500, for example at least 600 and at most 3200, for example at least 600 and at most 3000, for example at least 600 and at most 2500, for example at least 600 and at most 2200, for example at least 600 and at most 2000, for example at least 630 and at most 10000, for example at least 630 and at most 8000, for example at least 630 and at most 7000, for example at least 630 and at most 6000, for example at least 630 and at most 5500, for example at least 630 and at most 5000, for example at least 630 and at most 4000, for example at least 630 and at most 3500, for example at least 630 and at most 3200, for example at least 630 and at most 3000, for example at least 630 and at most 2500, for example at least 630 and at most 2200, for example at least 630 and at most 2000, for example at least 640 and at most 10000, for example at least 640 and at most 8000, for example at least 640 and at most 7000, for example at least 640 and at most 6000, for example at least 640 and at most 5500, for example at least 640 and at most 5000, for example at least 640 and at most 4000, for example at least 640 and at most 3500, for example at least 640 and at most 3200, for example at least 640 and at most 3000, for example at least 640 and at most 2500, for example at least 640 and at most 2200, for example at least 640 and at most 2000, for example at least 650 and at most 10000, for example at least 650 and at most 8000, for example at least 650 and at most 7000, for example at least 650 and at most 6000, for example at least 650 and at most 5500, for example at least 650 and at most 5000, for example at least 650 and at most 4000, for example at least 650 and at most 3500, for example at least 650 and at most 3200, for example at least 650 and at most 3000, for example at least 650 and at most 2500, for example at least 650 and at most 2200, for example at least 650 and at most 2000, for example at least 700 and at most 10000, for example at least 700 and at most 8000, for example at least 700 and at most 7000, for example at least 700 and at most 6000, for example at least 700 and at most 5500, for example at least 700 and at most 5000, for example at least 700 and at most 4000, for example at least 700 and at most 3500, for example at least 700 and at most 3200, for example at least 700 and at most 3000, for example at least 700 and at most 2500, for example at least 700 and at most 2200, for example at least 700 and at most 2000,for example at least 750 and at most 10000, for example at least 750 and at most 8000, for example at least 750 and at most 7000, for example at least 750 and at most 6000, for example at least 750 and at most 5500, for example at least 750 and at most 5000, for example at least 750 and at most 4000, for example at least 750 and at most 3500, for example at least 750 and at most 3200, for example at least 750 and at most 3000, for example at least 750 and at most 2500, for example at least 750 and at most 2200, for example at least 750 and at most 2000, for example at least 800 and at most 10000, for example at least 800 and at most 8000, for example

at least 800 and at most 7000, for example at least 800 and at most 6000, for example at least 800 and at most 5500, for example at least 800 and at most 5000, for example at least 800 and at most 4000, for example at least 800 and at most 3500, for example at least 800 and at most 3200, for example at least 800 and at most 3000, for example at least 800 and at most 2500, for example at least 800 and at most 2200, for example at least 800 and at most 2000, for example at least 850 and at most 10000, for example at least 850 and at most 8000, for example at least 850 and at most 7000, for example at least 850 and at most 6000, for example at least 850 and at most 5500, for example at least 850 and at most 5000, for example at least 850 and at most 4000, for example at least 850 and at most 3500, for example at least 850 and at most 3200, for example at least 850 and at most 3000, for example at least 850 and at most 2500, for example at least 850 and at most 2200, for example at least 850 and at most 2000, for example at least 900 and at most 10000, for example at least 900 and at most 8000, for example at least 900 and at most 7000, for example at least 900 and at most 6000, for example at least 900 and at most 5500, for example at least 900 and at most 5000, for example at least 900 and at most 4000, for example at least 900 and at most 3500, for example at least 900 and at most 3200, for example at least 900 and at most 3000, for example at least 900 and at most 2500, for example at least 900 and at most 2200, for example at least 900 and at most 2000, for example at least 950 and at most 10000, for example at least 950 and at most 8000, for example at least 950 and at most 7000, for example at least 950 and at most 6000, for example at least 950 and at most 5500, for example at least 950 and at most 5000, for example at least 950 and at most 4000, for example at least 950 and at most 3500, for example at least 950 and at most 3200, for example at least 950 and at most 3000, for example at least 950 and at most 2500, for example at least 950 and at most 2200, for example at least 950 and at most 2000, for example at least 1000 and at most 10000, for example at least 1000 and at most 8000, for example at least 1000 and at most 7000, for example at least 1000 and at most 6000, for example at least 1000 and at most 5500, for example at least 1000 and at most 5000, for example at least 1000 and at most 4000, for example at least 1000 and at most 3500, for example at least 1000 and at most 3200, for example at least 1000 and at most 3000, for example at least 1000 and at most 2500, for example at least 1000 and at most 2200, for example at least 1000 and at most 2000, for example at least 1100 and at most 10000, for example at least 1100 and at most 8000, for example at least 1100 and at most 7000, for example at least 1100 and at most 6000, for example at least 1100 and at most 5500, for example at least 1100 and at most 5000, for example at least 1100 and at most 4000, for example at least 1100 and at most 3500, for example at least 1100 and at most 3200, for example at least 1100 and at most 3000, for example at least 1100 and at most 2500, for example at least 1100 and at most 2200, for example at least 1100 and at most 2000, for example at least 1200 and at most 10000, for example at least 1200 and at most 8000, for example at least 1200 and at most 7000, for example at least 1200 and at most 6000, for example at least 1200 and at most 5500, for example at least 1200 and at most 5000, for example at least 1200 and at most 4000, for example at least 1200 and at most 3500, for example at least 1200 and at most 3200, for example at least 1200 and at most 3000, for example at least 1200 and at most 2500, for example at least 1200 and at most 2200, for example at least 1200 and at most 2000 Da,
and wherein
the $X_1$ and the $X_2$ and the $X_3$ and the $X_4$ and the $X_5$ does not contain one or any combination of the following structural units BP1, BP2, BP3, and BS

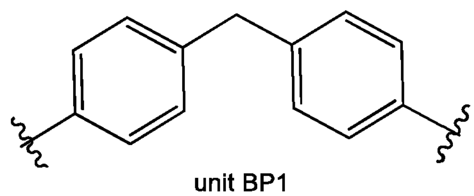

unit BP1

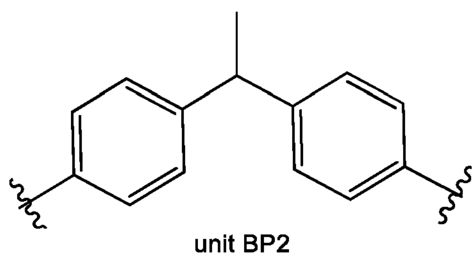

unit BP2

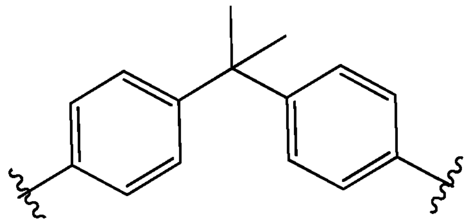

unit BP3

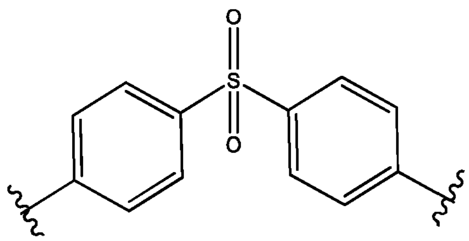

**unit BS**

**A2** The AZ-component according to A1a or A1 or any combination derived from the disclosure in section 1 and the entire specification, wherein the $X_1$ is a bivalent aliphatic organic radical or a bivalent aromatic organic radical other than a bivalent organic radical selected from the group consisting of bivalent radical of bisphenol A, bivalent radical of bisphenol AP, bivalent radical of bisphenol AF, bivalent radical of bisphenol B, bivalent radical of bisphenol BP, bivalent radical of bisphenol C, bivalent radical of bisphenol C2, bivalent radical of bisphenol E, bivalent radical of bisphenol F, bivalent radical of bisphenol G, bivalent radical of bisphenol M, bivalent radical of bisphenol S, bivalent radical of bisphenol P, bivalent radical of bisphenol PH, bivalent radical of bisphenol TMC, bivalent radical of bisphenol Z, bivalent radical of dinitrobisphenol A, bivalent radical of tetrabromobisphenol A, and combinations thereof, preferably $X_1$ is a bivalent aliphatic organic radical.

**A3** The AZ-component according to A1a or to any one of A1 to A2 or any combination derived from the disclosure in section 1 and the entire specification, wherein the $X_1$, and/or the $X_2$ and/or the $X_3$ and/or the $X_4$ and/or the $X_5$ comprises at least one structural unit or a combination of structural units selected from the group consisting of unit 1, unit 2, unit 3, unit 4, unit 5, unit 6, unit 7, unit 8, unit 9, unit 10, and unit 11, preferably comprises at least one structural unit or a combination of structural units selected from the group consisting of unit 1, unit 4, unit 9, unit 10 and unit 11, as the units 1 to 11 are depicted below:

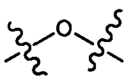

**unit 1**

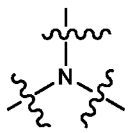

**unit 2**

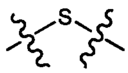

unit 3

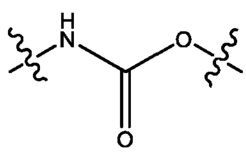

unit 4

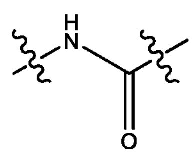

unit 5

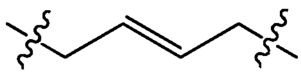

unit 6

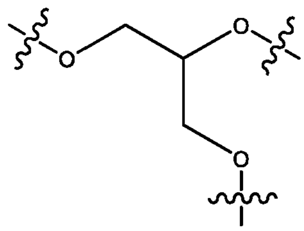

unit 7

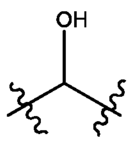

unit 8

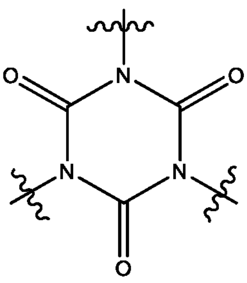

unit 9

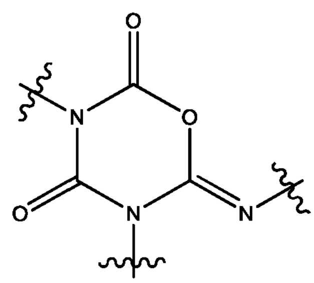

unit 10

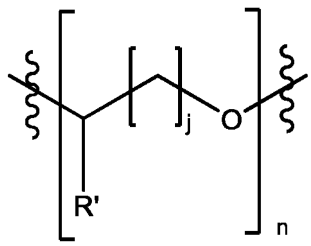

unit 11

wherein

R' is selected from the group consisting of hydrogen and methyl; and
j is an integer ranging from 1 to 5, preferably from 1 to 3; and
n is an integer ranging from and including 2 up to and including 50, preferably from and including 2 up to and including 40, more preferably from and including 2 up to and including 30, most preferably from and including 2 up to and including 20, for example from and including 2 up to and including 15, for example from and including 2 up to and including 10, for example from and including 2 up to and including 5, for example the n is 2, for example from and including 3 up to and including 50, for example from and including 3 up to and including 40, for example from and including 3 up to and including 30, for example from and including 3 up to and including 20, for example from and including 3 up to and including 15, for example from and including 3 up to and including 10, for example from and including 3 up to and including 5,for example the n is 3, for example from and including 4 up to and including 50, for example from and including 4 up to and including 40, for example from and including 4 up to and including 30, for example from and including 4 up to and including 20, for example from and including 4 up to and including 15, for example from and including 4 up to and including 10, for example from and including 4 up to and including 5, for example the n is 4, for example from and including 5 up to and including 50, for example from and including 5 up to and including 40, for example from and including 5 up to and including 30, for example from and including 5 up to and including 20, for example from and including 5 up to and including 15, for example from and including 5 up to and including 10, for example the n is 5, for example from and including 6 up to and including 50, for example from and including 6 up to and including 40, for example from and including 6 up to and including 30, for example from and including 6 up to and including 20, for example from and including 6 up to and including 15, for example from and including 6 up to and including 10, for example the n is 6, for example from and including 7 up to and including 50, for example from and including 7 up to and including 40, for example from and including 7 up to and including 30, for example from and including 7 up to and including 20, for example from and including 7 up to and including 15, for example from and including 7 up to and including 10, for example the n is 7, for example from and including 8 up to and including 50, for example from and including 8 up to and including 40, for example from and including 8 up to and including 30, for example from and including 8 up to and including 20, for example from and including 8 up to and including 15, for example from and including 8 up to and including 10, for example the n is 8, for example from and including 9 up to and including 50, for example from and including 9 up to and including 40, for example from and including 9 up to and including 30, for example from and including 9 up to and including 20, for example from and including 9 up to and including 15, for example from and including 9 up to and including 10, for example the n is 9, for example from and including 10 up to and including 50, for example from and including 10 up to and including 40, for example from and including 10 up to and including 30, for example from and including 10 up to and including 20, for example from and including 10 up to and including 15, for example the n is 10.

**A4** The AZ-component according to A1a or to any one of A1 to A3 or any combination derived from the disclosure in section 1 and the entire specification, wherein

$R_1$ is selected from the group consisting of hydrogen and methyl; and
$R_2$ is selected from the group consisting of hydrogen, methyl, and ethyl; and
$R_3$ is selected from the group consisting of methyl, and $C_2$-$C_4$ alkyl; and
$R_4$ is selected from the group consisting of hydrogen, methyl, and ethyl.

**A5** The AZ-component according to A1a or to any one of A1 to A4 or any combination derived from the disclosure in section 1 and the entire specification, wherein the aggregate number of carbon atoms in $R_1$, and $R_2$ and $R_3$ and $R_4$ is at most 9, preferably at most 4, more preferably at most 2, for example at most 1.

**A6** The AZ-component according to A1a or to any one of A1 to A5 or any combination derived from the disclosure in section 1 and the entire specification, wherein the $X_1$, and/or the $X_2$ and/or the $X_3$ and/or the $X_4$ and/or the $X_5$ comprises at least one unit 11 as a structural unit.

**A7** The AZ-component according to A1a or to any one of A1 to A6 or any combination derived from the disclosure in section 1 and the entire specification, wherein the $X_1$ is the bivalent aliphatic organic radical of Formula A1a'

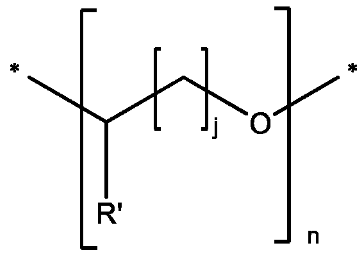

Formula A1a'

wherein

R' is selected from the group consisting of hydrogen and methyl; and
j is an integer ranging from 1 to 5, preferably from 1 to 3; and
n is an integer ranging from and including 2 up to and including 50, preferably from and including 2 up to and including 40, more preferably from and including 2 up to and including 30, most preferably from and including 2 up to and including 20, for example from and including 2 up to and including 15, for example from and including 2 up to and including 10, for example from and including 2 up to and including 5, for example the n is 2, for example from and including 3 up to and including 50, for example from and including 3 up to and including 40, for example from and including 3 up to and including 30, for example from and including 3 up to and including 20, for example from and including 3 up to and including 15, for example from and including 3 up to and including 10, for example from and including 3 up to and including 5, for example the n is 3, for example from and including 4 up to and including 50, for example from and including 4 up to and including 40, for example from and including 4 up to and including 30, for example from and including 4 up to and including 20, for example from and including 4 up to and including 15, for example from and including 4 up to and including 10, for example from and including 4 up to and including 5, for example the n is 4, for example from and including 5 up to and including 50, for example from and including 5 up to and including 40, for example from and including 5 up to and including 30, for example from and including 5 up to and including 20, for example from and including 5 up to and including 15, for example from and including 5 up to and including 10, for example the n is 5, for example from and including 6 up to and including 50, for example from and including 6 up to and including 40, for example from and including 6 up to and including 30, for example from and including 6 up to and including 20, for example from and including 6 up to and including 15, for example from and including 6 up to and including 10, for example the n is 6, for example from and including 7 up to and including 50, for example from and including 7 up to and including 40, for example from and including 7 up to and including 30, for example from and including 7 up to and including 20, for example from and including 7 up to and including 15, for example from and including 7 up to and including 10, for example the n is 7, for example from and including 8 up to and including 50, for example from and including 8 up to and including 40, for example from and including 8 up to and including 30, for example from and including 8 up to and including 20, for example from and including 8 up to and including 15, for example from and including 8 up to and including 10, for example the n is 8, for example from and including 9 up to and including 50, for example from and including 9 up to and including 40,

for example from and including 9 up to and including 30, for example from and including 9 up to and including 20, for example from and including 9 up to and including 15, for example from and including 9 up to and including 10, for example the n is 9, for example from and including 10 up to and including 50, for example from and including 10 up to and including 40, for example from and including 10 up to and including 30, for example from and including 10 up to and including 20, for example from and including 10 up to and including 15, for example the n is 10.

**A8** The AZ-component according to A1a or to any one of A1 to A7 or any combination derived from the disclosure in section 1 and the entire specification, wherein each of the $X_1$ to $X_5$ contains only single covalent bonds, or both single and double covalent bonds, and wherein the single covalent bonds are selected from the group consisting of carbon-carbon single bond, carbon-hydrogen single bond, carbon-nitrogen single bond, carbon-sulphur single bond, carbon-silicon single bond, silicon-oxygen single bond, nitrogen-hydrogen single bond, sulphur-oxygen single bond, carbon-oxygen single bond wherein the oxygen is bonded to a hydrogen forming a hydroxyl group, silicon-oxygen-silicon single bonds, and wherein the double covalent bonds are selected from the group consisting of carbon-carbon double bond, carbon-nitrogen double bond, sulphur-oxygen double bond, carbon-oxygen double bond wherein the carbon is a member of a ring structure, and carbon-oxygen double bond wherein the carbon is bonded to another two carbons via carbon-carbon single bonds, carbon-oxygen double bond wherein the carbon is bonded to another oxygen via single bond and to a nitrogen via a single bond, carbon-oxygen double bond wherein the carbon is bonded to two nitrogens via carbon-nitrogen single bonds, carbon-oxygen double bond wherein the carbon is bonded to another two oxygens via single bonds; preferably the double covalent bonds are selected from the group consisting of carbon-carbon double bond, carbon-nitrogen double bond, sulphur-oxygen double bond, carbon-oxygen double bond wherein the carbon is a member of a ring structure, and carbon-oxygen double bond wherein the carbon is bonded to another two carbons via carbon-carbon single bonds, carbon-oxygen double bond wherein the carbon is bonded to another oxygen via single bond and to a nitrogen via a single bond, carbon-oxygen double bond wherein the carbon is bonded to two nitrogens via carbon-nitrogen single bonds.

**A9** The AZ-component according to A1a or to any one of A1 to A8 or any combination derived from the disclosure in section 1 and the entire specification, wherein the aggregate number of carbon and hydrogen atoms in $X_1$ and/or $X_2$ and/or $X_3$ and/or $X_4$ and/or $X_5$ is at least 5, preferably at least 10, more preferably at least 15, most preferably at least 20, for example at least 25, for example at least 30, for example at least 35, for example at least 40, for example at least 45, for example at least 50, for example at least 55, for example at least 60, for example at least 65, for example at least 70, for example at least 75, for example at least 80, for example at least 85, for example at least 90, for example at least 95, for example at least 97, for example at least 98, for example at least 99, for example 100 % as to the aggregate number of all the atoms present in $X_1$ and/or $X_2$ and/or $X_3$ and/or $X_4$ and/or $X_5$, respectively.

**A10** The AZ-component according to A1a or to any one of A1 to A9 or any combination derived from the disclosure in section 1 and the entire specification, wherein the aggregate number of all the atoms present in $X_1$ and/or $X_2$ and/or $X_3$ and/or $X_4$ and/or $X_5$ is at most 600, preferably at most 550, more preferably at most 500, most preferably at most 450, for examples at most 400, for example at most 350, for example at most 300, for example at most 250, for example at most 200.

**A11** The AZ-component according to A1a or to any one of A1 to A6 and A8 to A10 or any combination derived from the disclosure in section 1 and the entire specification, wherein the AZ-component is selected from the group consisting of i) to v): i) (aziridinyl hydroxy)-functional organic compound AZ1 of Formula A1 (AZ1-compound), ii) (aziridinyl hydroxy)-functional organic compound AZ2 of Formula A2 (AZ2-compound), iii) (aziridinyl hydroxy)-functional organic compound AZ3 of Formula A3 (AZ3-compound), iv) (aziridinyl hydroxy)-functional organic compound AZ5 of Formula A5 (AZ5-compound), and vi) mixtures thereof, and
wherein

the AZ1-compound is selected from the group consisting of compounds having the Formula A1a, and compounds having the Formula A1b, as each of these Formulae A1a-A1d is described below

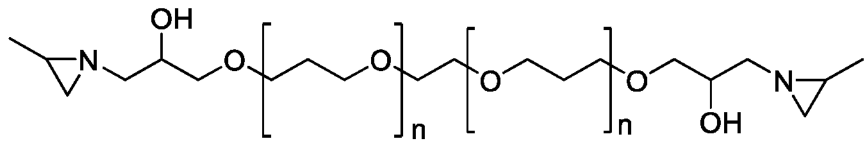

Formula A1a

wherein each of the n in Formula A1a is independently selected, and each of the n in Formula A1a is an integer

ranging from and including 2 up to and including 20, preferably from and including 2 up to and including 10, more preferably from and including 2 up to and including 7, most preferably from and including 2 up to and including 5, for example from and including 2 up to and including 4, for example from and including 3 up to and including 20, for example from and including 3 up to and including 10, for example from and including 3 up to and including 7, for example from and including 3 up to and including 5, for example from and including 3 up to and including 4, for example n is equal to 4; and

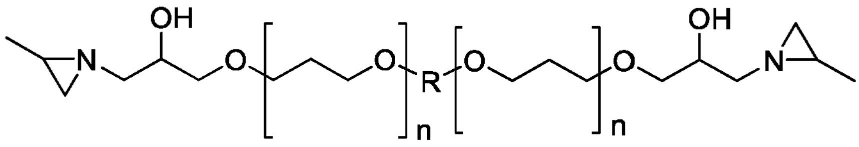

Formula A1b

wherein the R in Formula A1b is a $C_3$-$C_{10}$ saturated hydrocarbylene, and wherein each of the n in Formula A1b is independently selected and each of the n in Formula A1a is an integer ranging from and including 2 up to and including 20, preferably from and including 2 up to and including 10, more preferably from and including 2 up to and including 7, most preferably from and including 2 up to and including 5, for example from and including 2 up to and including 4, for example from and including 3 up to and including 20, for example from and including 3 up to and including 10, for example from and including 3 up to and including 7, for example from and including 3 up to and including 5, for example from and including 3 up to and including 4, for example n is equal to 4; and

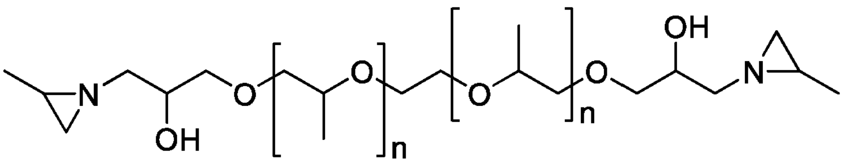

Formula A1c

wherein each of the n in Formula A1c is independently selected, and each of the n in Formula A1b is an integer ranging from and including 2 up to and including 20, preferably from and including 2 up to and including 10, more preferably from and including 2 up to and including 7, most preferably from and including 2 up to and including 5, for example from and including 2 up to and including 4, for example from and including 3 up to and including 20, for example from and including 3 up to and including 10, for example from and including 3 up to and including 7, for example from and including 3 up to and including 5, for example from and including 3 up to and including 4, for example n is equal to 4, and

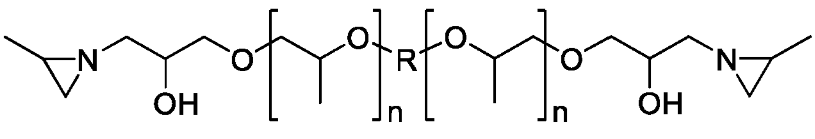

Formula A1d

wherein the R in Formula A1d is a $C_3$-$C_{10}$ saturated hydrocarbylene, and wherein each of the n in Formula A1d is independently selected and each of the n in Formula A1b is an integer ranging from and including 2 up to and including 20, preferably from and including 2 up to and including 10, more preferably from and including 2 up to and including 7, most preferably from and including 2 up to and including 5, for example from and including 2 up to and including 4, for example from and including 3 up to and including 20, for example from and including 3 up to and including 10, for example from and including 3 up to and including 7, for example from and including 3 up to and including 5, for example from and including 3 up to and including 4, for example n is equal to 4, and

wherein

the AZ2-compound is selected from the group consisting of compounds having the Formula A2a, compounds having the Formula A2b, compounds having the Formula A2c, compounds having the Formula A2d, compounds having the Formula A2e, as each of these Formulae A2a-A2e is described below

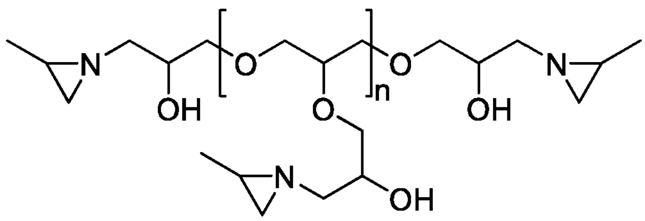

Formula A2a

wherein the n in Formula A2a is an integer ranging from and including 2 up to and including 20, for example from and including 2 up to and including 10, for example from and including 2 up to and including 8, for example from and including 3 up to and including 20, for example from and including 3 up to and including 10, for example from and including 3 up to and including 8, for example from and including 4 up to and including 20, for example from and including 4 up to and including 10, for example from and including 4 up to and including 8, for example from and including 5 up to and including 20, for example from and including 5 up to and including 10, for example from and including 5 up to and including 8, for example from and including 6 up to and including 20, for example from and including 6 up to and including 10, for example from and including 6 up to and including 8, for example n is equal to 7;
and

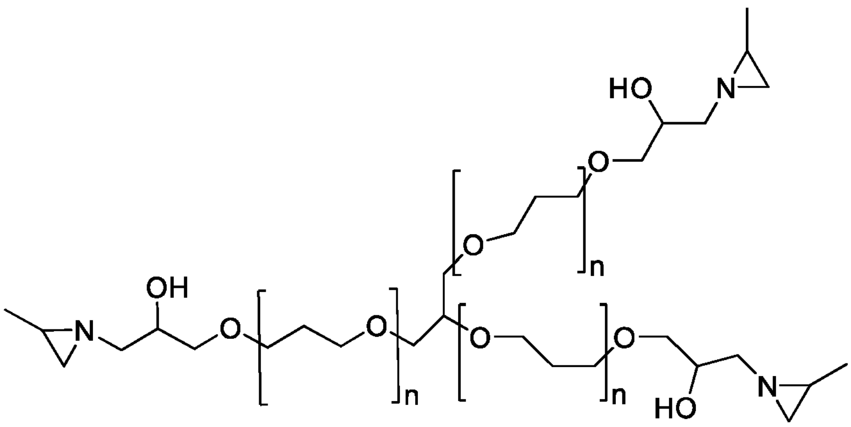

Formula A2b

wherein each of the n in Formula A2b is independently selected and each of the n in Formula A2b is an integer ranging from and including 2 up to and including 20, preferably from and including 2 up to and including 15, more preferably from and including 2 up to and including 12, most preferably from and including 2 up to and including 11, for example from and including 3 up to and including 20, for example from and including 3 up to and including 15 for example from and including 3 up to and including 12 for example from and including 3 up to and including 11, for example from and including 4 up to and including 20, for example from and including 4 up to and including 15 for example from and including 4 up to and including 12 for example from and including 4 up to and including 11, for example from and including 5 up to and including 20, for example from and including 5 up to and including 15 for example from and including 5 up to and including 12 for example from and including 5 up to and including 11, for example from and including 6 up to and including 20, for example from and including 6 up to and including 15 for example from and including 6 up to and including 12 for example from and including 6 up to and including 11, for example from and including 7 up to and including 20, for example from and including 7 up to and including 15 for example from and including 7 up to and including 12 for example from and including 7 up to and including 11,

for example from and including 8 up to and including 20, for example from and including 8 up to and including 15 for example from and including 8 up to and including 12 for example from and including 8 up to and including 11, for example from and including 9 up to and including 20, for example from and including 9 up to and including 15 for example from and including 9 up to and including 12 for example from and including 9 up to and including 11, for example from and including 10 up to and including 20, for example from and including 10 up to and including 15 for example from and including 10 up to and including 12 for example from and including 10 up to and including 11, for example n is equal to 11;
and

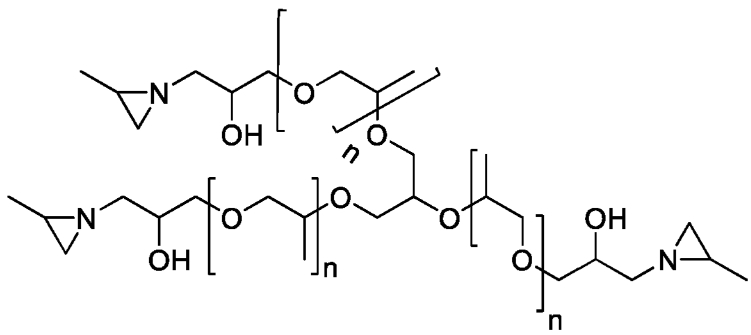

Formula A2c

wherein each of the n in Formula A2c is independently selected and each of the n in Formula A2c is an integer ranging from and including 2 up to and including 20, preferably from and including 2 up to and including 15, more preferably from and including 2 up to and including 12, most preferably from and including 2 up to and including 11, for example from and including 3 up to and including 20, for example from and including 3 up to and including 15 for example from and including 3 up to and including 12 for example from and including 3 up to and including 11, for example from and including 4 up to and including 20, for example from and including 4 up to and including 15 for example from and including 4 up to and including 12 for example from and including 4 up to and including 11, for example from and including 5 up to and including 20, for example from and including 5 up to and including 15 for example from and including 5 up to and including 12 for example from and including 5 up to and including 11, for example from and including 6 up to and including 20, for example from and including 6 up to and including 15 for example from and including 6 up to and including 12 for example from and including 6 up to and including 11, for example from and including 7 up to and including 20, for example from and including 7 up to and including 15 for example from and including 7 up to and including 12 for example from and including 7 up to and including 11, for example from and including 8 up to and including 20, for example from and including 8 up to and including 15 for example from and including 8 up to and including 12 for example from and including 8 up to and including 11, for example from and including 9 up to and including 20, for example from and including 9 up to and including 15 for example from and including 9 up to and including 12 for example from and including 9 up to and including 11, for example from and including 10 up to and including 20, for example from and including 10 up to and including 15 for example from and including 10 up to and including 12 for example from and including 10 up to and including 11, for example n is equal to 11;
and

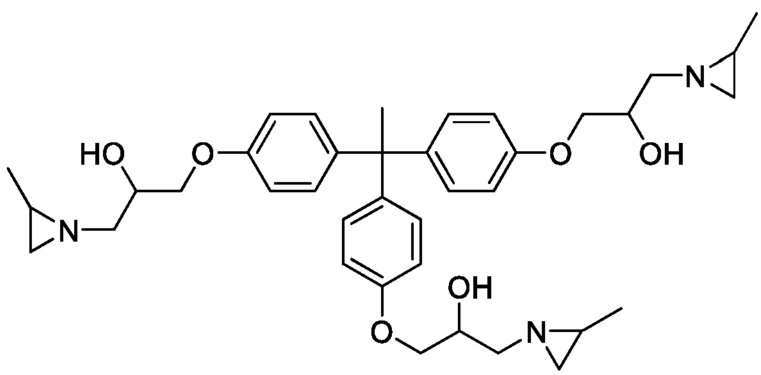

Formula A2d

and

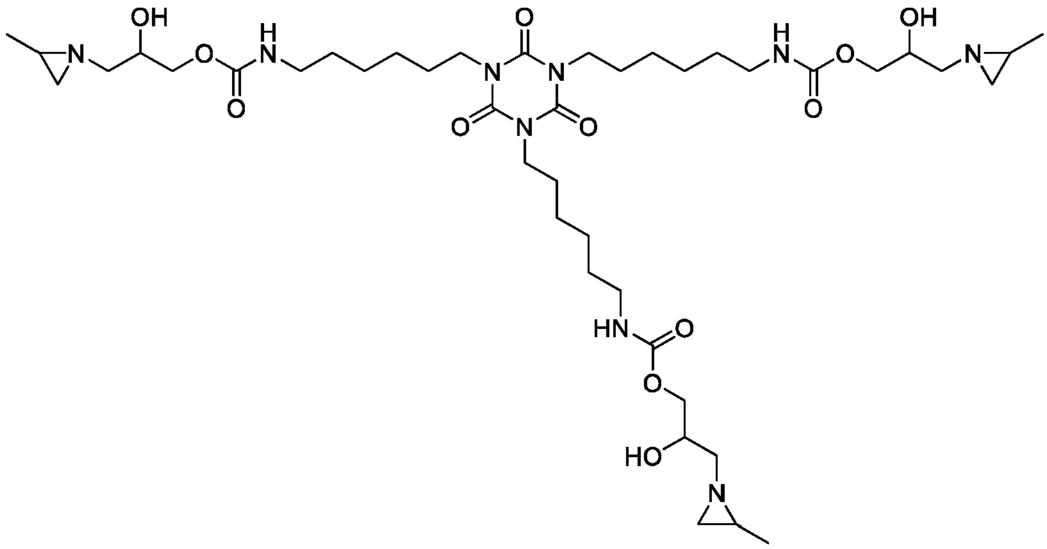

Formula A2e

and

wherein

the AZ3-compound is selected from the group consisting of compounds having the Formula A3a

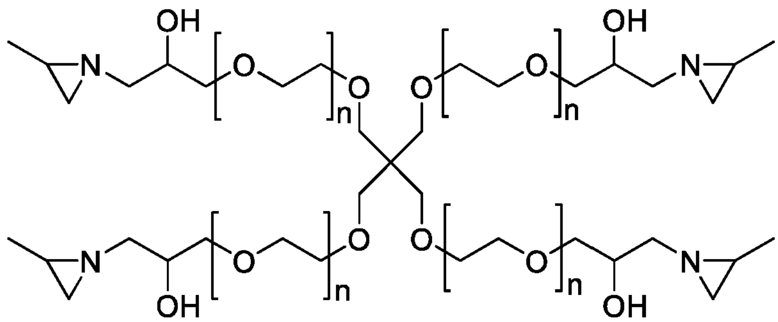

Formula A3a

wherein each of the n in Formula A3a is independently selected, and each of the n in Formula A3a is an integer ranging from and including 2 up to and including 20, preferably from and including 2 up to and including 15, more preferably from and including 2 up to and including 10, most preferably from and including 2 up to and including 9, for example from and including 2 up to and including 8, for example from and including 2 up to and including 7, for example from and including 2 up to and including 6, for example from and including 2 up to and including 5, for example from and including 2 up to and including 4, for example from and including 2 up to and including 3, for example n is equal to 3, for example n is equal to 2;
and

wherein

the AZ5-compound is selected from the group consisting of compounds having the Formula A5a

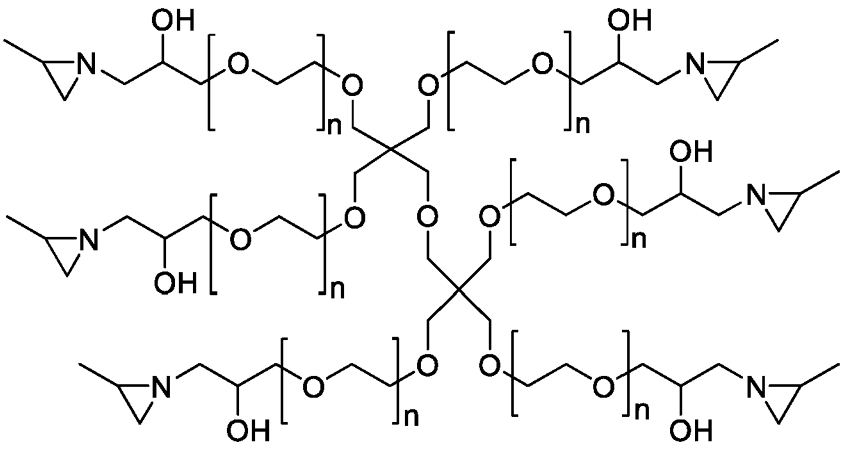

Formula A5a

wherein each of the n in Formula A5a is independently selected, and each of the n in Formula A5a is an integer ranging from and including 2 up to and including 40, preferably from and including 2 up to and including 30, more preferably from and including 2 up to and including 20, most preferably from and including 2 up to and including 10, for example from and including 2 up to and including 9, for example from and including 2 up to and including 8, for example from and including 2 up to and including 7, for example from and including 2 up to and including 6, for example from and including 2 up to and including 5, for example from and including 2 up to and including 4, for example from and including 3 up to and including 40, for example from and including 3 up to and including 30, for example from and including 3 up to and including 20, for example from and including 3 up to and including 10, for example from and including 3 up to and including 9, for example from and including 3 up to and including 8, for example from and including 3 up to and including 7, for example from and including 3 up to and including 6, for example from and including 3 up to and including 5, for example from and including 3 up to and including 4, for example n is equal to 4, for example n is equal to 3.

**A12** The AZ-component according to A1a or to any one of A1 to A11 or any combination derived from the disclosure in section 1 and the entire specification, wherein the $X_1$ and/or the $X_2$ and/or the $X_3$ and/or the $X_4$ and/or the $X_5$ does not contain any structural unit of (or equally derived from) phenol formaldehyde resins (also known as phenolic resins); examples of phenol-formaldehyde (PF) resins include novolacs (acid-catalyzed PF resins with a formaldehyde to phenol ratio of equal to or lower than one), and resols (base-catalyzed PF resins with a formaldehyde to phenol ratio of greater than one, usually equal to 1.5).

**A13** The AZ-component according to A1a or to any one of A1 to A6 and A8 to A10 or any combination derived from the disclosure in section 1 and the entire specification, wherein the AZ-component is selected from the group consisting of AZ1-compound and wherein the AZ1-compound is the compound of the following formula

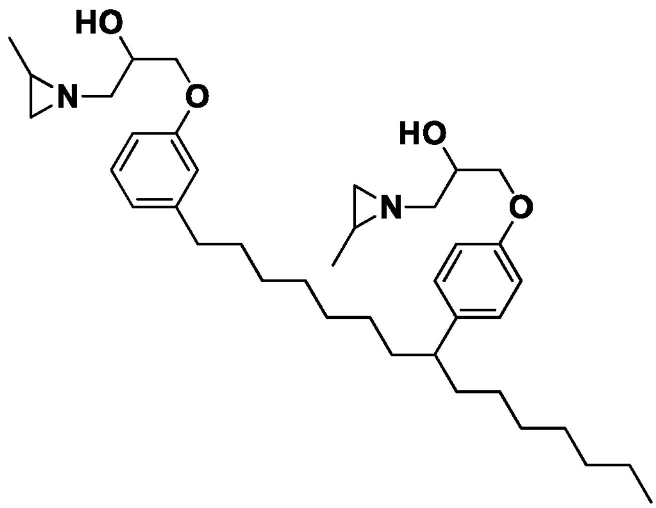

**A14** The AZ-component according to A1a or to any one of A1 to A6 and A8 to A10 or any combination derived from the disclosure in section 1 and the entire specification, wherein the AZ-component is selected from the group consisting of AZ2-compound and wherein the AZ2-compound is the compound of the following formula

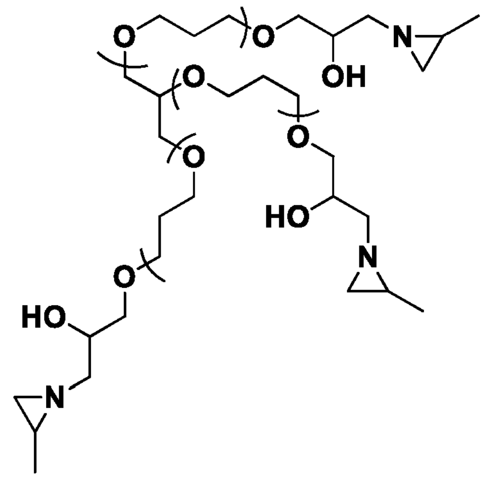

**A15** The AZ-component according to A1a or to any one of A1 to A6 and A8 to A10 or any combination derived from the disclosure in section 1 and the entire specification, wherein the AZ-component is selected from the group consisting of AZ2-compound and wherein the AZ2-compound is the compound of the following formula

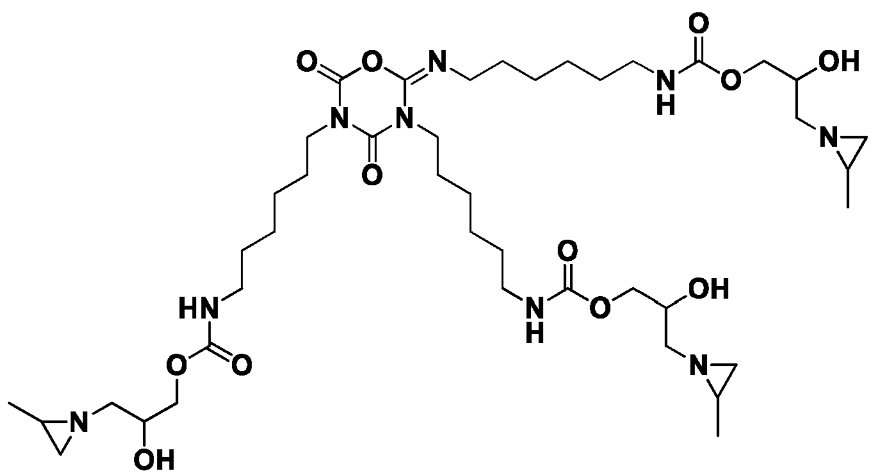

**A16** The AZ-component according to A1a or to any one of A1 to A6 and A8 to A10 or any combination derived from the disclosure in section 1 and the entire specification, wherein the AZ-component is selected from the group consisting of AZ2-compound and wherein the AZ2-compound is the compound of the following formula

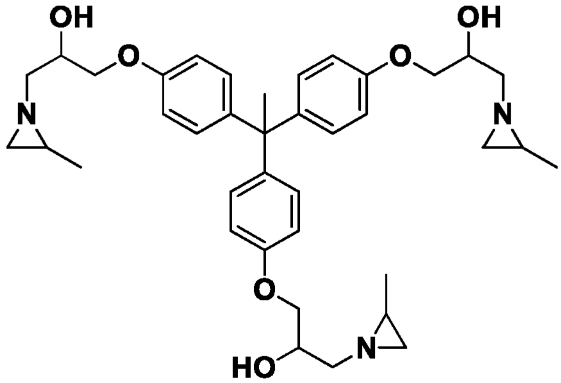

**A17** The AZ-component according to A1a or to any one of A1 to A6 and A8 to A10 or any combination derived from the disclosure in section 1 and the entire specification, wherein the AZ-component is selected from the group consisting of AZ1-compound, AZ2-compound and mixtures thereof, and wherein the AZ1-compound is the compound of the following formula,

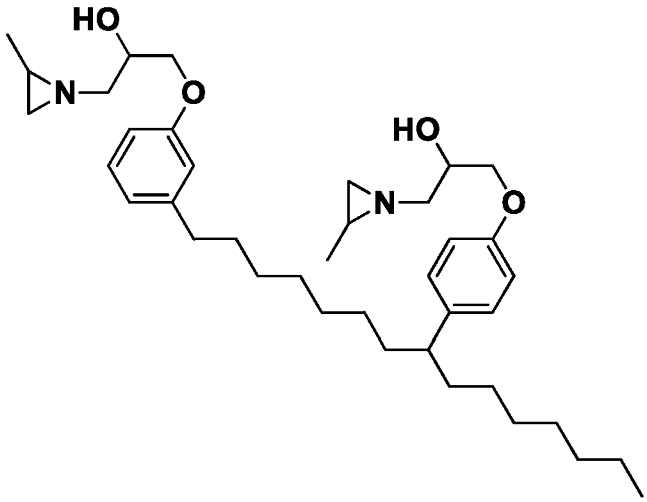

and wherein the AZ2-compound is selected from the group consisting of compounds of the following formulae,

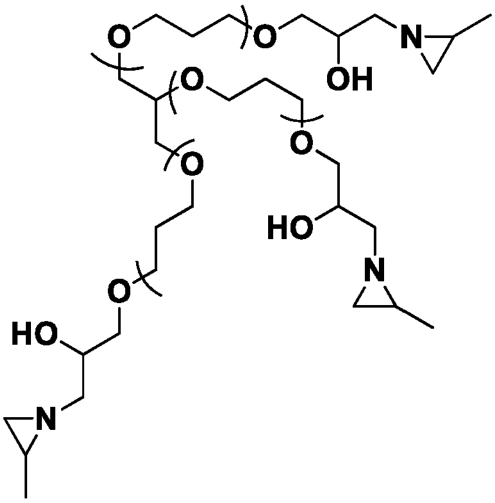

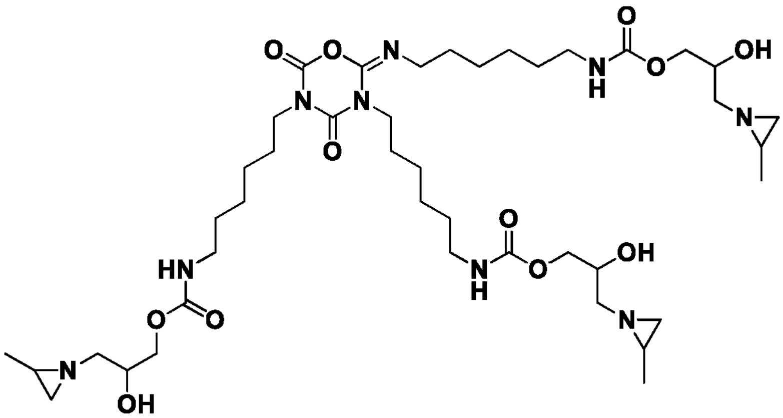

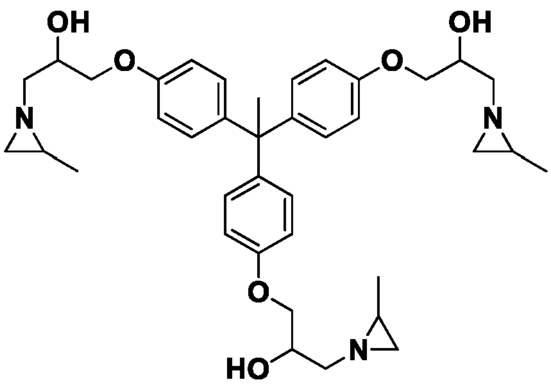

**A18** A liquid composition according to any combination derived from the disclosure in sections 1 and 3 and the entire specification, comprising:

i) a liquid medium which is selected from the group consisting of organic solvents, water and a mixture thereof, in an amount of at most 90, preferably at most 85, more preferably at most 80, for example at most 75, for example at most 70, for example at most 65, for example at most 60, for example at most 55, for example at most 50, for example at most 45, for example at most 40, for example at most 35, for example at most 30, for example at most 25, for example at most 20, for example at most 15, for example at most 10 for example at most 5, for example at most 2, for example at most 1 wt% on the total weight of the liquid composition, for example the liquid composition is free of the liquid medium; and

ii) an AZ-component according to A1a or to any one of A1 to A17 or any combination derived from the disclosure

in sections 1 and 3 and the entire specification; and wherein the total amount of all the components that make up the liquid composition totals 100 wt%.

**A19** The liquid composition according to A18 or any combination derived from the disclosure in sections 1 and 3 and the entire specification, wherein the liquid composition comprises water in an amount of at most 90, preferably at most 85, more preferably at most 80, most preferably at most 75, for example at most 70, for example at most 65, for example at most 60, for example at most 55, for example at most 50, for example at most 45, for example at most 40, for example at most 35, for example at most 30, for example at most 25, for example at most 20, for example at most 15, for example at most 10, for example at most 5, for example at most 2, for example at most 1 wt% on the total weight of the liquid composition, for example the liquid composition is free of water.

**A20** The liquid composition according to any one of A18-A19 or any combination derived from the disclosure in sections 1 and 3 and the entire specification, wherein the liquid composition comprises water in an amount of at least 70 and at most 90, preferably at least 75 and at most 85 wt% on the total weight of the liquid composition, and the AZ-compound in an amount of at least 10 and at most 30, preferably at least 15 and at most 25 wt% on the total weight of the liquid composition.

**A21** The liquid composition according to any one of A18-A20 or any combination derived from the disclosure in sections 1 and 3 and the entire specification, wherein the liquid composition comprises water in an amount of at least 50 and at most 75, preferably at least 55 and at most 70 wt% on the total weight of the liquid composition, and the AZ-compound in an amount of at least 25 and at most 50, preferably at least 30 and at most 45 wt% on the total weight of the liquid composition.

**A22** The liquid composition according to any one of A18-A21 or any combination derived from the disclosure in sections 1 and 3 and the entire specification, wherein the liquid composition comprises water in an amount of at least 40 and at most 70, preferably at least 45 and at most 65 wt% on the total weight of the liquid composition, and the AZ-compound in an amount of at least 30 and at most 60, preferably at least 35 and at most 55 wt% on the total weight of the liquid composition.

**A23** The liquid composition according to any one of A18-A22 or any combination derived from the disclosure in sections 1 and 3 and the entire specification, wherein the water is present in an amount of at least 30 and at most 95, preferably at least 40 and at most 85, for example at least 45 and at most 85, for example at least 50 and at most 70, for example at least 55 and at most 65 wt% on the total weight of the liquid composition, and wherein the total amount of all the components that make up the liquid composition totals 100 wt%.

**A24** The liquid composition according to any one of A18-A23 or any combination derived from the disclosure in sections 1 and 3 and the entire specification, the liquid composition comprises an organic solvent in an amount of at most 40, preferably at most 30, for example at most 25, for example at most 20, for example at most 12, for example at most 10, for example at most 8, for example at most 5, for example at most 4, for example at most 3, for example at most 2, for example at most 1, for example at most 0.5, for example at most 0.2, for example at most 0.1 wt% on the total weight of the liquid composition.

**A25** The liquid composition according to any one of A18-A24 or any combination derived from the disclosure in sections 1 and 3 and the entire specification, wherein the liquid composition is free of an organic solvent.

**A26** The liquid composition according to any one of A18-A25 or any combination derived from the disclosure in sections 1 and 3 and the entire specification, wherein the liquid composition has a pH determined according to the ISO 976:2013 and according to the description, of at least 7.5 and at most 14.0, preferably at least 8.0 and at most 14.0, for example at least 8.5 and at most 13.5, for example at least 9.0 and at most 13.0, for example at least 9.2 and at most 12.5, for example at least 9.4 and at most 12.0, for example at least 10 and at most 13, for example at least 10.5 and at most 12.0, for example at least 10.5 and at most 11.6, for example at least 10.5 and at most 11.5, with the proviso that the liquid composition comprises water in an amount of at least 20, preferably at least 25, more preferably at least 30, most preferably at least 35 wt% on the total weight of the liquid composition.

**A27** The liquid composition according to any one of A18-A26 or any combination derived from the disclosure in sections 1 and 3 and the entire specification, wherein the liquid composition comprises a component T selected from the group consisting of: i) organic compounds having a molecular weight determined via MALDI-TOF MS according to the description, lower than 600 Da and comprising at least one aziridine ring, and ii) mixtures thereof, in an amount, determined via LC-MS according to the description, of at most 5, preferably at most 4, more preferably at most 3, for example at most 2, for example at most 1, for example at most 0.5, for example at most 0.1, for example at most 0.05 wt% on the total weight of the AZ-compound.

**A28** The liquid composition according to any one of A18-A27 or any combination derived from the disclosure in sections 1 and 3 and the entire specification, wherein the liquid composition is free of component T.

**A29** The liquid composition according to any one of A18-A28 or any combination derived from the disclosure in sections 1 and 3 and the entire specification, wherein the amount of chloride determined according to the ASTM D1726-11(2019), is at most 0.3, preferably at most 0.2, more preferably at most 0.1, for example at most 0.05, for

example at most 0.03 wt% on the total weight of the liquid composition.

**A30** The liquid composition according to any one of A18-A29 or any combination derived from the disclosure in sections 1 and 3 and the entire specification, further comprising:
iii) a polymer which has an acid value determined according to the ASTM D1639-90(1996)e1 of at least 5 and at most 300, preferably at least 8 and at most 200, more preferably at least 10 and at most 150 mg KOH/g and wherein the polymer may optionally comprise ionic functional groups.

**A31** The liquid composition according to any one of A18-A30 or any combination derived from the disclosure in sections 1 and 3 and the entire specification, wherein the polymer is selected from the group consisting of polyesters, polyamides, polycarbonates, polyimides, alkyds, uralkyds, polyacrylics, polyurethanes, poly(urethane-acrylic)s, and mixtures thereof, preferably the polymer is selected from the group consisting of polyamides, polyacrylics, polyurethanes, poly(urethane-acrylic)s, and mixtures thereof, most preferably the polymer is selected from the group consisting of polyacrylics, polyurethanes, poly(urethane-acrylic)s, and mixtures thereof.

**A32** The liquid composition according to any one of A18-A31 or any combination derived from the disclosure in sections 1 and 3 and the entire specification, wherein the polymer is present in an amount of at least 5 and at most 65, preferably at least 10 and at most 60, more preferably at least 20 and at most 55, for example at least 30 and at most 50 wt% on the total weight of the aqueous composition, and wherein the total amount of all the components that make up the mixture totals 100 wt%.

**A33** The liquid composition according to any one of A18-A32 or any combination derived from the disclosure in sections 1 and 3 and the entire specification, wherein the liquid composition is a coating composition, preferably a liquid coating composition.

**A34** A kit-of-parts according to any combination derived from the disclosure in sections 4, 3 and 1 and the entire specification, comprising parts A and B which are physically separated from each other, wherein:

i) the part A comprises a liquid composition according to any one of A18-A33 or any combination derived from the disclosure in sections 1 and 3 and the entire specification, and
ii) the part B comprises a polymer which has an acid value determined according to the ASTM D1639-90(1996)e1 in the range from 5 to 300, preferably from 8 to 200, more preferably from 10 to 150 mg KOH/g and wherein the polymer may optionally comprise ionic functional groups, and wherein the polymer is preferably selected from the group consisting of polyesters, polyamides, polycarbonates, polyimides, alkyds, uralkyds, polyacrylics, polyurethanes, poly(urethane-acrylic)s, and mixtures thereof, more preferably the polymer is selected from the group consisting of polyamides, polyacrylics, polyurethanes, poly(urethane-acrylic)s, and mixtures thereof, most preferably the polymer is selected from the group consisting of polyacrylics, polyurethanes, poly(urethane-acrylic)s, and mixtures thereof,

wherein the part A does not comprise the polymer of the part B, and the part B does not comprise the liquid composition of the part A.

**A35** A cured form of an AZ-component according to A1a or any one of A1-A17 or any combination derived from the disclosure in sections 4, 3 and 1 and the entire specification, or of a liquid composition according to any one of A18-A33 or any combination derived from the disclosure in sections 4, 3 and 1 and the entire specification.

**A36** The cured form according to A35, wherein the cured form is a film.

**A37** An article comprising: i) an AZ-component according to A1a or any one of A1-A17 or any combination derived from the disclosure in sections 4, 3 and 1 and the entire specification, or ii) a liquid composition according to any one of A18-A33 or any combination derived from the disclosure in sections 4, 3 and 1 and the entire specification, and/or iii) a cured form according to any one of A35-A36 or any combination derived from the disclosure in sections 4, 3 and 1 and the entire specification.

**A38** The article according to A37 or any combination derived from the disclosure in sections 4, 3 and 1 and the entire specification, wherein the article is selected from the group consisting of textile, glass, metal, composite, plastic, wood, engineered wood, wood-like, leather, artificial leather, paper, fibers.

**A39** Use of an AZ-component according to A1a or any one of A1-A17, or a liquid composition according to any one of A18-A33, as a crosslinker.

**A40** Use of any one or any combination of the following:

i) an AZ-component according to A1a or any one of A1-A17 or any combination derived from the disclosure in sections 4, 3 and 1 and the entire specification;
ii) a liquid composition according to any one of A18-A33 or any combination derived from the disclosure in sections 4, 3 and 1 and the entire specification;
iii) a kit-of-parts according to A34 or any combination derived from the disclosure in sections 4, 3 and 1 and the entire specification;

iv) a cured form according to any one of A35-A36 or any combination derived from the disclosure in sections 4, 3 and 1 and the entire specification;

v) an article according to any one of A37-A38 or any combination derived from the disclosure in sections 4, 3 and 1 and the entire specification;

in coatings, paints, inks, varnishes, lubricants, adhesives, additive manufacturing, 3D-printing, textiles, waxes, fuels, photography, plastics, medical devices and in the preparation of medical compositions.

All combinations of minimum and maximum values of the parameters disclosed in the specification may be used to define the parameter ranges for various preferments or embodiments of the invention.

Unless otherwise explicitly stated, any feature, element, component, embodiment, aspect, range and especially any preferred feature, preferred element, preferred embodiment, preferred aspect, preferred range, preferred combination of ranges, preferments, embodiments and aspects in connection with any piece of the disclosure disclosed in any one of A1a or A1 to A40, shown above can be combined with each other and with any other feature, element, component, embodiment, aspect, range and especially any preferred feature, preferred element, preferred embodiment, preferred aspect, preferred range, preferred combination of ranges, preferments, embodiments and aspects of the invention as these are disclosed in the entire specification including the claims.

Unless otherwise explicitly stated, any feature, element, component, embodiment, aspect, range and especially any preferred feature, preferred element, preferred embodiment, preferred aspect, preferred range, preferred combination of ranges, preferments, embodiments and aspects of the invention as these are disclosed in the entire specification including the claims can be combined with each other.

**Definitions**

By ‘aziridine ring’ is meant in the specification a 3-membered ring system composed of one nitrogen and two carbon atoms.

By the term ’saturated hydrocarbylene’ is meant a bivalent organic group formed by removing two hydrogen atoms from a saturated hydrocarbon, the free valences of which are not engaged in a double bond. Exemplary hydrocarbylenes include but are not limited to methylene.

By the term ’saturated’ is meant that the relevant entity does not contain any unsaturation.

By the term ’ionic functional group’ is meant herein a functional group that comprises one or both of a cation and an anion and said functional group is covalently bonded to the entity to which it relates to.

By ’poor chemical resistance’ -referring to a cured coating (film)- is meant in the specification that the chemical resistance of a film measured as described in the specification is at most 1 [rating scale 0-5, wherein 5 represents the best performance and 0 represents the worst performance, as the rating scale is disclosed in the specification].

By ’reasonable chemical resistance’ -referring to a cured coating (film)-is meant in the specification that the chemical resistance of a film measured as described in the specification is 2 [rating scale 0-5, wherein 5 represents the best performance and 0 represents the worst performance, as the rating scale is disclosed in the specification].

By ’good chemical resistance’ -referring to a cured coating (film)- is meant in the specification that the chemical resistance of a film measured as described in the specification is 3 [rating scale 0-5, wherein 5 represents the best performance and 0 represents the worst performance, as the rating scale is disclosed in the specification].

By ’very good chemical resistance’ -referring to a cured coating (film)-is meant in the specification that the chemical resistance of a film measured as described in the specification is 4 [rating scale 0-5, wherein 5 represents the best performance and 0 represents the worst performance, as the rating scale is disclosed in the specification].

By ’excellent chemical resistance’ -referring to a cured coating (film)-is meant in the specification that the chemical resistance of a film measured as described in the specification is 5 [rating scale 0-5, wherein 5 represents the best performance and 0 represents the worst performance, as the rating scale is disclosed in the specification].

By ’poor crosslinking efficiency’ -referring to an entity- is meant in the specification that the entity has an end chemical resistance which is poor, or equally that the end chemical resistance of the entity is poor.

By ’reasonable crosslinking efficiency’ -referring to an entity- is meant in the specification that the entity has an end chemical resistance which is reasonable, or equally that the end chemical resistance of the entity is reasonable.

By ’good crosslinking efficiency’ -referring to an entity- is meant in the specification that the entity has an end chemical resistance which is good, or equally that the end chemical resistance of the entity is good.

By ’very good crosslinking efficiency’ -referring to an entity- is meant in the specification that the entity has an end chemical resistance which is very good, or equally that the end chemical resistance of the entity is very good.

By ’excellent crosslinking efficiency’ -referring to an entity- is meant in the specification that the entity has an end chemical resistance which is excellent, or equally that the end chemical resistance of the entity is excellent.

In the context of the specification, the genotoxicity is measured according to the ToxTracker® assay (Toxys, Leiden, the Netherlands) as described in the Examples. In the context of the specification by *’positive induced genotoxicity’*

is meant that the induction level of the biomarkers Bscl2-GFP and Rtkn-GFP is equal to or higher than 2-fold in at least one of 10, 25 and 50% cytotoxicity in the absence or presence of the metabolizing system rat S9 liver extract. In the context of the specification by *'weakly positive induced genotoxicity'* is meant that the induction level of the biomarkers Bscl2-GFP and Rtkn-GFP is higher than 1.5-fold and lower than 2-fold in at least one of 10, 25 and 50% cytotoxicity (but lower than 2-fold at 10, 25 and 50% cytotoxicity) in the absence or presence of rat S9 liver extract-based metabolizing systems (aroclor1254-induced rats, Moltox, Boone, NC, USA). In the context of the specification by *'genotoxicity comparable with the naturally occurring background'* is meant that the induction level of the biomarkers Bscl2-GFP and Rtkn-GFP is lower than or equal to 1.5-fold at 10, 25 and 50% cytotoxicity in the absence and presence of rat S9 liver extract-based metabolizing systems (aroclor1254-induced rats, Moltox, Boone, NC, USA). The induction level of the genotoxicity reporters Bscl2-GFP and Rtkn-GFP is preferably lower than or equal to 1.5-fold at 10, 25 and 50% cytotoxicity in the absence and presence of rat S9 liver extract-based metabolizing systems (aroclor1254-induced rats, Moltox, Boone, NC, USA). In the context of the specification by 'non-genotoxic' (or equally 'not genotoxic') is meant that the induction level of the biomarkers Bscl2-GFP and Rtkn-GFP is lower than 2-fold -preferably equal to or lower than 1.9-fold, more preferably equal to or lower than 1.8-fold, for example equal to or lower than 1.7-fold, for example equal to or lower than 1.6-fold for example equal to or lower than 1.5-fold at 10, 25 and 50% cytotoxicity in the absence and presence of rat S9 liver extract-based metabolizing systems (aroclor1254-induced rats, Moltox, Boone, NC, USA).

By 'room temperature' is meant herein 23t1 °C.

By 'atmospheric pressure' is meant in the specification pressure of 1 atm. By 'standard conditions' is meant in the specification room temperature and atmospheric pressure, collectively.

By 'lower than' is meant in the specification that the relevant maximum boundary value is not included in the range. By 'higher than' is meant in the specification that the relevant minimum boundary value is not included in the range.

By 'rpm' is meant revolutions per minute.

By 'polyacrylics' is meant in the specification any polymer comprising of reacted residues of acrylic acid and/or methacrylic acid and/or an ester of acrylic acid and/or an ester of methacrylic acid, and/or styrene, and/or acrylonitrile, and/or acrylamide, and/or esters of itaconic acid, and/or itaconic acid, and/or divinyl benzene, and/or methacrylonitrile, and/or vinyl esters and/or vinyl halides, and/or esters of fumaric acid, and/or fumaric acid; preferably by 'polyacrylics' is meant in the specification any polymer consisting of reacted residues of acrylic acid and/or methacrylic acid and/or an ester of acrylic acid and/or an ester of methacrylic acid, and/or styrene, and/or acrylonitrile, and/or acrylamide, and/or esters of itaconic acid, and/or itaconic acid, and/or divinyl benzene, and/or methacrylonitrile, and/or vinyl esters and/or vinyl halides, and/or esters of fumaric acid, and/or fumaric acid; preferably by 'vinyl polymer' is meant in the specification any polymer consisting of reacted residues of acrylic acid and/or methacrylic acid and/or an ester of acrylic acid and/or an ester of methacrylic acid, and/or styrene, and/or acrylonitrile, and/or acrylamide, and/or esters of itaconic acid, and/or itaconic acid, and/or divinyl benzene, and/or methacrylonitrile, and/or vinyl esters and/or vinyl halides, and/or esters of fumaric acid, and/or fumaric acid.

By 'epoxide protons' is meant in the specification the two protons of the methylene group ($-CH_2-$) of an oxirane (also known as epoxy) group which group has the following formula

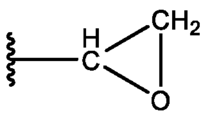

By 'curing' or 'cure' is meant in the specification the process of becoming 'set' that is to form an irreversibly crosslinked network (the so-called 'cured form' or 'cured composition'), a material that can no longer flow, be melted or dissolved. Herein, the terms 'curing' 'cure' and 'crosslinking' are used interchangeably. the curing may take place either at standard conditions (as these are defined in the specification), or by using heat, or by using pressure, or by applying vacuum, or by irradiation, e.g. UV-radiation, or by any combination thereof.

The characterization of the physical state of a chemical entity, e.g. a composition is assessed at and refers to standard conditions. For example, if a composition is characterized as ' liquid composition' it means that this composition is liquid at room temperature and at atmospheric pressure.

**1. The (aziridinyl hydroxy)-functional organic component (AZ-component) of the invention**

The (aziridinyl hydroxy)-functional organic component (AZ-component) of the invention is as disclosed in the entire specification (including the claims). The term 'AZ-component' as used in the specification includes any and all of its preferments, combinations of its features and ranges as well as combinations of any and all of its preferments with any and all of the combinations of its features and ranges. Thus, any and all of the references to the AZ-component disclosed in this section 1 -and its subsections- includes any and all of their preferments, combinations of their features

and ranges as well as combinations of any and all of their preferments with any and all of the combinations of their features and ranges, are collectively referred to -in the entire specification- as the AZ-component.

The AZ-component is according to any one of A1a or A1 to A17 or any combination disclosed in the entire specification including the claims. More specifically, the (aziridinyl hydroxy)-functional organic component (AZ-component) is selected from the group consisting of i) to vi): i) (aziridinyl hydroxy)-functional organic compound AZ1 of Formula A1 having only two aziridine rings (AZ1-compound), ii) (aziridinyl hydroxy)-functional organic compound AZ2 of Formula A2 having only three aziridine rings (AZ2-compound), iii) (aziridinyl hydroxy)-functional organic compound AZ3 of Formula A3 having only four aziridine rings (AZ3-compound), iv) (aziridinyl hydroxy)-functional organic compound AZ4 of Formula A4 having only five aziridine rings (AZ4-compound), v) (aziridinyl hydroxy)-functional organic compound AZ5 of Formula A5 having only six aziridine rings (AZ5-compound), and vi) mixtures thereof, preferably the AZ-component is selected from the group consisting of i) to iv): i) (aziridinyl hydroxy)-functional organic compound AZ1 of Formula A1 having only two aziridine rings (AZ1-compound), ii) (aziridinyl hydroxy)-functional organic compound AZ2 of Formula A2 having only three aziridine rings (AZ2-compound), iii) (aziridinyl hydroxy)-functional organic compound AZ3 of Formula A3 having only four aziridine rings (AZ3-compound), and iv) mixtures thereof,

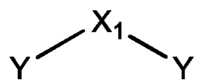
Formula A1

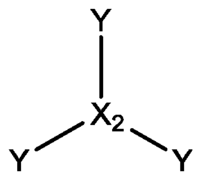
Formula A2

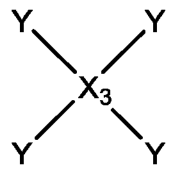
Formula A3

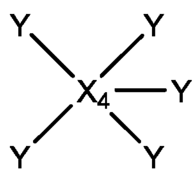
Formula A4

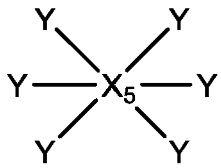
Formula A5

wherein

$X_1$ is a bivalent aliphatic organic radical or a bivalent aromatic organic radical, preferably $X_1$ is a bivalent aliphatic organic radical;
$X_2$ is a trivalent aliphatic organic radical or a trivalent aromatic organic radical, preferably $X_2$ is a trivalent aliphatic organic radical;
$X_3$ is a quadrivalent aliphatic organic radical or a quadrivalent aromatic organic radical, preferably $X_3$ is a quadrivalent aliphatic organic radical;
$X_4$ is a pentavalent aliphatic organic radical or a pentavalent aromatic organic radical, preferably $X_4$ is a pentavalent aliphatic organic radical;
$X_5$ is a hexavalent aliphatic organic radical or a hexavalent aromatic organic radical, preferably $X_5$ is a hexavalent aliphatic organic radical;
and wherein each of the $X_1$ to $X_5$ consists of a collection of atoms covalently connected in a configuration that comprises -preferably consists of- linear and/or branched and/or ring structures, which collection of atoms is selected from the group consisting of i) to x): i) carbon and hydrogen atoms, ii) carbon, hydrogen and oxygen atoms, iii) carbon, hydrogen and nitrogen atoms, iv) carbon, hydrogen and sulphur atoms, v) carbon, hydrogen, oxygen and nitrogen atoms, vi) carbon, hydrogen, nitrogen and sulphur atoms, vii) carbon, hydrogen, oxygen and sulphur atoms, viii) carbon, hydrogen, oxygen, nitrogen and sulphur, atoms, ix) carbon, hydrogen and silicon atoms, and x) carbon, hydrogen, oxygen and silicon atoms and xi) any combination of ix) and/or x) with any one or all of the iii) to viii),
and wherein each of the $X_1$ to $X_5$ has carbon atoms and hydrogen atoms,
and wherein each of the $X_1$ to $X_5$ has optionally oxygen atoms and/or nitrogen atoms and/or sulphur atoms and/or silicon atoms,
and wherein the $X_1$, and/or the $X_2$ and/or the $X_3$ and/or the $X_4$ and/or the $X_5$ may optionally comprise an ionic

functional group, preferably the $X_1$, and/or the $X_2$ and/or the $X_3$ and/or the $X_4$ and/or the $X_5$ does not comprise an ionic functional group,
and wherein
Y is a monovalent organic radical selected from the group consisting of: i) monovalent (aziridinyl hydroxyisopropyl) organic radical of Formula B1, ii) monovalent (aziridinyl hydroxycyclohexane) organic radical of Formula B2, and iii) monovalent (aziridinyl hydroxycyclohexane) organic radical of Formula B3, preferably Y is a monovalent (aziridinyl hydroxyisopropyl) organic radical of Formula B1,

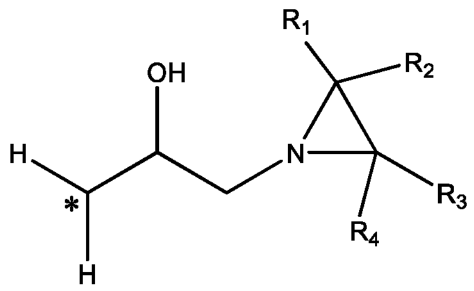

Formula B1

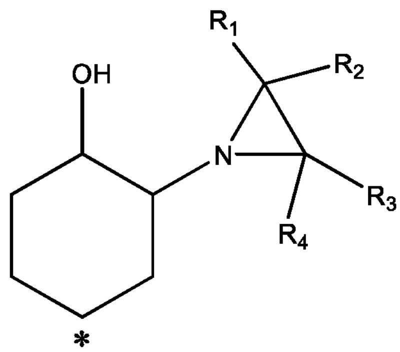

Formula B2

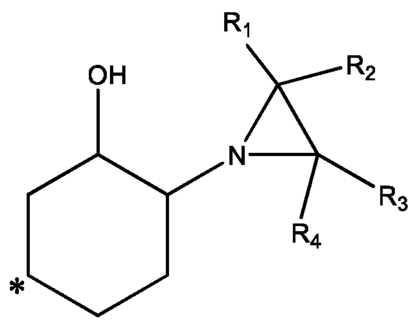

Formula B3

and wherein
$R_1$ is selected from the group consisting of hydrogen and methyl; and
$R_2$ is selected from the group consisting of hydrogen, methyl, and $C_2$-$C_5$ alkyl; and
$R_3$ is selected from the group consisting of methyl, and $C_2$-$C_4$ alkyl; and
$R_4$ is selected from the group consisting of hydrogen, methyl, and $C_2$-$C_4$ alkyl;
and wherein
the Y in each of the compounds A1 to A5 may be the same or different to each other and wherein each of the single

covalent bonds between the Y and each one of the $X_1$ to $X_5$ is selected from the group consisting of carbon-carbon single bond and carbon-oxygen single bond preferably each of the single covalent bonds between the Y and each one of the $X_1$ to $X_5$ is carbon-oxygen single bond,
and wherein
each of the AZ1- to AZ5-compounds has a molecular weight determined via MALDI-TOF MS according to the description, of at least 600 and at most 10000, preferably at least 600 and at most 8000, more preferably at least 600 and at most 7000, even more preferably at least 600 and at most 6000, for example at least 600 and at most 5500, for example at least 600 and at most 5000, for example at least 600 and at most 4000, for example at least 600 and at most 3500, for example at least 600 and at most 3200, for example at least 600 and at most 3000, for example at least 600 and at most 2500, for example at least 600 and at most 2200, for example at least 600 and at most 2000, for example at least 630 and at most 10000, for example at least 630 and at most 8000, for example at least 630 and at most 7000, for example at least 630 and at most 6000, for example at least 630 and at most 5500, for example at least 630 and at most 5000, for example at least 630 and at most 4000, for example at least 630 and at most 3500, for example at least 630 and at most 3200, for example at least 630 and at most 3000, for example at least 630 and at most 2500, for example at least 630 and at most 2200, for example at least 630 and at most 2000, for example at least 640 and at most 10000, for example at least 640 and at most 8000, for example at least 640 and at most 7000, for example at least 640 and at most 6000, for example at least 640 and at most 5500, for example at least 640 and at most 5000, for example at least 640 and at most 4000, for example at least 640 and at most 3500, for example at least 640 and at most 3200, for example at least 640 and at most 3000, for example at least 640 and at most 2500, for example at least 640 and at most 2200, for example at least 640 and at most 2000, for example at least 650 and at most 10000, for example at least 650 and at most 8000, for example at least 650 and at most 7000, for example at least 650 and at most 6000, for example at least 650 and at most 5500, for example at least 650 and at most 5000, for example at least 650 and at most 4000, for example at least 650 and at most 3500, for example at least 650 and at most 3200, for example at least 650 and at most 3000, for example at least 650 and at most 2500, for example at least 650 and at most 2200, for example at least 650 and at most 2000, for example at least 700 and at most 10000, for example at least 700 and at most 8000, for example at least 700 and at most 7000, for example at least 700 and at most 6000, for example at least 700 and at most 5500, for example at least 700 and at most 5000, for example at least 700 and at most 4000, for example at least 700 and at most 3500, for example at least 700 and at most 3200, for example at least 700 and at most 3000, for example at least 700 and at most 2500, for example at least 700 and at most 2200, for example at least 700 and at most 2000,for example at least 750 and at most 10000, for example at least 750 and at most 8000, for example at least 750 and at most 7000, for example at least 750 and at most 6000, for example at least 750 and at most 5500, for example at least 750 and at most 5000, for example at least 750 and at most 4000, for example at least 750 and at most 3500, for example at least 750 and at most 3200, for example at least 750 and at most 3000, for example at least 750 and at most 2500, for example at least 750 and at most 2200, for example at least 750 and at most 2000, for example at least 800 and at most 10000, for example at least 800 and at most 8000, for example at least 800 and at most 7000, for example at least 800 and at most 6000, for example at least 800 and at most 5500, for example at least 800 and at most 5000, for example at least 800 and at most 4000, for example at least 800 and at most 3500, for example at least 800 and at most 3200, for example at least 800 and at most 3000, for example at least 800 and at most 2500, for example at least 800 and at most 2200, for example at least 800 and at most 2000, for example at least 850 and at most 10000, for example at least 850 and at most 8000, for example at least 850 and at most 7000, for example at least 850 and at most 6000, for example at least 850 and at most 5500, for example at least 850 and at most 5000, for example at least 850 and at most 4000, for example at least 850 and at most 3500, for example at least 850 and at most 3200, for example at least 850 and at most 3000, for example at least 850 and at most 2500, for example at least 850 and at most 2200, for example at least 850 and at most 2000, for example at least 900 and at most 10000, for example at least 900 and at most 8000, for example at least 900 and at most 7000, for example at least 900 and at most 6000, for example at least 900 and at most 5500, for example at least 900 and at most 5000, for example at least 900 and at most 4000, for example at least 900 and at most 3500, for example at least 900 and at most 3200, for example at least 900 and at most 3000, for example at least 900 and at most 2500, for example at least 900 and at most 2200, for example at least 900 and at most 2000, for example at least 950 and at most 10000, for example at least 950 and at most 8000, for example at least 950 and at most 7000, for example at least 950 and at most 6000, for example at least 950 and at most 5500, for example at least 950 and at most 5000, for example at least 950 and at most 4000, for example at least 950 and at most 3500, for example at least 950 and at most 3200, for example at least 950 and at most 3000, for example at least 950 and at most 2500, for example at least 950 and at most 2200, for example at least 950 and at most 2000, for example at least 1000 and at most 10000, for example at least 1000 and at most 8000, for example at least 1000 and at most 7000, for example at least 1000 and at most 6000, for example at least 1000 and at most 5500, for example at least 1000 and at most 5000, for example at least 1000 and at most 4000, for example at least

1000 and at most 3500, for example at least 1000 and at most 3200, for example at least 1000 and at most 3000, for example at least 1000 and at most 2500, for example at least 1000 and at most 2200, for example at least 1000 and at most 2000, for example at least 1100 and at most 10000, for example at least 1100 and at most 8000, for example at least 1100 and at most 7000, for example at least 1100 and at most 6000, for example at least 1100 and at most 5500, for example at least 1100 and at most 5000, for example at least 1100 and at most 4000, for example at least 1100 and at most 3500, for example at least 1100 and at most 3200, for example at least 1100 and at most 3000, for example at least 1100 and at most 2500, for example at least 1100 and at most 2200, for example at least 1100 and at most 2000, for example at least 1200 and at most 10000, for example at least 1200 and at most 8000, for example at least 1200 and at most 7000, for example at least 1200 and at most 6000, for example at least 1200 and at most 5500, for example at least 1200 and at most 5000, for example at least 1200 and at most 4000, for example at least 1200 and at most 3500, for example at least 1200 and at most 3200, for example at least 1200 and at most 3000, for example at least 1200 and at most 2500, for example at least 1200 and at most 2200, for example at least 1200 and at most 2000 Da,
and wherein
the $X_1$ and the $X_2$ and the $X_3$ and the $X_4$ and the $X_5$ does not contain one or any combination of the following structural units BP1, BP2, BP3, and BS

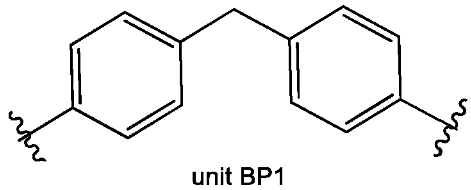

unit BP1

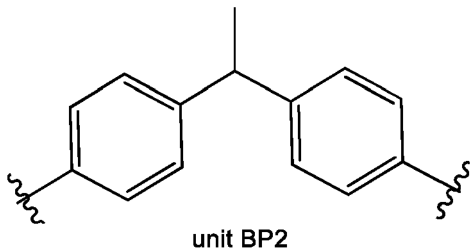

unit BP2

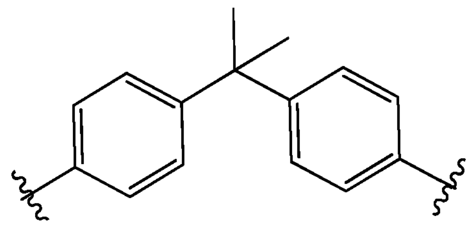

unit BP3

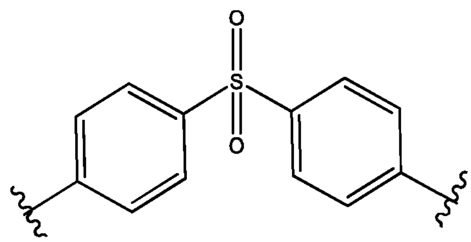

unit BS

Preferably, the AZ-component according to any one of the preceding claims, wherein the $X_1$ is a bivalent aliphatic organic radical or a bivalent aromatic organic radical other than a bivalent organic radical selected from the group consisting of bivalent radical of bisphenol A, bivalent radical of bisphenol AP, bivalent radical of bisphenol AF,

bivalent radical of bisphenol B, bivalent radical of bisphenol BP, bivalent radical of bisphenol C, bivalent radical of bisphenol C2, bivalent radical of bisphenol E, bivalent radical of bisphenol F, bivalent radical of bisphenol G, bivalent radical of bisphenol M, bivalent radical of bisphenol S, bivalent radical of bisphenol P, bivalent radical of bisphenol PH, bivalent radical of bisphenol TMC, bivalent radical of bisphenol Z, bivalent radical of dinitrobisphenol A, bivalent radical of tetrabromobisphenol A, and combinations thereof, preferably $X_1$ is a bivalent aliphatic organic radical.

Preferably, the AZ-component according to any one of the preceding claims, wherein the $X_1$, and/or the $X_2$ and/or the $X_3$ and/or the $X_4$ and/or the $X_5$ comprises at least one structural unit or a combination of structural units selected from the group consisting of unit 1, unit 2, unit 3, unit 4, unit 5, unit 6, unit 7, unit 8, unit 9, unit 10, and unit 11, preferably comprises at least one structural unit or a combination of structural units selected from the group consisting of unit 1, unit 4, unit 9, unit 10 and unit 11, as the units 1 to 11 are depicted below:

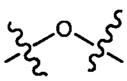
unit 1

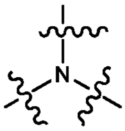
unit 2

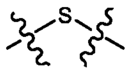
unit 3

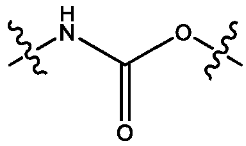
unit 4

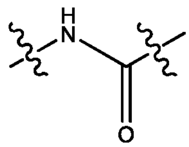
unit 5

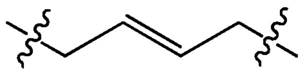
unit 6

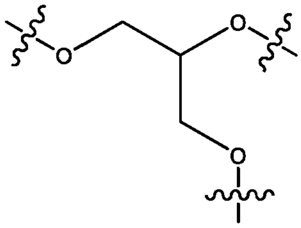
unit 7

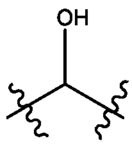
unit 8

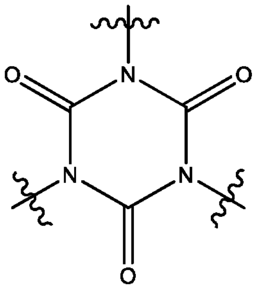
unit 9

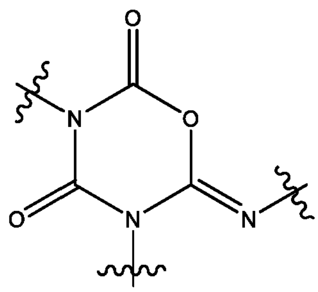
unit 10

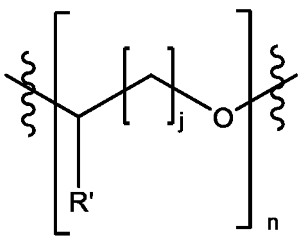
unit 11

wherein

R' is selected from the group consisting of hydrogen and methyl; and
j is an integer ranging from 1 to 5, preferably from 1 to 3; and

n is an integer ranging from and including 2 up to and including 50, preferably from and including 2 up to and including 40, more preferably from and including 2 up to and including 30, most preferably from and including 2 up to and including 20, for example from and including 2 up to and including 15, for example from and including 2 up to and including 10, for example from and including 2 up to and including 5, for example the n is 2, for example from and including 3 up to and including 50, for example from and including 3 up to and including 40, for example from and including 3 up to and including 30, for example from and including 3 up to and including 20, for example from and including 3 up to and including 15, for example from and including 3 up to and including 10, for example from and including 3 up to and including 5,for example the n is 3, for example from and including 4 up to and including 50, for example from and including 4 up to and including 40, for example from and including 4 up to and including 30, for example from and including 4 up to and including 20, for example from and including 4 up to and including 15, for example from and including 4 up to and including 10, for example from and including 4 up to and including 5, for example the n is 4, for example from and including 5 up to and including 50, for example from and including 5 up to and including 40, for example from and including 5 up to and including 30, for example from and including 5 up to and including 20, for example from and including 5 up to and including 15, for example from and including 5 up to and including 10, for example the n is 5, for example from and including 6 up to and including 50, for example from and including 6 up to and including 40, for example from and including 6 up to and including 30, for example from and including 6 up to and including 20, for example from and including 6 up to and including 15, for example from and including 6 up to and including 10, for example the n is 6, for example from and including 7 up to and including 50, for example from and including 7 up to and including 40, for example from and including 7 up to and including 30, for example from and including 7 up to and including 20, for example from and including 7 up to and including 15, for example from and including 7 up to and including 10, for example the n is 7, for example from and including 8 up to and including 50, for example from and including 8 up to and including 40, for example from and including 8 up to and including 30, for example from and including 8 up to and including 20, for example from and including 8 up to and including 15, for example from and including 8 up to and including 10, for example the n is 8, for example from and including 9 up to and including 50, for example from and including 9 up to and including 40, for example from and including 9 up to and including 30, for example from and including 9 up to and including 20, for example from and including 9 up to and including 15, for example from and including 9 up to and including 10, for example the n is 9, for example from and including 10 up to and including 50, for example from and including 10 up to and including 40, for example from and including 10 up to and including 30, for example from and including 10 up to and including 20, for example from and including 10 up to and including 15, for example the n is 10.

Preferably, the AZ-component according to any one of the preceding claims, wherein

$R_1$ is selected from the group consisting of hydrogen and methyl; and
$R_2$ is selected from the group consisting of hydrogen, methyl, and ethyl; and
$R_3$ is selected from the group consisting of methyl, and $C_2$-$C_4$ alkyl; and
$R_4$ is selected from the group consisting of hydrogen, methyl, and ethyl.

Preferably, the AZ-component according to any one of the preceding claims, wherein the aggregate number of carbon atoms in $R_1$, and $R_2$ and $R_3$ and $R_4$ is at most 9, preferably at most 4, more preferably at most 2, for example at most 1.

Preferably, the AZ-component according to any one of the preceding claims, wherein the $X_1$, and/or the $X_2$ and/or the $X_3$ and/or the $X_4$ and/or the $X_5$ comprises at least one unit 11 as a structural unit.

Preferably, the $X_1$ is the bivalent aliphatic organic radical of Formula A1a'

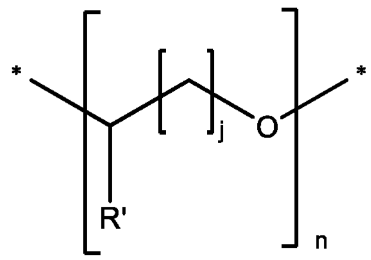

Formula A1a'

wherein

R' is selected from the group consisting of hydrogen and methyl; and
j is an integer ranging from 1 to 5, preferably from 1 to 3; and
n is an integer ranging from and including 2 up to and including 50, preferably from and including 2 up to and including 40, more preferably from and including 2 up to and including 30, most preferably from and including 2 up to and including 20, for example from and including 2 up to and including 15, for example from and including 2 up to and including 10, for example from and including 2 up to and including 5, for example the n is 2, for example from and including 3 up to and including 50, for example from and including 3 up to and including 40, for example from and including 3 up to and including 30, for example from and including 3 up to and including 20, for example from and including 3 up to and including 15, for example from and including 3 up to and including 10, for example from and including 3 up to and including 5, for example the n is 3, for example from and including 4 up to and including 50, for example from and including 4 up to and including 40, for example from and including 4 up to and including 30, for example from and including 4 up to and including 20, for example from and including 4 up to and including 15, for example from and including 4 up to and including 10, for example from and including 4 up to and including 5, for example the n is 4, for example from and including 5 up to and including 50, for example from and including 5 up to and including 40, for example from and including 5 up to and including 30, for example from and including 5 up to and including 20, for example from and including 5 up to and including 15, for example from and including 5 up to and including 10, for example the n is 5, for example from and including 6 up to and including 50, for example from and including 6 up to and including 40, for example from and including 6 up to and including 30, for example from and including 6 up to and including 20, for example from and including 6 up to and including 15, for example from and including 6 up to and including 10, for example the n is 6, for example from and including 7 up to and including 50, for example from and including 7 up to and including 40, for example from and including 7 up to and including 30, for example from and including 7 up to and including 20, for example from and including 7 up to and including 15, for example from and including 7 up to and including 10, for example the n is 7, for example from and including 8 up to and including 50, for example from and including 8 up to and including 40, for example from and including 8 up to and including 30, for example from and including 8 up to and including 20, for example from and including 8 up to and including 15, for example from and including 8 up to and including 10, for example the n is 8, for example from and including 9 up to and including 50, for example from and including 9 up to and including 40, for example from and including 9 up to and including 30, for example from and including 9 up to and including 20, for example from and including 9 up to and including 15, for example from and including 9 up to and including 10, for example the n is 9, for example from and including 10 up to and including 50, for example from and including 10 up to and including 40, for example from and including 10 up to and including 30, for example from and including 10 up to and including 20, for example from and including 10 up to and including 15, for example the n is 10.

Preferably, the AZ-component according to any one of the preceding claims, wherein each of the $X_1$ to $X_5$ contains only single covalent bonds, or both single and double covalent bonds, and wherein the single covalent bonds are selected from the group consisting of carbon-carbon single bond, carbon-hydrogen single bond, carbon-nitrogen single bond, carbon-sulphur single bond, carbon-silicon single bond, silicon-oxygen single bond, nitrogen-hydrogen single bond, sulphur-oxygen single bond, carbon-oxygen single bond wherein the oxygen is bonded to a hydrogen forming a hydroxyl group, silicon-oxygen-silicon single bonds, and wherein the double covalent bonds are selected from the group consisting of carbon-carbon double bond, carbon-nitrogen double bond, sulphur-oxygen double bond, carbon-oxygen double bond wherein the carbon is a member of a ring structure, and carbon-oxygen double bond wherein the carbon is bonded to another two carbons via carbon-carbon single bonds, carbon-oxygen double bond wherein the carbon is bonded to another oxygen via single bond and to a nitrogen via a single bond, carbon-oxygen double bond wherein the carbon is bonded to two nitrogens via carbon-nitrogen single bonds, carbon-oxygen double bond wherein the carbon is bonded to another two oxygens via single bonds; preferably the double covalent bonds are selected from the group consisting of carbon-carbon double bond, carbon-nitrogen double bond, sulphur-oxygen double bond, carbon-oxygen double bond wherein the carbon is a member of a ring structure, and carbon-oxygen double bond wherein the carbon is bonded to another two carbons via carbon-carbon single bonds, carbon-oxygen double bond wherein the carbon is bonded to another oxygen via single bond and to a nitrogen via a single bond, carbon-oxygen double bond wherein the carbon is bonded to two nitrogens via carbon-nitrogen single bonds.

Preferably, the aggregate number of carbon and hydrogen atoms in $X_1$ and/or $X_2$ and/or $X_3$ and/or $X_4$ and/or $X_5$ is at least 5, preferably at least 10, more preferably at least 15, most preferably at least 20, for example at least 25, for example at least 30, for example at least 35, for example at least 40, for example at least 45, for example at least 50, for example at least 55, for example at least 60, for example at least 65, for example at least 70, for example at least 75, for example at least 80, for example at least 85, for example at least 90, for example at least 95, for example at least 97, for example at least 98, for example at least 99, for example 100 % as to the aggregate number of all the atoms present in $X_1$ and/or $X_2$ and/or $X_3$ and/or $X_4$ and/or $X_5$, respectively.

Preferably, the aggregate number of all the atoms present in $X_1$ and/or $X_2$ and/or $X_3$ and/or $X_4$ and/or $X_5$ is at

most 600, preferably at most 550, more preferably at most 500, most preferably at most 450, for examples at most 400, for example at most 350, for example at most 300, for example at most 250, for example at most 200.

Preferably, the AZ-component is selected from the group consisting of i) to v): i) (aziridinyl hydroxy)-functional organic compound AZ1 of Formula A1 (AZ1-compound), ii) (aziridinyl hydroxy)-functional organic compound AZ2 of Formula A2 (AZ2-compound), iii) (aziridinyl hydroxy)-functional organic compound AZ3 of Formula A3 (AZ3-compound), iv) (aziridinyl hydroxy)-functional organic compound AZ5 of Formula A5 (AZ5-compound), and vi) mixtures thereof, and wherein

the AZ1-compound is selected from the group consisting of compounds having the Formula A1a, and compounds having the Formula A1b, as each of these Formulae A1a-A1d is described below

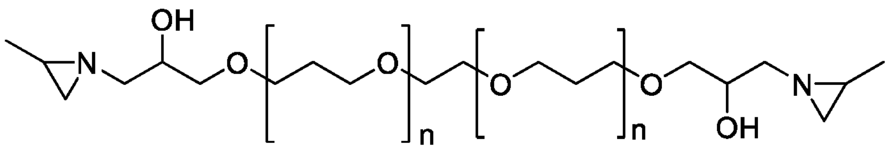

Formula A1a

wherein each of the n in Formula A1a is independently selected, and each of the n in Formula A1a is an integer ranging from and including 2 up to and including 20, preferably from and including 2 up to and including 10, more preferably from and including 2 up to and including 7, most preferably from and including 2 up to and including 5, for example from and including 2 up to and including 4, for example from and including 3 up to and including 20, for example from and including 3 up to and including 10, for example from and including 3 up to and including 7, for example from and including 3 up to and including 5, for example from and including 3 up to and including 4, for example n is equal to 4; and

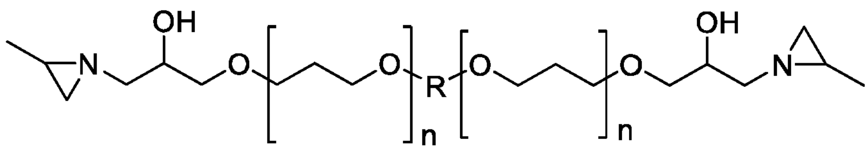

Formula A1b

wherein the R in Formula A1b is a $C_3$-$C_{10}$ saturated hydrocarbylene, and wherein each of the n in Formula A1b is independently selected and each of the n in Formula A1a is an integer ranging from and including 2 up to and including 20, preferably from and including 2 up to and including 10, more preferably from and including 2 up to and including 7, most preferably from and including 2 up to and including 5, for example from and including 2 up to and including 4, for example from and including 3 up to and including 20, for example from and including 3 up to and including 10, for example from and including 3 up to and including 7, for example from and including 3 up to and including 5, for example from and including 3 up to and including 4, for example n is equal to 4; and

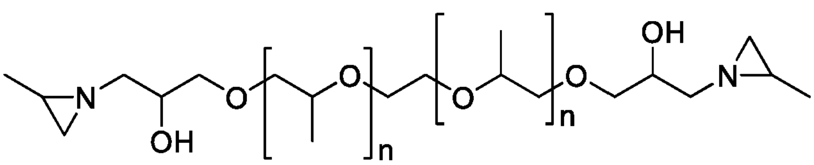

Formula A1c

wherein each of the n in Formula A1c is independently selected, and each of the n in Formula A1b is an integer ranging from and including 2 up to and including 20, preferably from and including 2 up to and including 10, more preferably from and including 2 up to and including 7, most preferably from and including 2 up to and including 5, for example from and including 2 up to and including 4, for example from and including 3 up to and including 20, for example from and including 3 up to and including 10, for example from and including 3 up to and including 7, for

example from and including 3 up to and including 5, for example from and including 3 up to and including 4, for example n is equal to 4, and

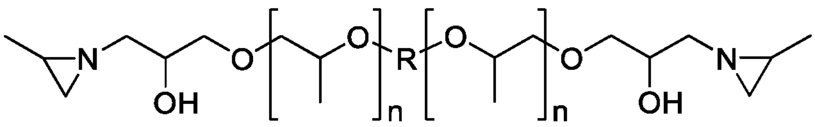

Formula A1d

wherein the R in Formula A1d is a $C_3$-$C_{10}$ saturated hydrocarbylene, and wherein each of the n in Formula A1d is independently selected and each of the n in Formula A1b is an integer ranging from and including 2 up to and including 20, preferably from and including 2 up to and including 10, more preferably from and including 2 up to and including 7, most preferably from and including 2 up to and including 5, for example from and including 2 up to and including 4, for example from and including 3 up to and including 20, for example from and including 3 up to and including 10, for example from and including 3 up to and including 7, for example from and including 3 up to and including 5, for example from and including 3 up to and including 4, for example n is equal to 4, and
wherein
the AZ2-compound is selected from the group consisting of compounds having the Formula A2a, compounds having the Formula A2b, compounds having the Formula A2c, compounds having the Formula A2d, compounds having the Formula A2e, as each of these Formulae A2a-A2e is described below

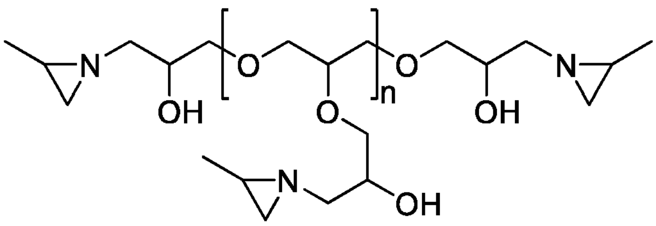

Formula A2a

wherein the n in Formula A2a is an integer ranging from and including 2 up to and including 20, for example from and including 2 up to and including 10, for example from and including 2 up to and including 8, for example from and including 3 up to and including 20, for example from and including 3 up to and including 10, for example from and including 3 up to and including 8, for example from and including 4 up to and including 20, for example from and including 4 up to and including 10, for example from and including 4 up to and including 8, for example from and including 5 up to and including 20, for example from and including 5 up to and including 10, for example from and including 5 up to and including 8, for example from and including 6 up to and including 20, for example from and including 6 up to and including 10, for example from and including 6 up to and including 8, for example n is equal to 7;
and

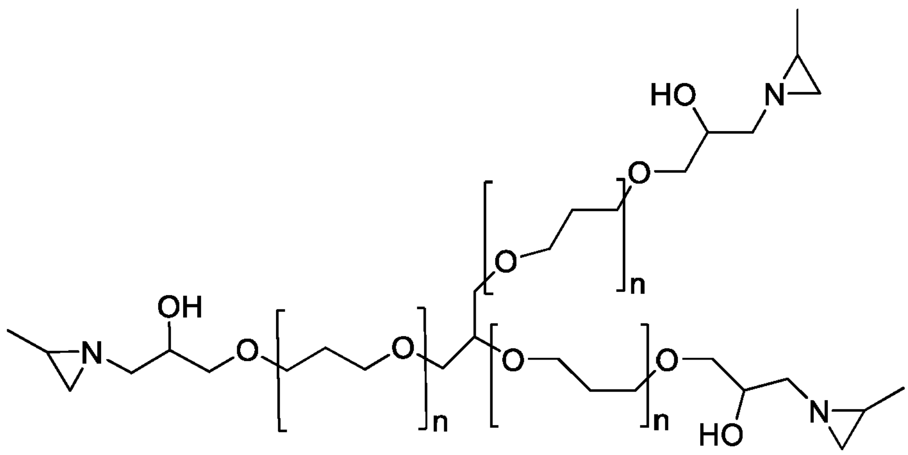

Formula A2b

wherein each of the n in Formula A2b is independently selected and each of the n in Formula A2b is an integer ranging from and including 2 up to and including 20, preferably from and including 2 up to and including 15, more preferably from and including 2 up to and including 12, most preferably from and including 2 up to and including 11, for example from and including 3 up to and including 20, for example from and including 3 up to and including 15 for example from and including 3 up to and including 12 for example from and including 3 up to and including 11, for example from and including 4 up to and including 20, for example from and including 4 up to and including 15 for example from and including 4 up to and including 12 for example from and including 4 up to and including 11, for example from and including 5 up to and including 20, for example from and including 5 up to and including 15 for example from and including 5 up to and including 12 for example from and including 5 up to and including 11, for example from and including 6 up to and including 20, for example from and including 6 up to and including 15 for example from and including 6 up to and including 12 for example from and including 6 up to and including 11, for example from and including 7 up to and including 20, for example from and including 7 up to and including 15 for example from and including 7 up to and including 12 for example from and including 7 up to and including 11, for example from and including 8 up to and including 20, for example from and including 8 up to and including 15 for example from and including 8 up to and including 12 for example from and including 8 up to and including 11, for example from and including 9 up to and including 20, for example from and including 9 up to and including 15 for example from and including 9 up to and including 12 for example from and including 9 up to and including 11, for example from and including 10 up to and including 20, for example from and including 10 up to and including 15 for example from and including 10 up to and including 12 for example from and including 10 up to and including 11, for example n is equal to 11;
and

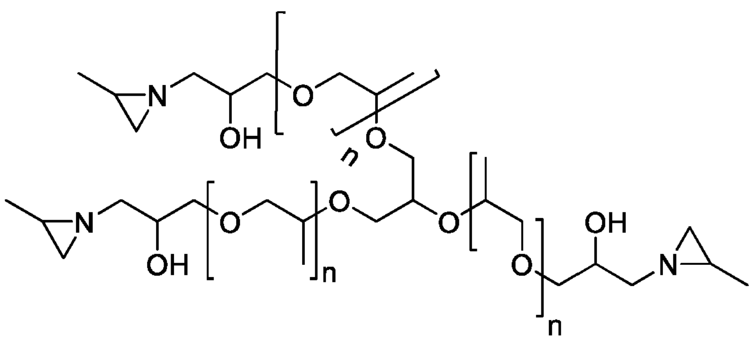

Formula A2c

wherein each of the n in Formula A2c is independently selected and each of the n in Formula A2c is an integer ranging from and including 2 up to and including 20, preferably from and including 2 up to and including 15, more preferably from and including 2 up to and including 12, most preferably from and including 2 up to and including 11,

for example from and including 3 up to and including 20, for example from and including 3 up to and including 15 for example from and including 3 up to and including 12 for example from and including 3 up to and including 11, for example from and including 4 up to and including 20, for example from and including 4 up to and including 15 for example from and including 4 up to and including 12 for example from and including 4 up to and including 11, for example from and including 5 up to and including 20, for example from and including 5 up to and including 15 for example from and including 5 up to and including 12 for example from and including 5 up to and including 11, for example from and including 6 up to and including 20, for example from and including 6 up to and including 15 for example from and including 6 up to and including 12 for example from and including 6 up to and including 11, for example from and including 7 up to and including 20, for example from and including 7 up to and including 15 for example from and including 7 up to and including 12 for example from and including 7 up to and including 11, for example from and including 8 up to and including 20, for example from and including 8 up to and including 15 for example from and including 8 up to and including 12 for example from and including 8 up to and including 11, for example from and including 9 up to and including 20, for example from and including 9 up to and including 15 for example from and including 9 up to and including 12 for example from and including 9 up to and including 11, for example from and including 10 up to and including 20, for example from and including 10 up to and including 15 for example from and including 10 up to and including 12 for example from and including 10 up to and including 11, for example n is equal to 11;

and

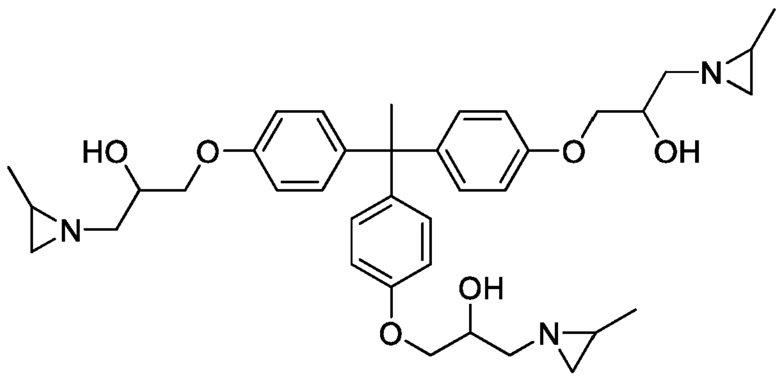

Formula A2d

and

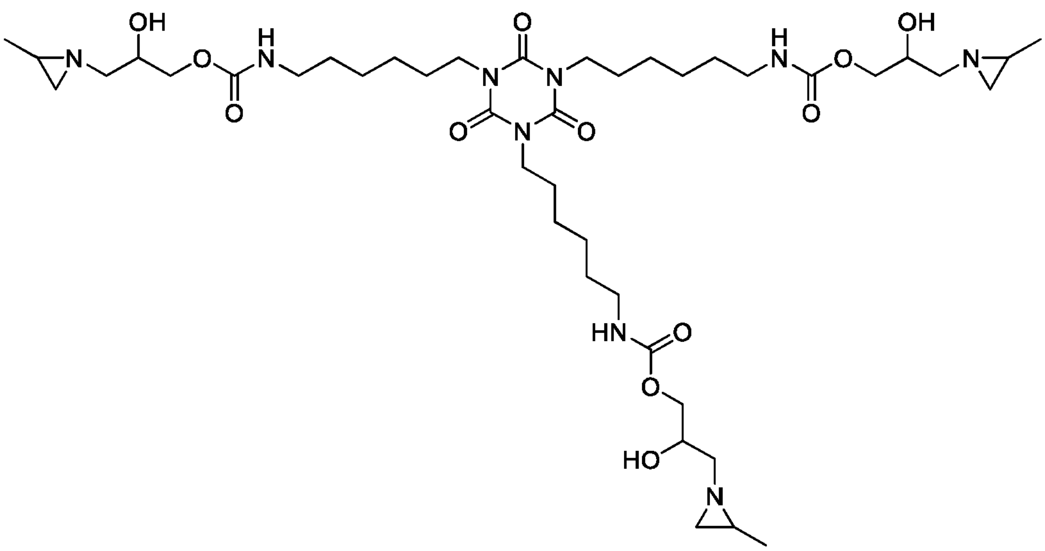

Formula A2e

and
wherein
the AZ3-compound is selected from the group consisting of compounds having the Formula A3a

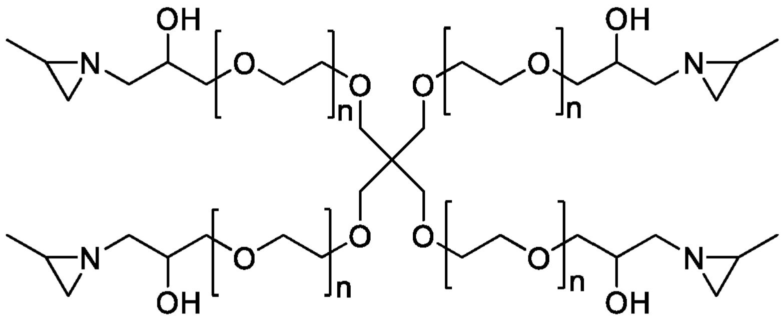

Formula A3a

wherein each of the n in Formula A3a is independently selected, and each of the n in Formula A3a is an integer ranging from and including 2 up to and including 20, preferably from and including 2 up to and including 15, more preferably from and including 2 up to and including 10, most preferably from and including 2 up to and including 9, for example from and including 2 up to and including 8, for example from and including 2 up to and including 7, for example from and including 2 up to and including 6, for example from and including 2 up to and including 5, for example from and including 2 up to and including 4, for example from and including 2 up to and including 3, for example n is equal to 3, for example n is equal to 2;
and
wherein
the AZ5-compound is selected from the group consisting of compounds having the Formula A5a

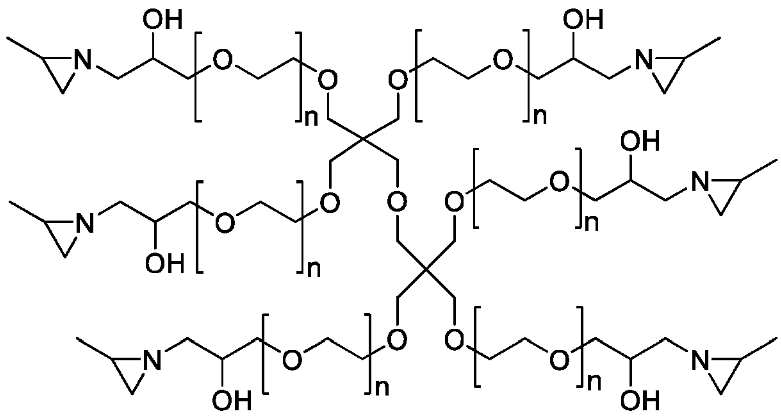

Formula A5a

wherein each of the n in Formula A5a is independently selected and each of the n in Formula A5a is an integer ranging from and including 2 up to and including 40, preferably from and including 2 up to and including 30, more preferably from and including 2 up to and including 20, most preferably from and including 2 up to and including 10, for example from and including 2 up to and including 9, for example from and including 2 up to and including 8, for example from and including 2 up to and including 7, for example from and including 2 up to and including 6, for example from and including 2 up to and including 5, for example from and including 2 up to and including 4, for example from and including 3 up to and including 40, for example from and including 3 up to and including 30, for example from and including 3 up to and including 20, for example from and including 3 up to and including 10, for example from and including 3 up to and including 9, for example from and including 3 up to and including 8, for example from and including 3 up to and including 7, for example from and including 3 up to and including 6, for example from and including 3 up to and including 5, for example from and including 3 up to and including 4, for example n is equal to 4, for example n is equal to 3.

Preferably, the $X_1$ and/or the $X_2$ and/or the $X_3$ and/or the $X_4$ and/or the $X_5$ does not contain any structural unit of (or equally derived from) phenol formaldehyde resins (also known as phenolic resins); examples of phenol-formaldehyde (PF) resins include novolacs (acid-catalyzed PF resins with a formaldehyde to phenol ratio of equal to or lower than one), and resols (base-catalyzed PF resins with a formaldehyde to phenol ratio of greater than one, usually equal to 1.5).

Preferably, the AZ-component is selected from the group consisting of AZ1-compound and wherein the AZ1-compound is the compound of the following formula

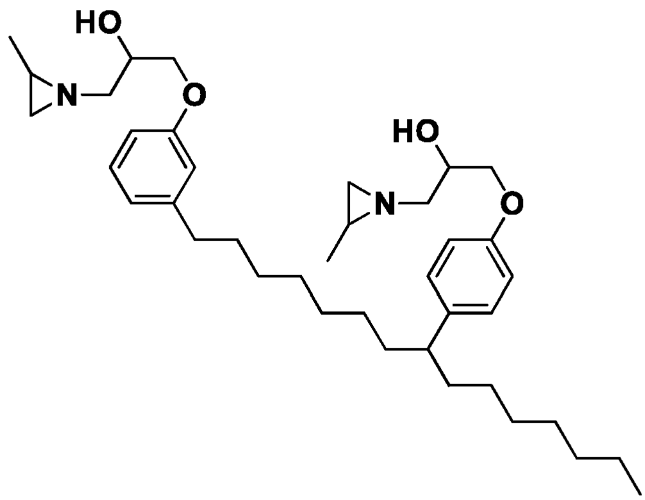

Preferably, the AZ-component is selected from the group consisting of AZ2-compound and wherein the AZ2-compound is the compound of the following formula

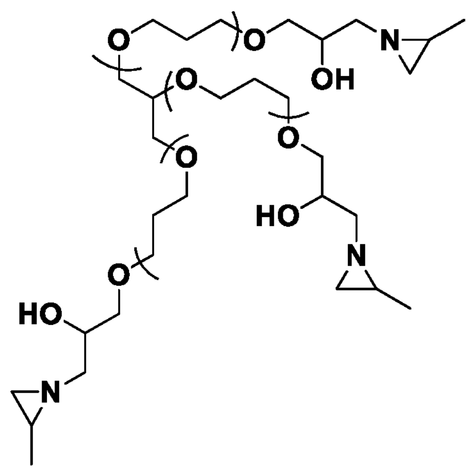

Preferably, the AZ-component is selected from the group consisting of AZ2-compound and wherein the AZ2-compound is the compound of the following formula

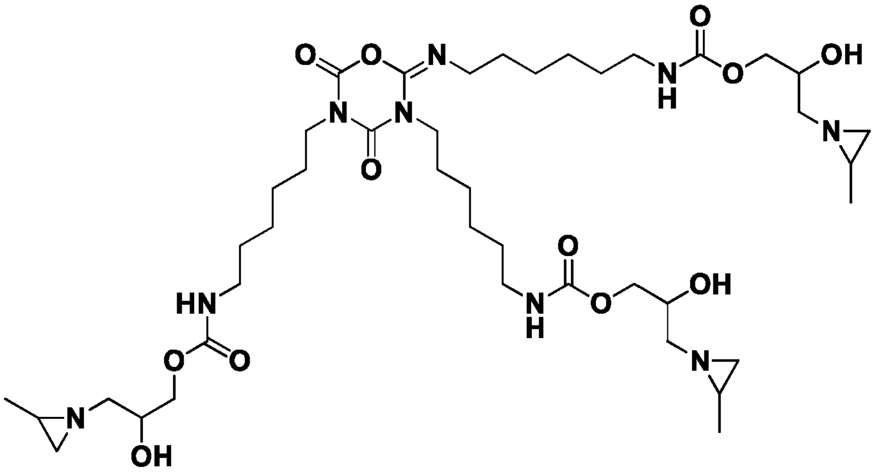

Preferably, the AZ-component is selected from the group consisting of AZ2-compound and wherein the AZ2-compound is the compound of the following formula

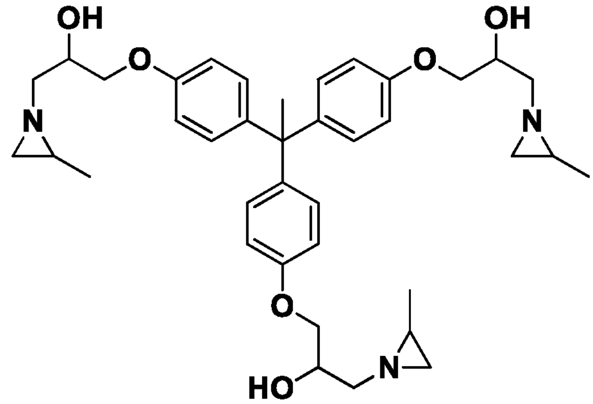

Preferably, the AZ1-compound and/or the AZ2-compound and/or the AZ3-compound and/or the AZ4-compound and/or the AZ5-compound comprises polyoxyethylene $(-O-CH_2-CH_2-)_x$ group(s), and/or polyoxypropylene $(-O-CHCH_3-CH_2-)_x$ group(s) and/or polytetrahydrofurane $(-O-CH_2-CH_2-CH_2-CH_2)_x$ groups, preferably in an amount of at least 10 and at most 92, more preferably at least 15 and at most 85, even more preferably at least 25 and at most 75, for example at least 30 and at most 65, for example at least 35 and at most 55 mol% relative to the corresponding AZ1-compound and/or the AZ2-compound and/or the AZ3-compound and/or the AZ4-compound and/or the AZ5-compound to which these mol% refer to.

Preferably, the AZ1-compound and/or the AZ2-compound and/or the AZ3-compound and/or the AZ4-compound and/or the AZ5-compound comprises methoxy poly(ethylene glycol) (MPEG) and/or poly(ethylene glycol) (PEG) groups (each of these groups with a calculated number average molecular weight ($M_n$) higher than 1600, preferably 2200 Da), preferably in an amount of at least 1 and at most 35, more preferably at least 3 and at most 30, even more preferably at least 5 and at most 27, for example at least 7 and at most 20, for example at least 8 and at most 17, for example at least 9 and at most 15, for example at least 10 and at most 13 mol% relative to the corresponding AZ1-compound and/or the AZ2-compound and/or the AZ3-compound and/or the AZ4-compound and/or the AZ5-compound to which these mol% refer to.

Preferably, the AZ1-compound and/or the AZ2-compound and/or the AZ3-compound and/or the AZ4-compound and/or the AZ5-compound comprise groups which have a sulfonate functionality, which can, for instance, be obtained by reacting epoxy groups with amino-functional sulfonic acid groups like 2-(cyclohexylamino) ethane sulfonic acid and 3-(cyclohexyl-amino)propane sulfonic acid. These groups which have a sulfonate functionality are preferably neutralized with a base, more preferably with an inorganic base. The reaction between an epoxy group and a group having a sulfonate functionality can, for instance, be done by reacting part of the epoxy groups of the polyepoxide prior to reaction with an aziridine-AZIR -as this is defined in the specification; see section 2-, or during the reaction with the aziridine-AZIR or stopping the reaction between the polyepoxide and the aziridine-AZIR at a certain moment, , e.g. by distilling off the excess of the aziridine-AZIR followed by reacting some or all of the remaining epoxy groups with the amino-functional

sulfonic acid molecules [2-(cyclohexylamino) ethane sulfonic acid and 3-cyclohexyl-amino)propane sulfonic acid]. Alternatively, a salt of taurine can be used as well, and preferably the taurine is neutralized with sodium hydroxide or potassium hydroxide or lithium hydroxide.

Preferably the AZ-component may be cured at a temperature of at least 0 and at most 85, preferably at least 15 and at most 80, more preferably at least 23 and at most 70, most preferably at least 30 and at most 85, for example at least 15 and at most 30 °C, for time periods that may vary and are preferably of at most 60, more preferably at most 30, even more preferably at most 15 minutes.

**2. Processes for preparing the AZ-component**

In principle, the (aziridinyl hydroxy)-functional organic component (AZ-component) as this is described in the specification, is preferably obtained by reacting an aziridine of Formula AZIR (abbreviated as 'aziridine-AZIR') with at least a polyepoxide having at least two and at most six epoxy groups.

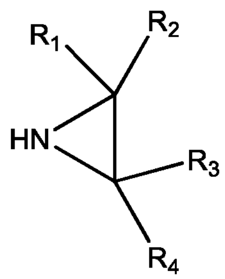

Formula AZIR

wherein

- $R_1$ is selected from the group consisting of hydrogen and methyl; and
- $R_2$ is selected from the group consisting of hydrogen, methyl, and $C_2$-$C_5$ alkyl; and
- $R_3$ is selected from the group consisting of methyl, and $C_2$-$C_4$ alkyl; and
- $R_4$ is selected from the group consisting of hydrogen, methyl, and $C_2$-$C_4$ alkyl.

Exemplary aziridines of Formula AZIR include but are not limited to propylene imine, 1,2-dimethyl aziridine, 2,2-dimethyl aziridine, 2-ethyl aziridine, butyl aziridine.

More particularly, the AZ1-compound (as this is described in the specification) is preferably obtained by reacting an aziridine of Formula AZIR with at least a polyepoxide having two epoxy groups. Exemplary polyepoxides having two epoxy groups include but are not limited to any glycidyl ether of a dihydroxy compound (which dihydroxy compound has a molecular weight of at least 374 Da), for instance, a polypropylene glycol diglycidyl ether like the ERISYS® GE-24 available by CVC Thermoset Specialties.

More particularly, the AZ2-compound (as this is described in the specification) is preferably obtained by reacting an aziridine of Formula AZIR with at least a polyepoxide having three epoxy groups. Exemplary polyepoxides having three epoxy groups include but are not limited to any glycidyl ether of a tri functional hydroxy compound with a molecular weight of at least 261 Da, as well as the ERISYS® GE-36, and EPALLOY® 9000 both available by CVC Thermoset Specialties.

More particularly, the AZ3-compound (as this is described in the specification) is preferably obtained by reacting an aziridine of Formula AZIR with at least a polyepoxide having four epoxy groups. Exemplary polyepoxides having four epoxy groups include but are not limited to any glycidyl ether of a tetra hydroxy compound with a molecular weight of at least 148 Da, like for instance the glycidyl ether of ethoxylated penta like Polyol R4410, available from Perstop.

More particularly, the AZ4-compound (as this is described in the specification) is preferably obtained by reacting an aziridine of Formula AZIR with at least a polyepoxide having five epoxy groups. Exemplary polyepoxides having five epoxy groups include but are not limited to any glycidyl ether of a penta hydroxy compound.

More particularly, the AZ5-compound (as this is described in the specification) is preferably obtained by reacting an aziridine of Formula AZIR with at least a polyepoxide having six epoxy groups. Exemplary polyepoxides having six epoxy groups include but are not limited to any glycidyl ether of a hexa hydroxy compound for instance the hexaglycidyl ether of dipentaerythritol.

The reaction (the terms refers to and encompasses any and all of the reactions mentioned just above in this

section) takes places at any temperature from 20 to 110°C, more preferably from 50 to 95°C, and most preferably from 70 to 90 °C and its progress can be monitored via $^1$H-NMR spectroscopy. The reaction is carried out for as long as the epoxy groups are reacted; this is monitored and verified by $^1$H-NMR spectroscopy where the characteristic $^1$H-NMR chemical shift of the epoxy protons (2.5 - 3 ppm) is disappeared. Preferably the reaction is carried out without solvent. However, if desired (for instance to reduce the viscosity), one or more solvents , e.g. methanol, ethanol, toluene, can be used during or after the reaction. If a solvent is used, it is often convenient to first dissolve the polyepoxide in the solvent (or mixture of solvents) before adding the aziridine-AZIR to the reaction mixture. The molar ratio of the mol of the aziridine groups of the aziridine-AZIR to the mol of the epoxy groups of the polyepoxide is at least 1 and at most 8, more preferably at least 1 and at most 4, even more preferably at least 1.1 and at most 3 and most preferably at least 1.2 and at most 2.2. Once the reaction is completed, the residual aziridine-AZIR is distilled off, preferably at a temperature from 60 to 90 °C, more preferably from 65 to 80°C, and at reduced pressure, for example from 20 to 50 mbar, preferably from 30-45 mbar. Preferably once the reaction is completed, the residual aziridine-AZIR is distilled off at reduced pressure from 20 to 50 mbar at 70°C, more preferably from 30 to 45 mbar at 70 °C. Subsequently, a further distillation step for the removal of any unreacted aziridine-AZIR and any other volatiles is carried out at 25 to 40°C at 2 to 4 mbar, until no aziridine-AZIR could be detected by $^1$H-NMR spectroscopy. It is often useful to add an additional solvent to the reaction mixture prior to or during distillation, to facilitate the removal of the excess of the aziridine-AZIR. If desired, a base can be used during the reaction, to reduce possible sources of acid. Bases include both organic bases, like tertiary amines or inorganic bases like sodium or potassium carbonate or for instance calcium hydroxide. The inorganic bases can be filtered off after the reaction is completed.

For example, an AZ2-compound of Formula A2d

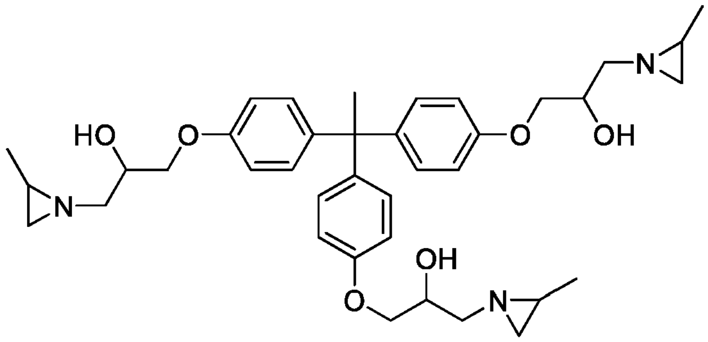

**Formula A2d**

may be prepared by reacting EPALLOY® 9000 (6.26 mmol) (available by CVC Thermoset Specialties) with propylene imine (70.22 mmol) at 80°C for 3 h in the presence of potassium carbonate (250 mg).

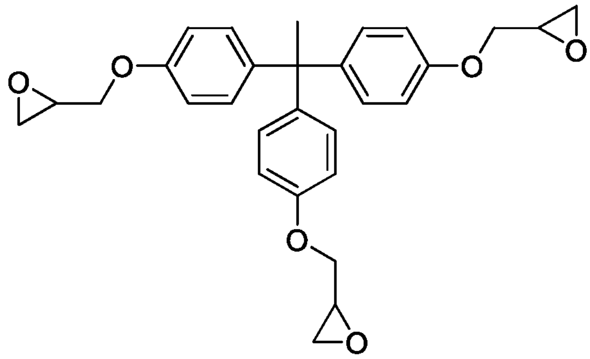

**EPALLOY®9000**

A high excess of propylene imine is typically used to dissolve EPALLOY® 9000 (the molar ratio of the mol of aziridine-AZIR to the mol of the epoxy groups. The reaction may be monitored via $^1$H-NMR. After 3 h at 80°C, the chemical shifts of the epoxide protons were not visible in the $^1$H-NMR; only some small peaks due to impurities. There has been no

change in the $^1$H-NMR spectrum once the reaction mixture was left at 80 °C from 3 to 22 h. The reaction mixture was cooled down to room temperature; it was diluted with toluene. The potassium carbonate was filtered off, and the excess of propylene imine was distilled off with toluene at 70°C and 40 mbar. The residue was held at 90-95 °C and at a reduced pressure of 3-4 mbar (oil pump) for 2 h, to remove any residual propylene imine and the toluene. The reaction product solidified quickly after cooling. The $^1$H-NMR did not show any peaks attributed to any residual propylene imine.

In principle, a process for making an AZ-compound wherein the Y is a monovalent (aziridinyl hydroxycyclohexane) organic radical of Formula B2, or a monovalent (aziridinyl hydroxycyclohexane) organic radical of Formula B3, is preferably obtained by reacting a hydroxy cyclohexene oxide with an isocyanurate of a diisocyanate, followed by a reaction with an aziridine-AZIR , e.g. propylene imine. For example, an AZ-component wherein the Y is a monovalent (aziridinyl hydroxycyclohexane) organic radical of Formula B2, monovalent (aziridinyl hydroxycyclohexane) organic radical of Formula B3, may be prepared by 1-hydroxy cyclohexene oxide (see Formula below)

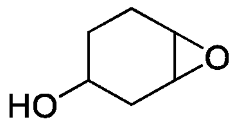

1-hydroxy cyclohexene oxide

which is obtained by reacting peracetic acid epoxidation of 1-hydroxy cyclohexene. The 1-hydroxy cyclohexene oxide is reacted with the isocyanurate of hexamethylene diisocyanate and subsequently reacted with a 3-fold excess of propylene imine, at 80 °C for 24 h. Afterwards, the excess of propylene imine is distilled off at a temperature of 50 °C and at reduced pressure. The resulting AZ-compound has a molecular weight of 1018 Da.

Alternatively, a polyoxyalkylene which comprises either at least one amino-functional group (preferably a secondary amino-functional group) or at least one carboxylic acid functional group, is reacted with some epoxy groups of a polyepoxide, before the reaction of the polyepoxide with the aziridine-AZIR, as this was described above. This may be a reaction scheme that may introduce polyether groups in any one of the $X_1$ to $X_5$.

Alternatively, an aziridine-AZIR may be partly reacted with a polyoxyalkylene which comprises at least one carboxylic acid functional group, prior to the reaction of the aziridine-AZIR with the polyepoxide, as this was described above. This may be a reaction scheme that may introduce polyether groups in any one of the $X_1$ to $X_5$.

Preferably, a mono-hydroxy or mono-amine functional polyether can be reacted in a 1:1 molar ratio with 2,4-toluene diisocyanate, and the reaction of which can be subsequently reacted with some of the hydroxyl groups which are formed after the reaction of the imine with the epoxy compound.

## 3. The liquid compositions of the invention

The liquid compositions of the invention are as disclosed in the entire specification, including the claims. The term 'liquid compositions of the invention' (or alternatively 'inventive liquid compositions') as used in the specification includes any and all of its preferments, combinations of its features and ranges as well as combinations of any and all of its preferments with any and all of the combinations of its features and ranges. Thus, any and all of the inventive liquid compositions disclosed in this section 3 includes any and all of their preferments, combinations of their features and ranges as well as combinations of any and all of their preferments with any and all of the combinations of their features and ranges, are collectively referred to -in the entire specification including the claims- as the inventive liquid compositions.

The inventive liquid compositions are according to any one of A18 to A33 and as disclosed in the entire specification, including the claims. More specifically, the inventive liquid compositions at 23$\pm$1 °C and at atmospheric pressure, comprise

i) a liquid medium which is selected from the group consisting of organic solvents, water and a mixture thereof, in an amount of at most 90, preferably at most 85, more preferably at most 80, for example at most 75, for example at most 70, for example at most 65, for example at most 60, for example at most 55, for example at most 50, for example at most 45, for example at most 40, for example at most 35, for example at most 30, for example at most 25, for example at most 20, for example at most 15, for example at most 10 for example at most 5, for example at most 2, for example at most 1 wt% on the total weight of the liquid composition, for example the liquid composition is free of the liquid medium; and

ii) an AZ-component according to A1a or to any one of A1 to A17; and wherein the total amount of all the components that make up the liquid composition totals 100 wt%.

The inventive liquid compositions may be prepared by mixing the liquid medium -as this disclosed in the entire

specification including the claims with the AZ-component -as this is disclosed in the entire specification including the claims. The mixing of the liquid medium and the AZ-component may be carried out at standard conditions.

The inventive liquid compositions may be solutions in an organic solvent, solutions in water, aqueous dispersions (with or without an organic solvent). Solubilizing the AZ-component in an organic solvent can be done by gentle stirring, if desired under mild heating, e.g. up to 40 °C to facilitate and accelerate the dissolution. If desired, mixtures of organic solvents or mixtures of water and organic solvents can be used. If desired other components can be combined in the solution of the AZ-component, including for example plasticizers, bases (preferred bases for neutralization are inorganic bases like the hydroxides of sodium, potassium or lithium, secondary amines, and tertiary amines), and dispersants. If desired the AZ-component can be dispersed in water by any technique, preferably using one or more bases like tertiary amines or inorganic bases like sodium hydroxide, by either using the AZ-component as such or first solubilizing the AZ-component in a preferably water-miscible solvent, adding a dispersant preferably a non-ionic dispersant; even more preferably, the dispersant is polymeric in nature and contains ionic functional groups, e.g. polyethylene oxide groups and/or sulphonate groups. Water is added to the mixture above under stirring and the mixture can be diluted with additional water if desired to reach the targeted solids content of the final dispersion. If desired, the solvent can be removed once the aqueous dispersion is obtained.

Preferably, the inventive liquid compositions are aqueous dispersions comprising water in an amount of at least 20 and at most 55 wt% on the total weight of the liquid composition (in this case on the total weight of the aqueous dispersion), and wherein and said aqueous dispersions have a pH determined according to the ISO 976:2013 and according to the description, of at least 7.5 and at most 14.0, preferably at least 8.0 and at most 14.0, for example at least 8.5 and at most 13.5, for example at least 9.0 and at most 13.0, for example at least 9.2 and at most 12.5, for example at least 9.4 and at most 12.0, for example at least 10 and at most 13, for example at least 10.5 and at most 12.0, for example at least 10.5 and at most 11.6, for example at least 10.5 and at most 11.5. Dispersions in water facilitate dosing to aqueous polymer systems and can reduce VOC levels since less or no organic solvent is needed to obtain a manageable viscosity for dosing. The dispersions can optionally be made with the help of an organic solvent which can be removed after dissipating the components in water. Typically, the removal of the organic solvent is done by distillation.

Preferably, the inventive liquid composition comprises water in an amount of at most 90, preferably at most 85, more preferably at most 80, most preferably at most 75, for example at most 70, for example at most 65, for example at most 60, for example at most 55, for example at most 50, for example at most 45, for example at most 40, for example at most 35, for example at most 30, for example at most 25, for example at most 20, for example at most 15, for example at most 10, for example at most 5, for example at most 2, for example at most 1 wt% on the total weight of the inventive liquid composition, for example the inventive liquid composition is free of water.

Preferably, the inventive liquid composition comprises water in an amount of at least 70 and at most 90, preferably at least 75 and at most 85 wt% on the total weight of the inventive liquid composition, and the AZ-compound in an amount of at least 10 and at most 30, preferably at least 15 and at most 25 wt% on the total weight of the inventive liquid composition.

Preferably, the inventive liquid composition comprises water in an amount of at least 50 and at most 75, preferably at least 55 and at most 70 wt% on the total weight of the inventive liquid composition, and the AZ-compound in an amount of at least 25 and at most 50, preferably at least 30 and at most 45 wt% on the total weight of the inventive liquid composition.

Preferably, the inventive liquid composition comprises water in an amount of at least 40 and at most 70, preferably at least 45 and at most 65 wt% on the total weight of the inventive liquid composition, and the AZ-compound in an amount of at least 30 and at most 60, preferably at least 35 and at most 55 wt% on the total weight of the inventive liquid composition.

Preferably, the water -in the inventive liquid composition- is present in an amount of at least 30 and at most 95, preferably at least 40 and at most 85, for example at least 45 and at most 85, for example at least 50 and at most 70, for example at least 55 and at most 65 wt% on the total weight of the inventive liquid composition, and wherein the total amount of all the components that make up the inventive liquid composition totals 100 wt%.

Preferably, the inventive liquid composition comprises an organic solvent in an amount of at most 40, preferably at most 30, for example at most 25, for example at most 20, for example at most 12, for example at most 10, for example at most 8, for example at most 5, for example at most 4, for example at most 3, for example at most 2, for example at most 1, for example at most 0.5, for example at most 0.2, for example at most 0.1 wt% on the total weight of the inventive liquid composition.

Preferably, the inventive liquid composition is free of an organic solvent.

Preferably, the inventive liquid composition has a pH determined according to the ISO 976:2013 and according to the description, of at least 7.5 and at most 14.0, preferably at least 8.0 and at most 14.0, for example at least 8.5 and at most 13.5, for example at least 9.0 and at most 13.0, for example at least 9.2 and at most 12.5, for example at least 9.4 and at most 12.0, for example at least 10 and at most 13, for example at least 10.5 and at most 12.0, for example at least 10.5 and at most 11.6, for example at least 10.5 and at most 11.5, with the proviso that the inventive liquid

composition comprises water in an amount of at least 20, preferably at least 25, more preferably at least 30, most preferably at least 35 wt% on the total weight of the inventive liquid composition.

Preferably, the inventive liquid composition comprises a component T selected from the group consisting of: i) organic compounds having a molecular weight determined via MALDI-TOF MS according to the description, lower than 600 Da and comprising at least one aziridine ring, and ii) mixtures thereof, in an amount, determined via LC-MS according to the description, of at most 5, preferably at most 4, more preferably at most 3, for example at most 2, for example at most 1, for example at most 0.5, for example at most 0.1, for example at most 0.05 wt% on the total weight of the AZ-compound.

Preferably, the inventive liquid composition is free of component T.

The inventive liquid composition may comprise chloride; preferably the amount of chloride determined according to the ASTM D1726-11(2019), is at most 0.3, preferably at most 0.2, more preferably at most 0.1, for example at most 0.05, for example at most 0.03 wt% on the total weight of the inventive liquid composition.

Preferably, the inventive liquid composition is free of chloride.

Preferably, the inventive liquid composition further comprises:

iii) a polymer which has an acid value determined according to the ASTM D1639-90(1996)e1 of at least 5 and at most 300, preferably at least 8 and at most 200, more preferably at least 10 and at most 150 mg KOH/g and wherein the polymer may optionally comprise ionic functional groups.

When the inventive liquid compositions further comprise a polymer -as this disclosed in the entire specification- then such inventive liquid composition may be prepared by mixing the liquid medium -as this disclosed in the entire specification including the claims with the AZ-component-as this is disclosed in the entire specification including the claims and the polymer -as this is disclosed in the entire specification-. The mixing of the liquid medium and the AZ-component and the polymer may be carried out at standard conditions.

Preferably, the polymer is selected from the group consisting of polyesters, polyamides, polycarbonates, polyimides, alkyds, uralkyds, polyacrylics, polyurethanes, poly(urethane-acrylic)s, and mixtures thereof, preferably the polymer is selected from the group consisting of polyamides, polyacrylics, polyurethanes, poly(urethane-acrylic)s, and mixtures thereof, most preferably the polymer is selected from the group consisting of polyacrylics, polyurethanes, poly(urethane-acrylic)s, and mixtures thereof.

Preferably, the polymer is present in an amount of at least 5 and at most 65, preferably at least 10 and at most 60, more preferably at least 20 and at most 55, for example at least 30 and at most 50 wt% on the total weight of the aqueous composition, and wherein the total amount of all the components that make up the mixture totals 100 wt%.

Preferably, the inventive liquid compositions are coating compositions, preferably liquid coating compositions.

The inventive liquid compositions may optionally (further) comprise other components, such as pigments and/or waxes, and/or the usual (processing) additives, for example degassing agents -if the inventive liquid compositions are used in powder coatings-, smoothness, appearance enhancing agents or (light) stabilizers. Suitable stabilizers include for example primary and/or secondary antioxidants and UV stabilizers for example quinones, (sterically hindered) phenolic compounds, phosphonites, phosphites, thioethers and HALS (hindered amine light stabilizers). Examples of suitable degassing agents include cyclohexane dimethanol bisbenzoate, benzoin.

In yet another aspect, the invention provides for a cured form of an inventive liquid composition as the latter disclosed in the entire specification.

The curing of the inventive liquid compositions may take place either by chemical reaction (resulting in the formation of irreversible covalent chemical bonds) or a combination of physical drying and chemical reaction. The curing of an inventive liquid composition may take place either at standard conditions (as these are defined in the specification), or by using heat, or by using pressure, or by applying vacuum, or by irradiation, e.g. UV-radiation, or by any combination thereof. Preferably an inventive liquid composition may be cured at a temperature of at least 0 and at most 85, preferably at least 15 and at most 80, more preferably at least 23 and at most 70, most preferably at least 30 and at most 85, for example at least 15 and at most 30 °C, for time periods that may vary and are preferably of at most 60, more preferably at most 30, even more preferably at most 15 minutes.

The liquid compositions of the invention may be cured in the presence of a cationic initiator.

The liquid compositions of the invention may be cured in the presence of a suitable polymer such as for example a polymer (preferably employed in an aqueous dispersion) which has an acid value determined according to the ASTM D1639-90(1996)e1 in the range from 5 to 300, preferably from 8 to 200, more preferably from 10 to 150 mg KOH/g and wherein the polymer may optionally comprise ionic functional groups, at standard conditions for example 12-48 h (preferably 24-48 h), and/or by heatcuring at elevated temperatures and at atmospheric pressure for example 70-80 °C for 10-60 minutes (preferably 15-30 minutes, more preferably 20 minutes), and/or any combination of curing at room temperature and curing at elevated temperatures; annealing steps (, e.g. 50°C for 16 h) may also be included in between curing at elevated temperatures and at atmospheric pressure and curing at standard conditions, wherein the annealing step follows the curing at elevated temperatures and at atmospheric pressure. Such inventive liquid compositions may preferably be cured at a temperature of at least 0 and at most 85, preferably at least 15 and at most 80, more preferably

at least 23 and at most 70, most preferably at least 30 and at most 85, for example at least 15 and at most 30 °C, for time periods that may vary and are preferably of at most 60, more preferably at most 30, even more preferably at most 15 minutes.

Obviously, curing at elevated temperatures and at atmospheric pressure requires shorter curing time; curing may also be effected by using pressure or by applying vacuum, or by irradiation , e.g. UV-radiation, or by any combination thereof. Such polymers may be selected from the group consisting of polyesters, polyamides, polycarbonates, polyimides, alkyds, uralkyds, polyacrylics, polyurethanes, poly(urethane-acrylic)s, and mixtures thereof, preferably the polymer is selected from the group consisting of polyamides, polyacrylics, polyurethanes, poly(urethane-acrylic)s, and mixtures thereof, most preferably the polymer is selected from the group consisting of polyacrylics, polyurethanes, poly(urethane-acrylic)s, and mixtures thereof.

**4. Other aspects and embodiments of the invention**

In yet another aspect, the invention provides for a kit-of-parts comprising parts A and B which are physically separated from each other, wherein:

i) the part A comprises a liquid composition according to any one of A18 to A33 or any combination derived from the disclosure in sections 1 and 3 and as disclosed in the entire specification including the claims; and
ii) the part B comprises a polymer which has an acid value determined according to the ASTM D1639-90(1996)e1 in the range from 5 to 300, preferably from 8 to 200, more preferably from 10 to 150 mg KOH/g and wherein the polymer may optionally comprise ionic functional groups, and wherein the polymer is preferably selected from the group consisting of polyesters, polyamides, polycarbonates, polyimides, alkyds, uralkyds, polyacrylics, polyurethanes, poly(urethane-acrylic)s, and mixtures thereof, more preferably the polymer is selected from the group consisting of polyamides, polyacrylics, polyurethanes, poly(urethane-acrylic)s, and mixtures thereof, most preferably the polymer is selected from the group consisting of polyacrylics, polyurethanes, poly(urethane-acrylic)s, and mixtures thereof,

wherein the part A does not comprise the polymer of the part B, and the part B does not comprise the liquid composition of the part A.

In yet another aspect, the invention provides for a cured form of an AZ-component according to A1a or to any one of A1 to A17 or any combination derived from the disclosure in sections 1 and 3 and as disclosed in the entire specification including the claims, of a liquid composition according to any one of A18-A33 or any combination derived from the disclosure in sections 1 and 3 and as disclosed in the entire specification including the claims; preferably the cured form is a film.

In yet another aspect, the invention provides for an article comprising:

i) an AZ-component according to A1a or to any one of A1 to A17 or any combination derived from the disclosure in section 1 and as disclosed in the entire specification including the claims, or ii) a liquid composition according to any one of A18-A33 or any combination derived from the disclosure in sections 1 and 3 and as disclosed in the entire specification including the claims; preferably the article is selected from the group consisting of textile, glass, metal, composite, plastic, wood, engineered wood, wood-like, leather, artificial leather, paper, fibers.

In yet another aspect, the invention provides for a use of any one or any combination of the following:

i) an AZ-component according to A1a or to any one of A1 to A17 or any combination derived from the disclosure in sections 1 and 3 and as disclosed in the entire specification including the claims;
ii) a liquid composition according to any one of A18-A33 or any combination derived from the disclosure in sections 1 and 3 and as disclosed in the entire specification including the claims,

as a crosslinker.

In yet another aspect, the invention provides for a use of any one or any combination of the following:

i) an AZ-component according to A1a or to any one of A1 to A17 or any combination derived from the disclosure in section 1 and as disclosed in the entire specification including the claims;
ii) a liquid composition according to any one of A18-A33 or any combination derived from the disclosure in sections 1 and 3 and as disclosed in the entire specification including the claims;
iii) a kit-of-parts according to A34 or any combination derived from the disclosure in sections 1 and 3 and as disclosed in the entire specification including the claims;

iv) a cured form according to any of A35-A36 or any combination derived from the disclosure in sections 1 and 3 and as disclosed in the entire specification including the claims;
v) an article according to any one of A37-38 or any combination derived from the disclosure in sections 1 and 3 and as disclosed in the entire specification including the claims;

in coatings, paints, inks, varnishes, lubricants, adhesives, additive manufacturing, 3D-printing, textiles, waxes, fuels, photography, plastics, medical devices, and in the preparation of medical compositions.

Yet, another aspect of the invention is any one of the AZ-components shown in the Examples that is according to the invention (Examples 1, 2, 3 and 4).

Yet, another aspect of the invention is any one of the liquid compositions shown in the Examples that is according to the invention (Examples 1, 2, 3, and 4).

Many other variations and embodiments of the invention are apparent to those skilled in the art, and such variations are contemplated within the scope of the claims.

Any feature, element, component, embodiment, range and especially any preferred feature, preferred element, preferred embodiment, preferred range, preferred combination of ranges, preferment described in the entire specification including the claims can be combined with each other.

Further aspects of the invention and preferred features thereof are given in the claims in the specification.

The invention will now be described in detail with reference to the following non-limiting examples, which are by way of illustration only.

## Examples

The invention is explained in more detail with reference to the following non-limiting examples.

All the Examples shown in this section were carried out in a controlled laboratory environment at standard conditions (as these are defined in the specification), a relative humidity of 50$\pm$1 % and an airflow of $\leq$ 0.1 m/s, unless otherwise explicitly specified.

### 1.1 Chemicals, raw materials and other materials

Triethylamine (purity 99.7 %) was supplied by ARKEMA. Propylene imine (purity $\geq$ 99.0%) was supplied by Menadiona. Anhydrous potassium carbonate ($K_2CO_3$; purity $\geq$ 99.0% ) was supplied by Alfa Aesar. The trimethylolpropane tris(2-methyl-1-aziridinepropionate) (CAS No. 64265-57-2; see structure in Example 1C) was supplied by DSM (commercial product name 'crosslinker CX-100'). The DESMODUR® N 3600 is a low viscosity aliphatic polyisocyanate (trimer) based on hexamethylene diisocyanate (HDI) (NCO content: 23.0$\pm$ 0.5 % (M105- ISO 11909); viscosity at 23 °C: 1200$\pm$ 300 mPa.s (M014-ISO 3219/A.3); color value (hazen): s 40 (M017-EN 1557); monomeric HDI: $\leq$ 0.25 % (M106-ISO 10283); equivalent weight: approx. 183; flash point: approx. 159 °C (DIN 53213/1); density at 20 °C: approx. 1.16 g/ml (DIN EN ISO 2811), supplied by Covestro. The DESMODUR® N 3900 is a low viscosity aliphatic polyisocyanate based on hexamethylene diisocyanate (HDI) (NCO content: 23.0$\pm$ 0.5 % (M105- ISO 11909); viscosity at 23 °C: 730$\pm$ 100 mPa.s (M014-ISO 3219/A.3); color value (hazen): $\leq$ 40 (M017-EN 1557); monomeric HDI: $\leq$ 0.25 % (M106-ISO 10283); equivalent weight: approx. 179; flash point: approx. 203 °C (DIN 53213/1); density at 20 °C: approx. 1.15 g/ml (DIN EN ISO 2811), supplied by Covestro. The ERISYS® GE-36 is a triglycidyl ether of propoxylated glycerine (see Scheme 1) (EEW: 620- 680 g/eq; viscosity: 200- 320 cps at 25 °C; hydrolysable chloride: < 0.10 %; flash point: > 93 °C, residual epichlorohydrin: < 20 ppm) was supplied by CVC Thermoset Specialties (now HUNTSMAN). The ERISYS® GA-240 (see Scheme 2) is glycidyl amine of m-xylelenediamine (CAS No. 63738-22-7) (epoxy equivalent weight (EEW): 95-110 g/eq; specific gravity at 25 °C: 1.14- 1.16 g/ml; flash point: >215 °C; colour (Gardner): max. 5; viscosity at 25 °C: 1600- 3000 cP) supplied by CVC Thermoset Specialties (now HUNSTMAN). The Cardolite® NC-514S is a di-functional glycidyl ether epoxy resin (see Scheme 3) [reddish brown liquid; color (Gardner): $\leq$18 (ASTM D1544); viscosity at 25 °C: 1000- 3000 cP (ASTM D2196); epoxy equivalent weight (EEW): 320-420 (ASTM D1652-97); hydrolysable chlorine (%): $\leq$ 0.5 (ASTM D1726-11); volatile loss (%wt): $\leq$ 0.5 (ASTM D2369-98); density at 25 °C: 1.026 Kg/L (ASTM D1475); flash point: > 205 °C (ASTM D93)] was supplied by Cardolite Corporation. The EPPALOY® 9000 is tris hydroxyl phenyl ethane (CAS No. 87093-13-8) (see Scheme 4) [average epoxy functionality: 3.0; epoxy equivalent weight (EEW): 160-180 g/eq; viscosity at 72 °C: 5500-6500 cP; colour (Gardner): max. 2) supplied by CVC Thermoset Specialties (now HUNSTMAN]. The bisphenol A diglycidyl ether (CAS No. 1675-54-3) was supplied from Tokyo Chemical Industry Co., Ltd. The *N*,*N*-diglycidyl-4-glycidyloxyaniline (CAS No. 5026-74-4) was supplied by Sigma-Aldrich. Mehtylethylketone was supplied by Sigma-Aldrich. The polypropylene glycol with a calculated number average molecular weight ($M_n$) of 2000 Da and an OH-value of 56$\pm$2 mg KOH/g polypropylene glycol), and the polypropylene glycol with a calculated number average molecular weight ($M_n$) of 1000 Da and an OH-value of 112$\pm$2 mg KOH/g polypropylene glycol, were supplied by DOW. The DOWANOL™ DPM Glycol Ether [dipropylene glycol mono methyl ether; CAS No.: 34590-94-8; boiling

point 190 °C at 760 mmHg; density 0.948 g/mL; flash point (closed cup): 75 °C; freezing point: -83 °C; molecular weight: 148.2 Da; specific gravity (25 °C): 0.951; surface tension: 28 dynes/cm; viscosity (25 °C): 3.7 cP; vapor pressure (20 °C): 0.28 mmHg; solubility in water (25 °C): infinite (wt%)] is a (mid- to slow evaporating) organic solvent. Any other chemical that is not explicitly mentioned in this paragraph and used in the Examples section (and unless otherwise stated in the specification) has been supplied by Sigma-Aldrich.

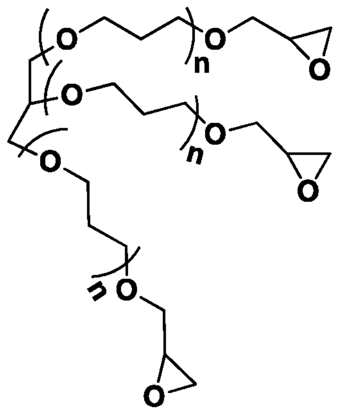

Scheme 1

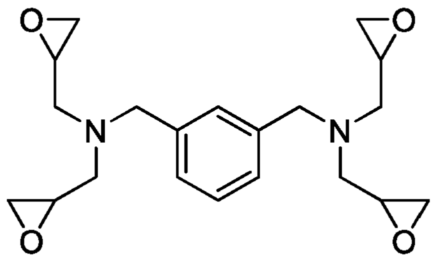

Scheme 2

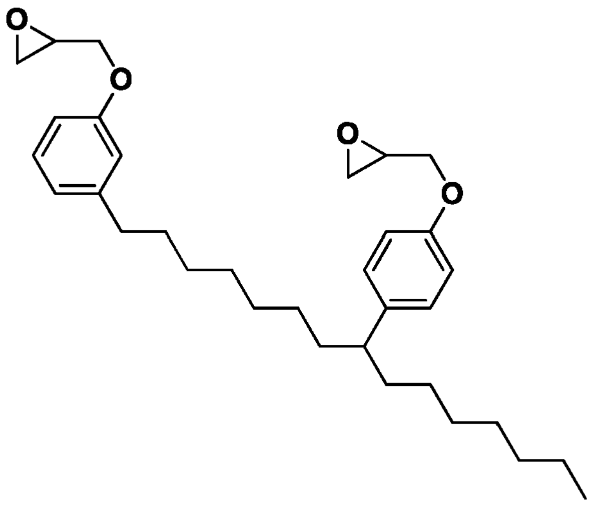

Scheme 3

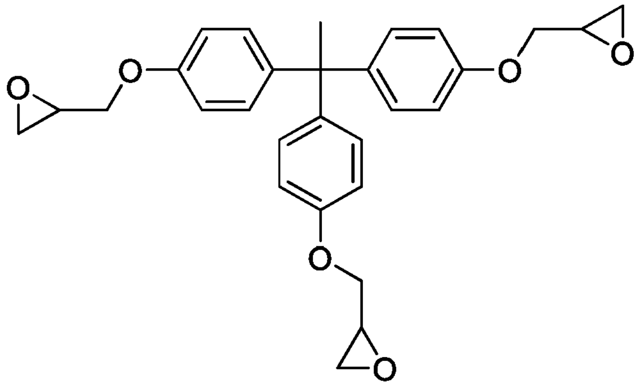

Scheme 4

**1.2 Preparation of the polyurethane A and its aqueous dispersion** (the latter is abbreviated as 'polyurethane A-AQD')

A one-litre flask (equipped with a thermometer and an overhead stirrer), was charged with 29.9 grams of dimethylol propionic acid, 282.1 grams of a polypropylene glycol with a calculated number average molecular weight ($M_n$) of 2000 Da and an OH-value of 56±2 mg KOH/g polypropylene glycol), 166.5 grams of a polypropylene glycol with a calculated number average molecular weight ($M_n$) of 1000 Da and an OH-value of 112±2 mg KOH/g polypropylene glycol, and 262.8 grams of isophorone diisocyanate (the number average molecular weight of each of the polyols is calculated from its OH-value according to the equation: $M_n$= 2*56100/[OH-value in mg KOH/g polypropylene glycol). The reaction mixture was placed under $N_2$ atmosphere, heated to 50 °C and subsequently, 0.07 g dibutyltin dilaurate were added to the reaction mixture. An exothermic reaction was observed; however, proper care was taken in order for the reaction temperature not to exceed 97 °C. The reaction was maintained at 95 °C for an hour. The NCO content of the resultant polyurethane A was 7.00% on solids determined according to the ISO 14896 Method A (year 2009) (theoretically 7.44%), and the acid value of the polyurethane A was 16.1±1 mg KOH/g polyurethane A. The polyurethane A was cooled down to 60 °C, and 18.7 grams of triethylamine were added, and the resulting mixture was stirred for 30 minutes. Subsequently, an aqueous dispersion of the polyurethane A (abbreviated as 'polyurethane A- AQD') was prepared as follows: the thus prepared mixture of the polyurethane A and triethylamine was fed -at room temperature over a time period of 60 minutes- to a mixture of 1100 grams of demineralized water, 19.5 grams of nonylphenol ethoxylate (9 ethoxylate groups), and 4.0 grams of triethylamine. After the feed was completed, the mixture was stirred for additional 5 minutes, and subsequently, 111.2 grams of hydrazine (16 wt% solution in water) were added to the mixture. The aqueous dispersion of the polyurethane A thus prepared was stirred for an additional 1 h.

**1.2 Determination of the molecular weight of the AZ1- to AZ5-compounds and of the component T (Matrix-assisted laser desorption/ionization on a time-of-flight mass spectrometry; MALDI-TOF MS)**

All MALDI-ToF-MS spectra were acquired using a Bruker UltrafleXtreme™ MALDI-ToF mass spectrometer. The instrument is equipped with a Nd:YAG laser emitting at 1064 nm and a collision cell (not used for these samples). Spectra were acquired in the positive-ion mode using the reflectron, using the highest resolution mode providing accurate masses (range 60-7000 m/z). Cesium Tri-iodide (range 0.3-3.5 kDa) was used for mass calibration (calibration method: IAV Molecular Characterization, code MC-MS-05). The laser energy was 20%. The samples were dissolved in THF at approx. 50 mg/mL. The matrix used was: DCTB (trans-2-[3-(4-tert-butylphenyl)-2-methyl-2-propenylidene]malononitrile), CAS Number 300364-84-5. The matrix solution was prepared by dissolving 20 mg in 1 mL of THF. Potassium trifluoroacetate (KTFA, CAS Number: 2923-16-2) or alternatively sodium iodide was used as salt (NaI, CAS Number 7681-82-5); 10 mg was dissolved in 1 ml THF with a drop of MeOH added. Ratio sample:matrix:salt = 10:200:10 (μL), after mixing, 0.5 μL was spot on MALDI plate and 20 allowed to air-dry. Reported signals are the major peaks within 0.5 Da of the calculated mass of the multi-aziridine compounds which are theoretically present in the composition in the largest amounts. In all cases, the reported peaks are the sodium or potassium adducts of the measured ions. The MALDI-TOF MS signals reported correspond to the major peaks of the sodium or potassium adducts of the measured ions of the theoretical formula of a multi-aziridine compound (the sodium and potassium cations. The theoretical formula of a multi-aziridine compound may be determined via analytical techniques well-known to one skilled in the art of analytical chemistry, e.g. NMR spectroscopy and/or liquid chromatography-mass spectroscopy (LC-MS), and/or liquid chromatography-mass spectroscopy-mass spectroscopy (LC-MS-MS), and/or theoretically from its method of preparation (if reactants and conditions are known); once the theoretical formula of a multi-aziridine compound is determined, then its theoretical molecular weight may also be determined.

The multi-aziridine compounds are identified by comparing the molecular weight which is defined just below (MW), with the exact molecular mass (i.e. the sum of the non-isotopically averaged atomic masses of its constituent atoms) of a theoretical structure, using a maximum deviation of 0.5 Da. In the context of this specification, the molecular weight (MW) attributed to a multi-aziridine compound is calculated from the following equation:

$$MW = Obs.\ [M + M_{cation}] - [M_{cation}]$$

wherein

the $M_{cation}$ is the exact molar mass of the sodium (22.99 Da), or potassium cation (38.96 Da) (depending on which cation was used in the MALDI-TOF MS method), and
the Obs. $[M + M_{cation}]$ is the MALDI-TOF MS signal (peak) which corresponds to the theoretical formula of the multi-aziridine compound.

**1.3 Determination of the genotoxicity (ToxTracker® assay)**

The genotoxicity was determined according to the ToxTracket® assay (Toxys, Leiden, the Netherlands) as described below. The ToxTracker® assay can be applied for pure substances or for compositions which are the direct products obtained in the preparation of compounds bearing the aziridine ring.
The ToxTracker assay is a panel of several validated Green Fluorescent Protein (GFP)-based mouse embryonic stem (mES) reporter cell lines that can be used to identify the biological reactivity and potential carcinogenic properties of newly developed compounds in a single test. This methodology uses a two-step approach.
In the first step a dose range-finding was performed using wild-type mES cells (strain B4418). 20 different concentrations for each compound was tested, starting at 10 mM in DMSO as the highest concentration and nineteen consecutive 2-fold dilutions. Next, the genotoxicity of samples (inventive and comparative examples) was evaluated using specific genes linked to reporter genes for the detection of DNA damage; i.e. Bscl2 (as elucidated by US9695481B2 and EP2616484B1) and Rtkn (Hendriks et al. Toxicol. Sci. 2015, 150, 190-203) biomarkers. Genotoxicity was evaluated at 10, 25 and 50% cytotoxicity in the absence and presence of rat S9 liver extract-based metabolizing systems (aroclor1254-induced rats, Moltox, Boone, NC, USA). The independent cell lines were seeded in 96-well cell culture plates, 24 h after seeding the cells in the 96-well plates, fresh ES cell medium containing the diluted test substance was added to the cells. For each tested compound, five concentrations are tested in 2-fold dilutions. The highest sample concentration will induce significant cytotoxicity (50-70%). In case of no or low cytotoxicity, 10 mM or the maximum soluble mixture concentration is used as maximum test concentration. Cytotoxicity is determined by cell count after 24 h exposure using a Guava easyCyte 10HT flow cytometer (Millipore). GFP reporter induction is always compared to a vehicle control treatment. DMSO concentration is similar in all wells for a particular compound and never exceeds 1%. All compounds were tested in at least three completely independent repeat experiments. Positive reference treatment with cisplatin (DNA damage) was included in all experiments. Metabolic was evaluated by the addition of S9 liver extract. Cells are exposed to five concentrations of the test compound in the presence of S9 and required co-factors (Regen-SysA+B, Moltox, Boone, NC, USA) for 3 h. After washing, cells are incubated for 24 h in fresh ES cell medium. Induction of the GFP reporters is determined after 24 h exposure using a Guava easyCyte 10HT flow cytometer (Millipore). Only GFP expression in single intact cells is determined. Mean GFP fluorescence and cell concentrations in each well are measured, which is used for cytotoxicity assessment. Data were analyzed using ToxPlot software (Toxys, Leiden, the Netherlands). The induction levels reported are at compound concentrations that induce 10%, 25% and 50% cytotoxicity after 3 h exposure in the presence of S9 rat liver extract and 24 h recovery or alternatively after 24 h exposure when not in the presence of S9 rat liver extract. A positive induction level of the biomarkers is defined as equal to or higher than a 2-fold induction in at least one of 10, 25 and 50% cytotoxicity in the absence or presence of the metabolizing system rat S9 liver extract; a weakly positive induction as higher than 1.5-fold and lower than 2-fold induction in at least one of 10, 25 and 50% cytotoxicity (but lower than 2-fold at 10, 25 and 50% cytotoxicity) in the absence or presence of the metabolizing system rat S9 liver extract and a negative as lower than or equal to a 1.5-fold induction at 10, 25 and 50% cytotoxicity in the absence and presence of rat S9 liver extract-based metabolizing systems. In the context of the specification by 'non-genotoxic' (or equally 'not genotoxic') is meant that the induction level of the biomarkers Bscl2-GFP and Rtkn-GFP is lower than 2-fold -preferably equal to or lower than 1.9-fold, more preferably equal to or lower than 1.8-fold, for example equal to or lower than 1.7-fold, for example equal to or lower than 1.6-fold for example equal to or lower than 1.5-fold at 10, 25 and 50% cytotoxicity in the absence and presence of rat S9 liver extract-based metabolizing systems (aroclor1254-induced rats, Moltox, Boone, NC, USA).

### 1.4 Assessment of the crosslinking efficiency

The crosslinking efficiency of organic compounds bearing aziridine ring(s) in liquid compositions [or equally the crosslinking efficiency of liquid compositions comprising organic compounds bearing aziridine ring(s)] was assessed by determining the chemical resistance of films (cured coatings).

The chemical resistance of a film (cured coating) was tested based on the DIN 68861-1:2011-01. The film was prepared as follows: 0.43 parts of the composition were mixed with 0.57 parts of DOWANOL™ DPM Glycol Ether and incubated at 80°C for 10 minutes under regular agitation. Subsequently, an amount of the resulting solution was added under continuous stirring to an amount of the polyurethane A-AQD, and the resulting mixture was stirred for additional 30 minutes to thus produce a liquid composition. These amounts were calculated on the basis that the molar ratio of the mol of aziridine rings present in the organic compound (or a mixture of organic compounds) bearing aziridine ring(s), e.g. AZ-component to the mol of carboxylic acid functional groups present in the polyurethane A was equal to 0.9. This liquid composition was filtered and subsequently applied onto Leneta test cards using a 100 μm wire rod applicator. The film was dried for 16 h at 25 °C, then annealed at 50 °C for 1 h and subsequently dried for 24 h at 25 °C.

Cotton wool pads (1x1 cm) were soaked in a solution of ethanol: demineralized water (1:1). They were then placed on the films and covered with Petri dishes for 240 minutes. Afterwards, the pads and the Petri dishes were removed; after 1 h the coatings were visually inspected for damages; the extent of damages was assessed according to the following rating scheme:

5: no visible changes
4: hardly noticeable changes in shine or colour
3: slight changes in shine or colour; the structure of the test surface has not changed
2: heavy changes noticeable; however, the structure of the test surface has remained more or less undamaged.
1: heavy changes noticeable; the structure of the test surface has changed.
0: the tested surface was heavily changed or destroyed.

In the context of this specification, the above integers 0-5 are mentioned as 'ranking points'.

The chemical resistance of a reference film (prepared from only polyurethane A- AQD) was poor (this applies for all examples inventive and comparatives shown in the Examples).

### 1.5 Determination of the pH

The pH of a sample was determined according to the ISO 976:2013. Samples were measured at room temperature using a Metrohm 691 pH-meter equipped with a combined glass electrode and a PT-1000 temperature sensor. The pH-meter was calibrated using buffer solutions of pH 7.00 and 9.21 prior to use.

### 1.6 Determination of the acid value

The acid value of a polymer is determined according to the ASTM D1639-90(1996)e1. According to the procedure, the sample was dissolved in a good solvent, was titrated with alcoholic potassium hydroxide solution of a known concentration (KOH). The difference in titration volume between the sample and a blank is the measure of the acid value on solids, according to the following formula:

$$AV=[(V_{blank} - V_{sample}) * N_{KOH} * 56.1] / (W * S /100)$$

where
AV is the acid number on solids in mg KOH/g solid material, $V_{blank}$ is the volume of KOH solution used in the blank, $V_{sample}$ is the volume of KOH solution used in the sample, $N_{KOH}$ is the normality of the KOH solution, W is the sample weight in grams and S is the solids content of the sample in %. Measurements are performed in duplicate using a potentiometric endpoint on a Metrohm 702SM Titrino titrator (accepting the measurement if the difference between duplicates is < 0.1 mg KOH/g solid material).

### 1.7 Determination of the NCO content

The NCO content of a sample is determined based on the ASTM D2572-19 standard. In the procedure, the sample is reacted with excess n-dibutylamine. The excess of n-dibutylamine is subsequently back-titrated with standard 1N hydrochloric acid (HCl). The difference in titration volume between the sample and a blank is the measure of the

isocyanate content on solids, according to the following formula:

$$\%NCO_{solids}=[(V_b - V_m) * N * 4.2] / (A * s /100)$$

where
$\%NCO_{solids}$ is the isocyanate content on solids, $V_b$ is the volume of HCl used in the blank, $V_m$ is the volume of HCl used in the sample, N is the normality of the HCl solution, A is the sample weight in grams and s is the solids content of the sample in %. Measurements are performed in duplicate using a potentiometric endpoint on a Metrohm 702SM Titrino titrator (accepting the measurement if the difference between duplicates is < $0.1\%_{NCO}$).

**1.8 Determination of the amount of component T [Liquid Chromatography coupled with Mass Spectroscopy (LC-MS)]**

The amount of component T (as the latter is defined in the specification including the claims; wt% on the total weight of the AZ-compound) was determined via liquid chromatography coupled with mass spectroscopy (LC-MS). Initially, a 0.01 wt% solution of sample in methanol was prepared. Subsequently, 0.5 μL of this solution was injected into an Agilent 1290 Infinity II Ultra High Pressure Liquid Chromatography (UHPLC) system equipped with: a) a High Strength Silica (HSS) technology C18 type T3 column supplied by Waters® [100 x 2.1 mm (length x diameter); 1.8 micron average particle size of the stationary phase] operating at 40 °C, and b) an Agilent 6550 iFunnel QTOF detector [ElectroSpray Ionization-Time-of-Flight Mass Spectrometer (ESI-TOF-MS) detector]. Once the sample was injected, then a gradient of a mobile phase [from 80/20 v/v A/B to 1/99 v/v A/B, wherein A was 10 mM $CH_3COO^-NH_4^+$ (set to pH 9.0 with $NH_3$) and B was acetonitrile; A and B making up the mobile phase], at a flow rate of 0.5 mL/min, for 10 min, was used to separate the various ingredients of the sample. Subsequently, a gradient of a mobile phase [from 1/99 v/v A/B to 1/49/50 A/B/C, wherein A was 10 mM $CH_3COO^-NH_4^+$ (set to pH 9.0 with $NH_3$), B was acetonitrile and C was tetrahydrofuran (THF); A, B and C making up the mobile phase] at a flow rate of 0.5 mL/min, for 5 min was applied to purge the column. Assuming a linear MS response of all the ingredients of the sample over all response ranges and an equal ionization efficiency for all the ingredients of the sample, the signals of the:

- total ion current, and
- extracted ion chromatograms of the component T,

were integrated.

The data acquisition was carried out via the MassHunter Build 10.1.48 software supplied by Agilent, while the data processing was carried out via the Qualitative Analysis Build 10.0.10305.0 software, also supplied by Agilent.

The amount of component T (wt% on the total weight of the AZ-compound) was determined by dividing the integrals of the extracted ion chromatograms of the component T by the integrals of the total ion current, multiplied by 100.

**1.9 Determination of the amount of chloride**

The amount of chloride in a liquid composition was determined according to the ASTM D1726-11(2019).

**2 Inventive Examples**

Example 1

100 grams of ERISYS® GE-36, 26.5 grams of propylene imine and 5 grams of potassium carbonate were charged to a reaction flask equipped with a thermometer and a condenser connected to a cryostat (3 °C). The mixture was stirred with a mechanical upper stirrer under a nitrogen atmosphere. The mixture was then heated to 80 °C. Samples were taken at regular intervals, and the reaction progress was monitored using a $^1$H-NMR until no signal (chemical shifts) correlated to the epoxide protons from the multifunctional epoxide was observed. The reaction mixture was diluted with toluene and filtered. The solvent and the excess of propylene imine were removed from the filtrate in vacuo to obtain from the filtrate was removed *in vacuo* to obtain a clear highly viscous liquid. The theoretical formula of the thus prepared (aziridinyl hydroxy)-functional organic compound is shown below (wherein x+y+z = 33), and the theoretical molecular weight was calculated to be 2346.68 Da.

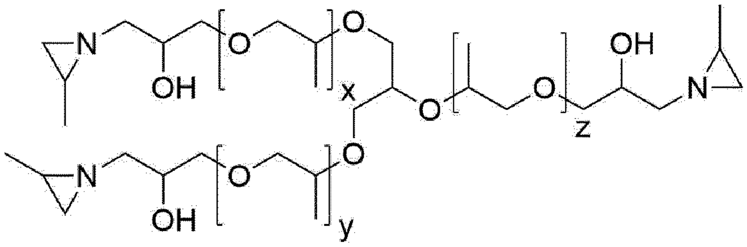

The molecular weight as defined and determined via MALDI-TOF MS -as this is defined and described in the specification- was 2346.50 Da (=Obs. [M + $M_{Na+}$] - [$M_{Na+}$]).

The crosslinking efficiency of the (aziridinyl hydroxy)-functional organic compound obtained above (and its liquid composition) was very good, and given the ToxTracker® assay results on the genotoxicity shown in the table below, this (aziridinyl hydroxy)-functional organic compound was not genotoxic.

| | **Without S9 rat liver extract** | | | | | | **With S9 rat liver extract** | | | | | |
|---|---|---|---|---|---|---|---|---|---|---|---|---|
| | Bscl 2 | | | Rtkn | | | Bscl 2 | | | Rtkn | | |
| **concentration** | 10 | 25 | 50 | 10 | 25 | 50 | 10 | 25 | 150 | 10 | 25 | 50 |
| **-fold induction** | 1.1 | 1.2 | 1.2 | 1.1 | 1.3 | 1.1 | 1.1 | 1.2 | 1.1 | 1.2 | 1.3 | 1.1 |

Example 2

75 grams of EPALLOY® 9000 was dissolved in 75 mL of toluene and charged to a reaction flask equipped with a thermometer and a condenser connected to a cryostat (3 °C). Next, 55 grams of propylene imine and 5 grams of potassium carbonate were added to the flask. The mixture was stirred with a mechanical upper stirrer under a nitrogen atmosphere. The mixture was than heated to 80 °C. Samples were taken at regular intervals and the reaction progress was monitored using a $^1$H-NMR until no signal (chemical shifts) correlated to the epoxide protons from the multifunctional epoxide was observed. After 22 hours of reaction, the mixture was filtered. The solvent and excess of propylene imine were removed from the filtrate in vacuo to obtain an off-white solid. The theoretical formula of the thus prepared (aziridinyl hydroxy)-functional organic compound is shown below and the theoretical molecular weight was calculated to be 645.38 Da.

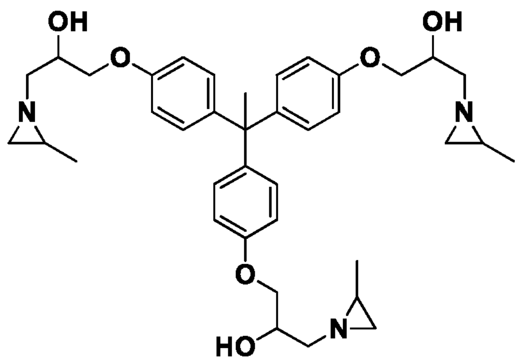

The molecular weight as defined and determined via MALDI-TOF MS -as this is defined and described in the specification- was 645.37 Da (=Obs. [M + $M_{Na+}$] - [$M_{Na+}$]).

The crosslinking efficiency of the (aziridinyl hydroxy)-functional organic compound obtained above (and its liquid composition) was good, and given the ToxTracker® assay results on the genotoxicity shown in the table below, this (aziridinyl hydroxy)-functional organic compound was not genotoxic.

| | **Without S9 rat liver extract** | | | | | | **With S9 rat liver extract** | | | | | |
|---|---|---|---|---|---|---|---|---|---|---|---|---|
| | Bscl 2 | | | Rtkn | | | Bscl 2 | | | Rtkn | | |
| **concentration** | 10 | 25 | 50 | 10 | 25 | 50 | 10 | 25 | 50 | 10 | 25 | 50 |

(continued)

| | Without S9 rat liver extract | | | | | | With S9 rat liver extract | | | | | |
|---|---|---|---|---|---|---|---|---|---|---|---|---|
| | Bscl 2 | | | Rtkn | | | Bscl 2 | | | Rtkn | | |
| **-fold induction** | 1.1 | 1.4 | 1.5 | 1.1 | 1.3 | 1.3 | 1.1 | 1.3 | 1.7 | 1.2 | 1.5 | 1.4 |

Example 3

130 grams of DESMODUR® N 3600 was dissolved in 130 grams of toluene, charged to a reaction flask equipped with a thermometer and heated to 50 °C. Next, 0.05 grams of triethylamine was added to the flask and over the course of 90 minutes 52 grams of glycidol was slowly added to the reaction mixture, while making sure that the reaction temperature stayed constant between 55-58 °C. After stirring for another 60 minutes at this temperature, the mixture was cooled down to room temperature and stirred for 18 hours at room temperature. The solvent was removed *in vacuo* and the resulting yellowish oil was transferred to a reaction flask equipped with a thermometer and a condenser connected to a cryostat (3 °C). Next, 200 grams of toluene, 150 grams of propylene imine and 5 grams of potassium carbonate were added to the flask. The mixture was stirred with a mechanical upper stirrer under a nitrogen atmosphere. The mixture was than heated to 60 °C. Samples were taken at regular intervals and the reaction progress was monitored using a $^1$H-NMR until no signal (chemical shifts) correlated to the epoxide protons from the multifunctional epoxide was observed. After 18 hours of reaction, the mixture was filtered. The solvent and excess of propylene imine were removed from the filtrate in vacuo to obtain a high viscous liquid. The theoretical formula of the thus prepared (aziridinyl hydroxy)-functional organic compound is shown below and the theoretical molecular weight was calculated to be 897.55 Da.

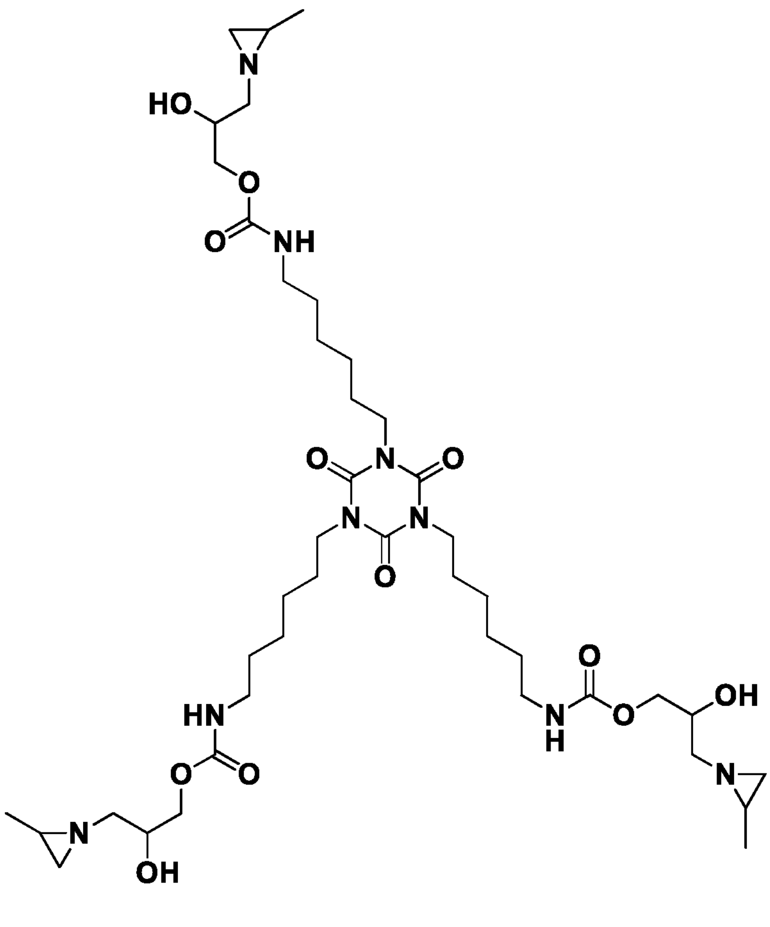

The molecular weight as defined and determined via MALDI-TOF MS -as this is defined and described in the specification- was 897.58 Da (=Obs. [M + $M_{Na+}$] - [$M_{Na+}$]).

The crosslinking efficiency of the (aziridinyl hydroxy)-functional organic compound obtained above (and its liquid composition) was good, and given the ToxTracker® assay results on the genotoxicity shown in the table below,

this (aziridinyl hydroxy)-functional organic compound was not genotoxic.

| | Without S9 rat liver extract | | | | | | With S9 rat liver extract | | | | | |
|---|---|---|---|---|---|---|---|---|---|---|---|---|
| | Bscl 2 | | | Rtkn | | | Bscl 2 | | | Rtkn | | |
| **concentration** | 10 | 25 | 50 | 10 | 25 | 50 | 10 | 25 | 50 | 10 | 25 | 50 |
| **-fold induction** | 1.1 | 1.3 | 1.5 | 1.2 | 1.7 | 1.9 | 1.2 | 1.5 | 1.5 | 1.1 | 1.5 | 1.7 |

Example 4

30.0 grams of Cardolite® NC-514S, 19.2 grams of propylene imine, 30 grams of toluene and 3 grams of potassium carbonate were charged to a reaction flask equipped with a thermometer and a condenser connected to a cryostat (3 °C). The mixture was stirred with a mechanical stirrer under a nitrogen atmosphere. The mixture was than heated to 70°C. Samples were taken at regular intervals and the reaction progress was monitored using a $^1$H-NMR until no signal (chemical shifts) correlated to the epoxide protons from the multifunctional epoxide was observed. After 20 hours of reaction, the mixture was diluted with toluene and filtered. The solvent and the excess of propylene imine were removed from the filtrate in vacuo to obtain to obtain a high viscous brownish oil. The theoretical formula of the thus prepared (aziridinyl hydroxy)-functional organic compound is shown below and the theoretical molecular weight was calculated to be 622.47 Da.

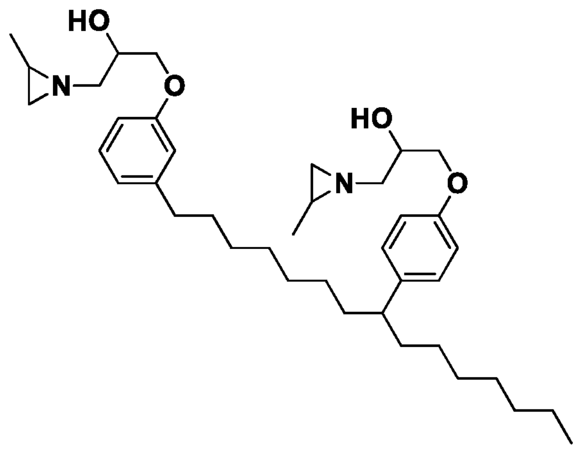

The molecular weight as defined and determined via MALDI-TOF MS -as this is defined and described in the specification- was 622.43 Da (=Obs. [M + $M_{Na+}$] - [$M_{Na+}$]).

The crosslinking efficiency of the (aziridinyl hydroxy)-functional organic compound obtained above (and its liquid composition) was good, and given the ToxTracker® assay results on the genotoxicity shown in the table below, this (aziridinyl hydroxy)-functional organic compound was not genotoxic.

| | Without S9 rat liver extract | | | | | | With S9 rat liver extract | | | | | |
|---|---|---|---|---|---|---|---|---|---|---|---|---|
| | Bscl 2 | | | Rtkn | | | Bscl 2 | | | Rtkn | | |
| **concentration** | 10 | 25 | 50 | 10 | 25 | 50 | 10 | 25 | 50 | 10 | 25 | 50 |
| **-fold induction** | 1.2 | 1.3 | 1.3 | 1.2 | 1.4 | 1.7 | 1.3 | 1.5 | 1.5 | 1.6 | 1.8 | 1.7 |

**3. Comparative examples**

Example 1C

The trimethylolpropane tris(2-methyl-1-aziridinepropionate) (commercial product name ‘crosslinker CX-100’) has the following structure:

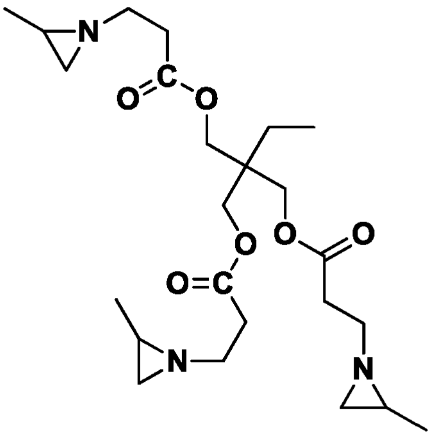

The theoretical molecular weight was calculated to be 467.30 Da.

The crosslinking efficiency of the trimethylolpropane tris(2-methyl-1-aziridinepropionate) (and its liquid composition) was very good and given the ToxTracker® assay results on the genotoxicity shown in the table below, the trimethylolpropane tris(2-methyl-1-aziridinepropionate) was genotoxic.

| | **Without S9 rat liver extract** | | | | | | **With S9 rat liver extract** | | | | | |
|---|---|---|---|---|---|---|---|---|---|---|---|---|
| | Bscl 2 | | | Rtkn | | | Bscl 2 | | | Rtkn | | |
| **concentration** | 10 | 25 | 50 | 10 | 25 | 50 | 10 | 25 | 50 | 10 | 25 | 50 |
| **-fold induction** | 1.2 | 1.5 | 2.0 | 1.4 | 2.0 | 3.2 | 1.7 | 2.3 | 2.1 | 3.0 | 4.3 | 3.4 |

Example 2C

75 grams of ERISYS® GA-240, 80 grams of propylene imine and 5 grams of potassium carbonate were charged to a reaction flask equipped with a thermometer and a condenser connected to a cryostat (3 °C). The mixture was stirred with a mechanical upper stirrer under a nitrogen atmosphere. The mixture was than heated to 71 °C. Samples were taken at regular intervals and the reaction progress was monitored using a $^1$H-NMR until no signal (chemical shifts) correlated to the epoxide protons from the multifunctional epoxide was observed. After 22 hours of reaction, the mixture was diluted with toluene and filtered. The solvent and the excess of propylene imine were removed from the filtrate in vacuo to obtain a clear, transparent solid. The theoretical formula of the thus prepare mutli-aziridine compound is shown below and the theoretical molecular weight was calculated to be 588.44 Da.

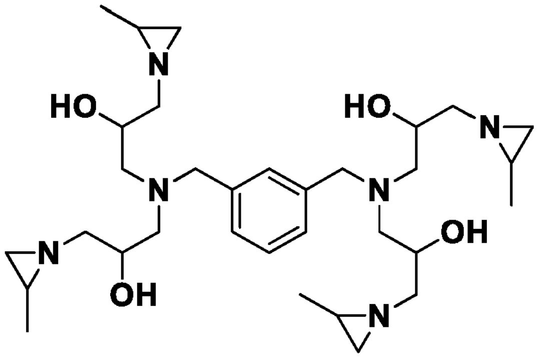

The molecular weight as defined and determined via MALDI-TOF MS -as this is defined and described in the specification- was 588.48 Da (=Obs. [M + $M_{Na+}$] - [$M_{Na+}$]).

The crosslinking efficiency of the mutli-aziridine compound obtained above (and its liquid composition) was good, and given the ToxTracker® assay results on the genotoxicity shown in the table below, this multi-aziridine compound was genotoxic.

| | Without S9 rat liver extract | | | | | | With S9 rat liver extract | | | | | |
|---|---|---|---|---|---|---|---|---|---|---|---|---|
| | Bscl 2 | | | Rtkn | | | Bscl 2 | | | Rtkn | | |
| **concentration** | 10 | 25 | 50 | 10 | 25 | 50 | 10 | 25 | 50 | 10 | 25 | 50 |
| **-fold induction** | 1.3 | 1.8 | 2.6 | 1.6 | 2.5 | 3.9 | 1.3 | 1.7 | 2.5 | 1.8 | 3.0 | 5.1 |

Example 3C

24.0 grams of N,N-dilycidyl-4-glycidyloxyaniline, 42.0 grams of propylene imine, 30 grams of toluene and 3 grams of potassium carbonate were charged to a reaction flask equipped with a thermometer and a condenser connected to a cryostat (3 °C). The mixture was stirred with a mechanical stirrer under a nitrogen atmosphere. The mixture was than heated to 70°C. Samples were taken at regular intervals and the reaction progress was monitored using a $^1$H-NMR until no signal (chemical shifts) correlated to the epoxide protons from the multifunctional epoxide was observed. After 24 hours of reaction, the mixture was diluted with toluene and filtered. The solvent and the excess of propylene imine were removed from the filtrate in vacuo to obtain a highly viscous yellow material. The theoretical formula of the thus prepare mutli-aziridine compound is shown below and the theoretical molecular weight was calculated to be 448.30 Da.

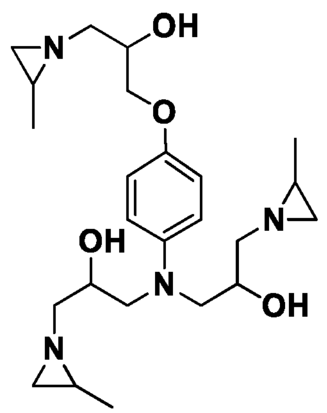

The molecular weight as defined and determined via MALDI-TOF MS -as this is defined and described in the specification- was 448.29 Da (=Obs. [M + $M_{Na+}$] - [$M_{Na+}$]).

This compound was disclosed in US 3329674 in cl. 2, II. 29-39.

The crosslinking efficiency of the mutli-aziridine compound obtained above (and its liquid composition) was good, and given the ToxTracker® assay results on the genotoxicity shown in the table below, this multi-aziridine compound was genotoxic.

| | Without S9 rat liver extract | | | | | | With S9 rat liver extract | | | | | |
|---|---|---|---|---|---|---|---|---|---|---|---|---|
| | Bscl 2 | | | Rtkn | | | Bscl 2 | | | Rtkn | | |
| **concentration** | 10 | 25 | 50 | 10 | 25 | 50 | 10 | 25 | 50 | 10 | 25 | 50 |
| **-fold induction** | 1.1 | 1.3 | 1.7 | 1.7 | 2.3 | 3.3 | 1.2 | 1.4 | 2.2 | 1.8 | 2.6 | 5.1 |

Example 4C

299 grams of bisphenol A diglycidyl ether, 250 grams of toluene, 255 grams of propylene imine and 10 grams of potassium carbonate were charged to a reaction flask equipped with a thermometer and a condenser connected to a cryostat (3 °C). The mixture was stirred with a mechanical upper stirrer under a nitrogen atmosphere. The mixture was than heated to 70°C. Samples were taken at regular intervals and the reaction progress was monitored using a $^1$H-NMR until no signal (chemical shifts) correlated to the epoxide protons from the multifunctional epoxide was observed. After 19 hours of reaction, the mixture was filtered. The solvent and the excess of propylene imine were removed from the filtrate in vacuo to obtain a highly viscous liquid. The theoretical formula of the thus prepare mutli-aziridine compound is shown below and the theoretical molecular weight was calculated to be 454.28 Da.

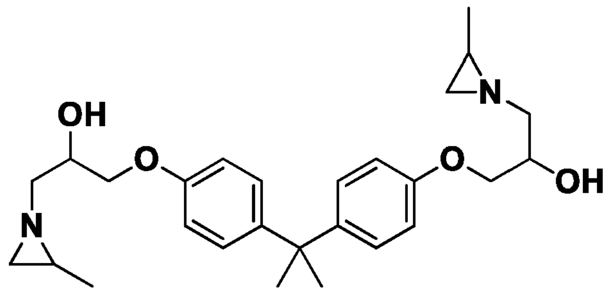

The molecular weight as defined and determined via MALDI-TOF MS -as this is defined and described in the specification- was 454.26 Da (=Obs. [M + $M_{Na+}$] - [$M_{Na+}$]).
The crosslinking efficiency of the mutli-aziridine compound obtained above (and its liquid composition) was good, and given the ToxTracker® assay results on the genotoxicity shown in the table below, this multi-aziridine compound was genotoxic.

| | **Without S9 rat liver extract** | | | | | | **With S9 rat liver extract** | | | | | |
|---|---|---|---|---|---|---|---|---|---|---|---|---|
| | Bscl 2 | | | Rtkn | | | Bscl 2 | | | Rtkn | | |
| **concentration** | 10 | 25 | 50 | 10 | 25 | 50 | 10 | 25 | 50 | 10 | 25 | 50 |
| **-fold induction** | 1.1 | 1.2 | 1.9 | 1.0 | 1.9 | 2.8 | 1.1 | 1.2 | 2.1 | 1.1 | 2.0 | 3.1 |

**4. Conclusion**

The US 3329674 to Thiokol Chemical Corporation disclosed low molecular weight aziridinyl derivatives of polyfunctional epoxides. The molecular weight of these aziridinyl derivatives was well below 600 Da as explained in the specification. The US 3329674 did not disclose (aziridinyl hydroxy)-functional organic compounds having a molecular weight of at least 600 and at most 10000 Da. The US 3329674 failed to provide for (aziridinyl hydroxy)-functional organic compounds (and its liquid compositions) that would be non-genotoxic and have at least good crosslinking efficiency. Evidence for that is the Example 3C shown in the specification, which was genotoxic.
Upon comparing the results of the inventive Examples 1 to 4 and those of the comparative Examples 1C to 4C, it is evident that only the AZ-component of the invention (and the liquid compositions of the invention) were not genotoxic and had at least good crosslinking efficiency.

**Claims**

**1.** An (aziridinyl hydroxy)-functional organic component (AZ-component) selected from the group consisting of i) to vi): i) (aziridinyl hydroxy)-functional organic compound AZ1 of Formula A1 having only two aziridine rings (AZ1-compound), ii) (aziridinyl hydroxy)-functional organic compound AZ2 of Formula A2 having only three aziridine rings (AZ2-compound), iii) (aziridinyl hydroxy)-functional organic compound AZ3 of Formula A3 having only four aziridine rings (AZ3-compound), iv) (aziridinyl hydroxy)-functional organic compound AZ4 of Formula A4 having only five aziridine rings (AZ4-compound), v) (aziridinyl hydroxy)-functional organic compound AZ5 of Formula A5 having only six aziridine rings (AZ5-compound), and vi) mixtures thereof,

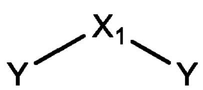

Formula A1

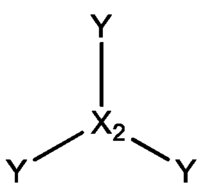

Formula A2

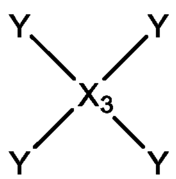

Formula A3

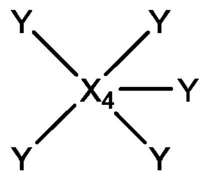

Formula A4

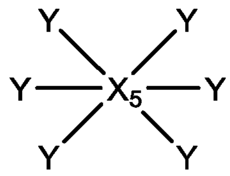

Formula A5

wherein

$X_1$ is a bivalent aliphatic organic radical or a bivalent aromatic organic radical, preferably $X_1$ is a bivalent aliphatic organic radical;
$X_2$ is a trivalent aliphatic organic radical or a trivalent aromatic organic radical, preferably $X_2$ is a trivalent aliphatic organic radical;
$X_3$ is a quadrivalent aliphatic organic radical or a quadrivalent aromatic organic radical, preferably $X_3$ is a quadrivalent aliphatic organic radical;
$X_4$ is a pentavalent aliphatic organic radical or a pentavalent aromatic organic radical, preferably $X_4$ is a pentavalent aliphatic organic radical;
$X_5$ is a hexavalent aliphatic organic radical or a hexavalent aromatic organic radical, preferably $X_5$ is a hexavalent aliphatic organic radical;
and wherein each of the $X_1$ to $X_5$ consists of a collection of atoms covalently connected in a configuration that comprises -preferably consists of- linear and/or branched and/or ring structures, which collection of atoms is selected from the group consisting of i) to x): i) carbon and hydrogen atoms, ii) carbon, hydrogen and oxygen atoms, iii) carbon, hydrogen and nitrogen atoms, iv) carbon, hydrogen and sulphur atoms, v) carbon, hydrogen, oxygen and nitrogen atoms, vi) carbon, hydrogen, nitrogen and sulphur atoms, vii) carbon, hydrogen, oxygen and sulphur atoms, viii) carbon, hydrogen, oxygen, nitrogen and sulphur, atoms, ix) carbon, hydrogen and silicon atoms, and x) carbon, hydrogen, oxygen and silicon atoms and xi) any combination of ix) and/or x) with any one or all of the iii) to viii),
and wherein each of the $X_1$ to $X_5$ has carbon atoms and hydrogen atoms,
and wherein each of the $X_1$ to $X_5$ has optionally oxygen atoms and/or nitrogen atoms and/or sulphur atoms and/or silicon atoms, and wherein the $X_1$, and/or the $X_2$ and/or the $X_3$ and/or the $X_4$ and/or the $X_5$ may optionally comprise an ionic functional group, wherein
Y is a monovalent organic radical selected from the group consisting of: i) monovalent (aziridinyl hydroxyisopropyl) organic radical of Formula B1, ii) monovalent (aziridinyl hydroxycyclohexane) organic radical of Formula B2, and iii) monovalent (aziridinyl hydroxycyclohexane) organic radical of Formula B3, preferably Y is a monovalent (aziridinyl hydroxyisopropyl) organic radical of Formula B1,

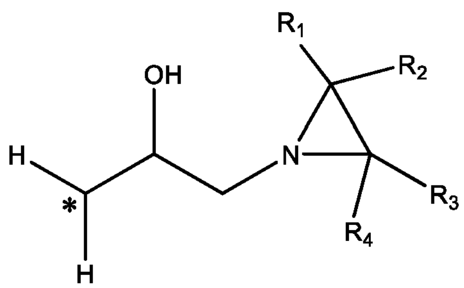

Formula B1

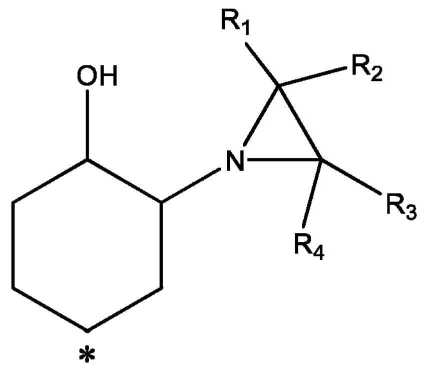

Formula B2

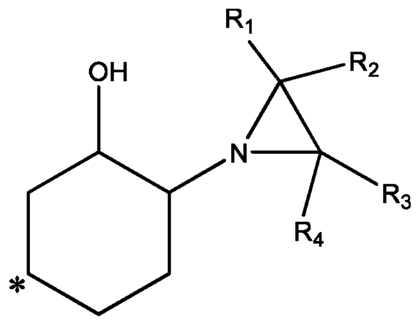

Formula B3

and wherein

$R_1$ is selected from the group consisting of hydrogen and methyl; and
$R_2$ is selected from the group consisting of hydrogen, methyl, and $C_2$-$C_5$ alkyl; and
$R_3$ is selected from the group consisting of methyl, and $C_2$-$C_4$ alkyl; and
$R_4$ is selected from the group consisting of hydrogen, methyl, and $C_2$-$C_4$ alkyl;

and wherein
the Y in each of the compounds A1 to A5 may be the same or different to each other, and wherein each of the single covalent bonds between the Y and each one of the $X_1$ to $X_5$ is selected from the group consisting of carbon-carbon single bond, and carbon-oxygen single bond, preferably each of the single covalent bonds between the Y and each one of the $X_1$ to $X_5$ is carbon-oxygen single bond,
and wherein
each of the AZ1- to AZ5-compounds has a molecular weight determined via MALDI-TOF MS according to the description, of at least 600 and at most 10000, preferably at least 600 and at most 8000, more preferably at least 600 and at most 6000, most preferably at least 600 and at most 5000, especially at least 600 and at most 4000, more especially at least 600 and at most 3500, most especially at least 600 and at most 3200, for example at least 600 and at most 3000, for example at least 600 and at most 2500 Da, and wherein
the $X_1$ and the $X_2$ and the $X_3$ and the $X_4$ and the $X_5$ does not contain one or any combination of the following structural units BP1, BP2, BP3, and BS

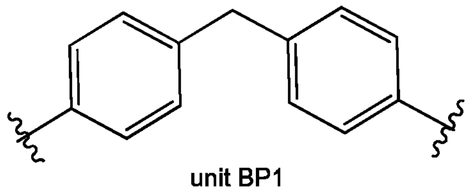

unit BP1

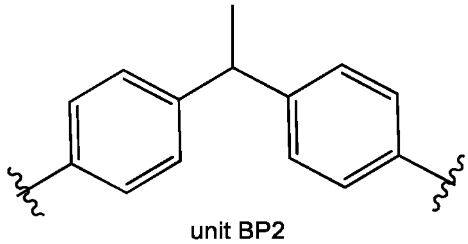

unit BP2

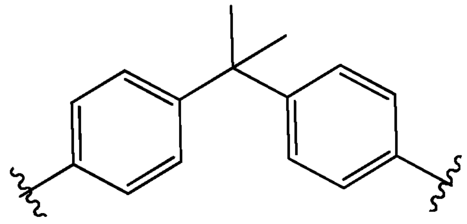

unit BP3

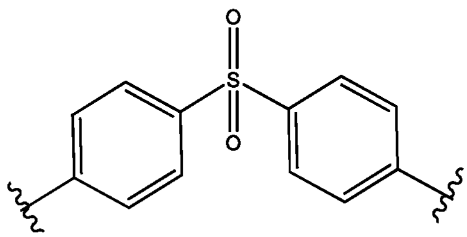

unit BS

2. The AZ-component according to claim 1, wherein the $X_1$, and/or the $X_2$ and/or the $X_3$ and/or the $X_4$ and/or the $X_5$ comprises at least one structural unit or a combination of structural units selected from the group consisting of unit 1, unit 2, unit 3, unit 4, unit 5, unit 6, unit 7, unit 8, unit 9, unit 10, and unit 11, as the units 1 to 11 are depicted below:

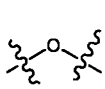

unit 1

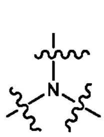

unit 2

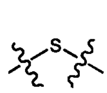

unit 3

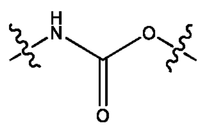

unit 4

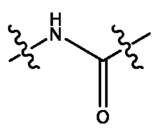

unit 5

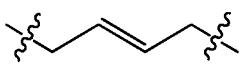

unit 6

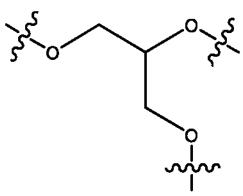

unit 7

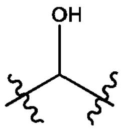

unit 8

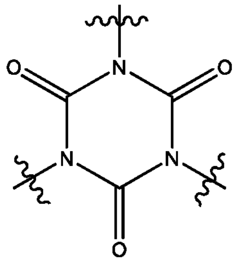

unit 9

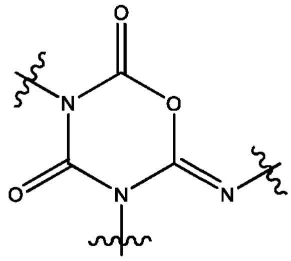

unit 10

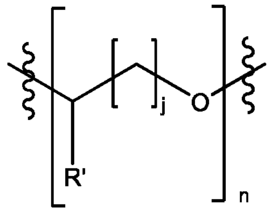

unit 11

wherein

R’ is selected from the group consisting of hydrogen and methyl; and
j is an integer ranging from 1 to 5, preferably from 1 to 3; and
n is an integer ranging from and including 2 up to and including 50.

**3.** The AZ-component according to any one of the preceding claims, wherein the aggregate number of carbon atoms in $R_1$, and $R_2$ and $R_3$ and $R_4$ is at most 9, preferably at most 4, more preferably at most 2, for example at most 1.

**4.** The AZ-component according to any one of the preceding claims, wherein the $X_1$ is the bivalent aliphatic organic radical of Formula A1a’

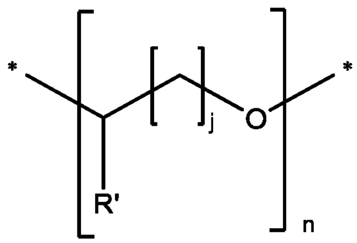

Formula A1a’

wherein

R’ is selected from the group consisting of hydrogen and methyl; and
j is an integer ranging from 1 to 5, preferably from 1 to 3; and
n is an integer ranging from and including 2 up to and including 50.

**5.** The AZ-component according to any one of the claims 1 to 4 wherein each of the $X_1$ to $X_5$ contains only single covalent bonds, or both single and double covalent bonds, and wherein the single covalent bonds are selected from the group consisting of carbon-carbon single bond, carbon-hydrogen single bond, carbon-nitrogen single bond, carbon-sulphur single bond, carbon-silicon single bond, silicon-oxygen single bond, nitrogen-hydrogen single bond, sulphur-oxygen single bond, carbon-oxygen single bond wherein the oxygen is bonded to a hydrogen forming a hydroxyl group, silicon-oxygen-silicon single bonds, and wherein the double covalent bonds are selected from the

group consisting of carbon-carbon double bond, carbon-nitrogen double bond, sulphur-oxygen double bond, carbon-oxygen double bond wherein the carbon is a member of a ring structure, and carbon-oxygen double bond wherein the carbon is bonded to another two carbons via carbon-carbon single bonds, carbon-oxygen double bond wherein the carbon is bonded to another oxygen via single bond and to a nitrogen via a single bond, carbon-oxygen double bond wherein the carbon is bonded to two nitrogens via carbon-nitrogen single bonds, carbon-oxygen double bond wherein the carbon is bonded to another two oxygens via single bonds; preferably the double covalent bonds are selected from the group consisting of carbon-carbon double bond, carbon-nitrogen double bond, sulphur-oxygen double bond, carbon-oxygen double bond wherein the carbon is a member of a ring structure, and carbon-oxygen double bond wherein the carbon is bonded to another two carbons via carbon-carbon single bonds, carbon-oxygen double bond wherein the carbon is bonded to another oxygen via single bond and to a nitrogen via a single bond, carbon-oxygen double bond wherein the carbon is bonded to two nitrogens via carbon-nitrogen single bonds.

**6.** The AZ-component according to any one of the preceding claims 1-3 and 5, wherein the AZ-component is selected from the group consisting of i) to v): i) (aziridinyl hydroxy)-functional organic compound AZ1 of Formula A1 (AZ1-compound), ii) (aziridinyl hydroxy)-functional organic compound AZ2 of Formula A2 (AZ2-compound), iii) (aziridinyl hydroxy)-functional organic compound AZ3 of Formula A3 (AZ3-compound), iv) (aziridinyl hydroxy)-functional organic compound AZ5 of Formula A5 (AZ5-compound), and vi) mixtures thereof, and wherein

the AZ1-compound is selected from the group consisting of compounds having the Formula A1a, and compounds having the Formula A1b, as each of these Formulae A1a-A1d is described below

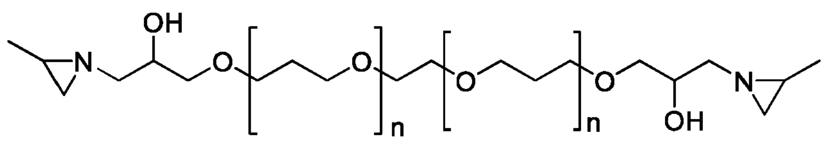

Formula A1a

wherein each of the n in Formula A1a is independently selected, and each of the n in Formula A1a is an integer ranging from and including 2 up to and including 20; and

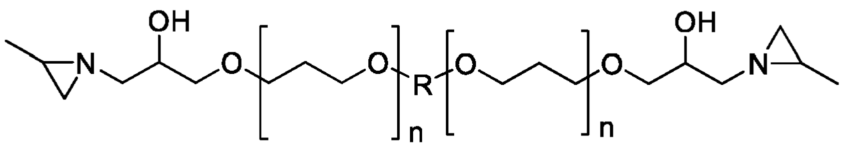

Formula A1b

wherein the R in Formula A1b is a $C_3$-$C_{10}$ saturated hydrocarbylene, and wherein each of the n in Formula A1b is independently selected and each of the n in Formula A1a is an integer ranging from and including 2 up to and including 20; and

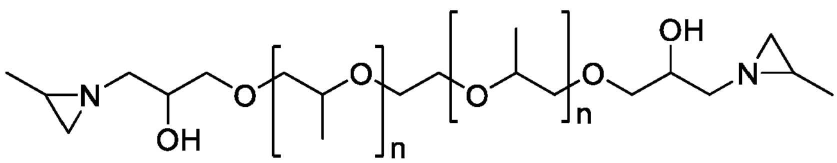

Formula A1c

wherein each of the n in Formula A1c is independently selected, and each of the n in Formula A1 b is an integer

ranging from and including 2 up to and including 20; and

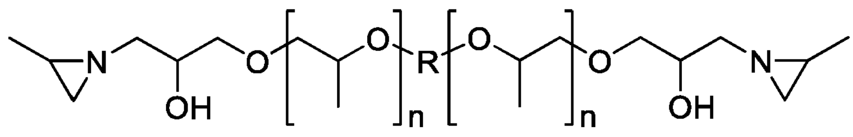

Formula A1d

wherein the R in Formula A1d is a $C_3$-$C_{10}$ saturated hydrocarbylene, and wherein each of the n in Formula A1d is independently selected, and each of the n in Formula A1b is an integer ranging from and including 2 up to and including 20;

and

wherein

the AZ2-compound is selected from the group consisting of compounds having the Formula A2a, compounds having the Formula A2b, compounds having the Formula A2c, compounds having the Formula A2d, compounds having the Formula A2e, as each of these Formulae A2a-A2e is described below

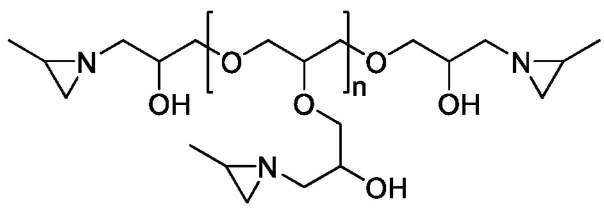

Formula A2a

wherein the n in Formula A2a is an integer ranging from and including 2 up to and including 20;

and

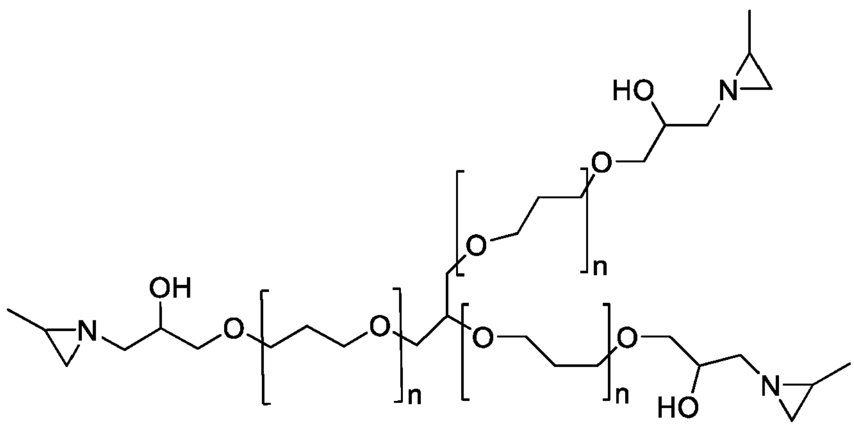

Formula A2b

wherein each of the n in Formula A2b is independently selected, and each of the n in Formula A2b is an integer ranging from and including 2 up to and including 20;

and

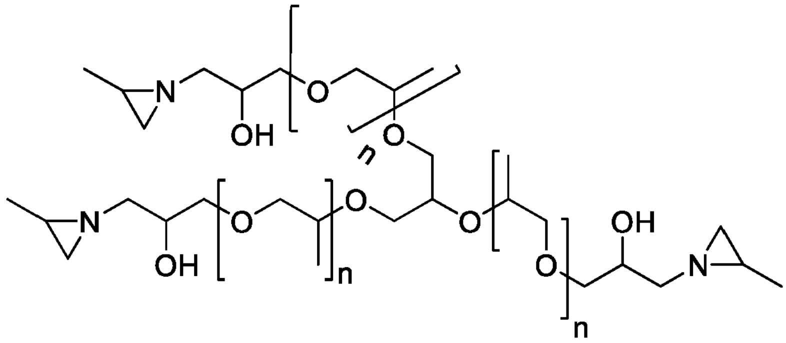

Formula A2c

wherein each of the n in Formula A2c is independently selected, and each of the n in Formula A2c is an integer ranging from and including 2 up to and including 20;
and

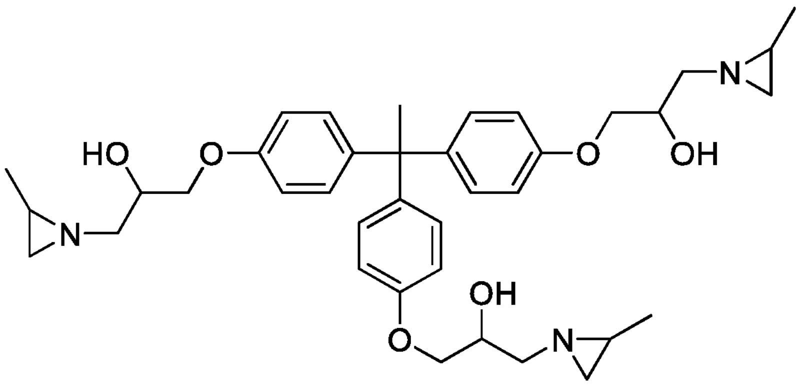

Formula A2d

and

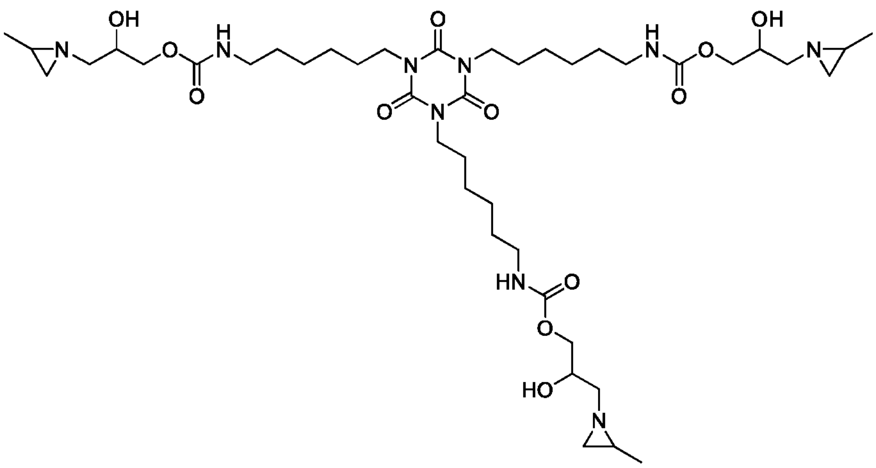

Formula A2e

and
wherein
the AZ3-compound is selected from the group consisting of compounds having the Formula A3a

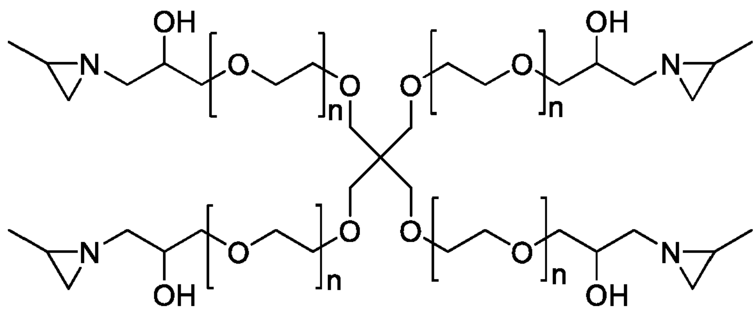

Formula A3a

wherein each of the n in Formula A3a is independently selected, and each of the n in Formula A3a is an integer ranging from and including 2 up to and including 20;
and
wherein
the AZ5-compound is selected from the group consisting of compounds having the Formula A5a

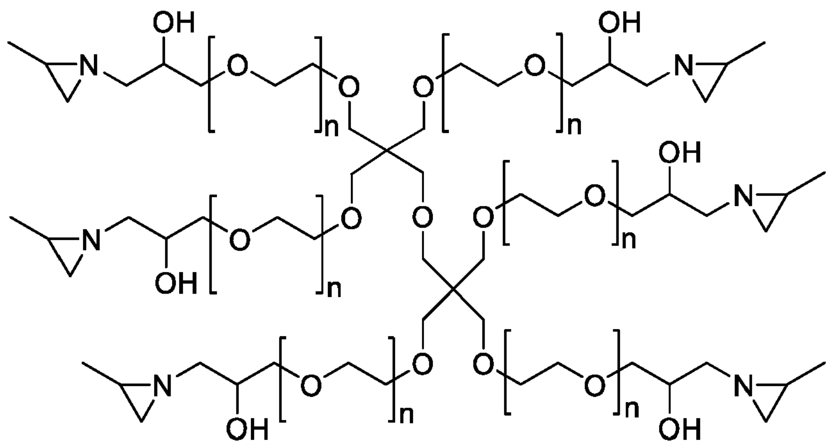

Formula A5a

wherein each of the n in Formula A5a is independently selected, and each of the n in Formula A5a is an integer ranging from and including 2 up to and including 40.

7. The AZ-component according to any one of the preceding claims 1-3 and 5, wherein the AZ-component is selected from the group consisting of AZ1-compound and wherein the AZ1-compound is the compound of the following formula

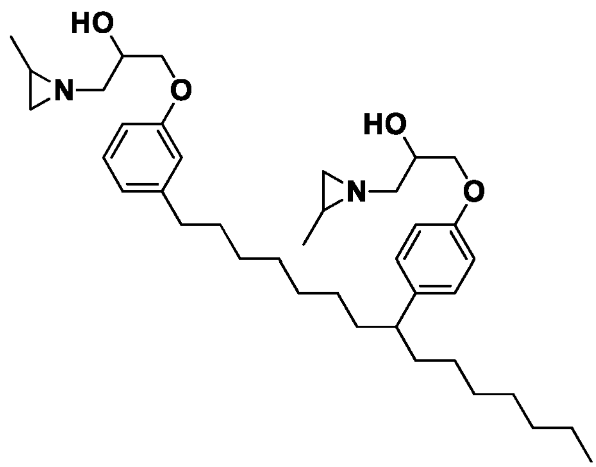

8. The AZ-component according to any one of the preceding claims 1-3 and 5, wherein the AZ-component is selected from the group consisting of AZ2-compound and wherein the AZ2-compound is the compound of the following formula

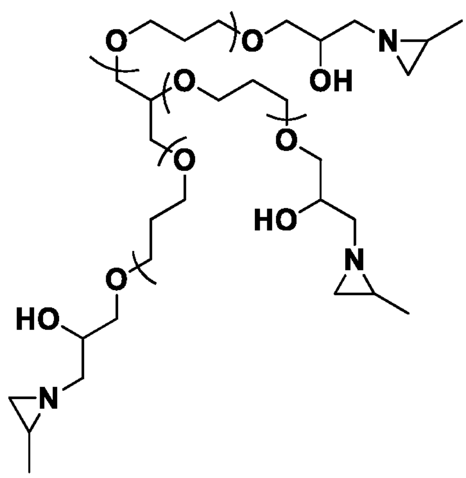

9. The AZ-component according to any one of the preceding claims 1-3 and 5, wherein the AZ-component is selected from the group consisting of AZ2-compound and wherein the AZ2-compound is the compound of the following formula

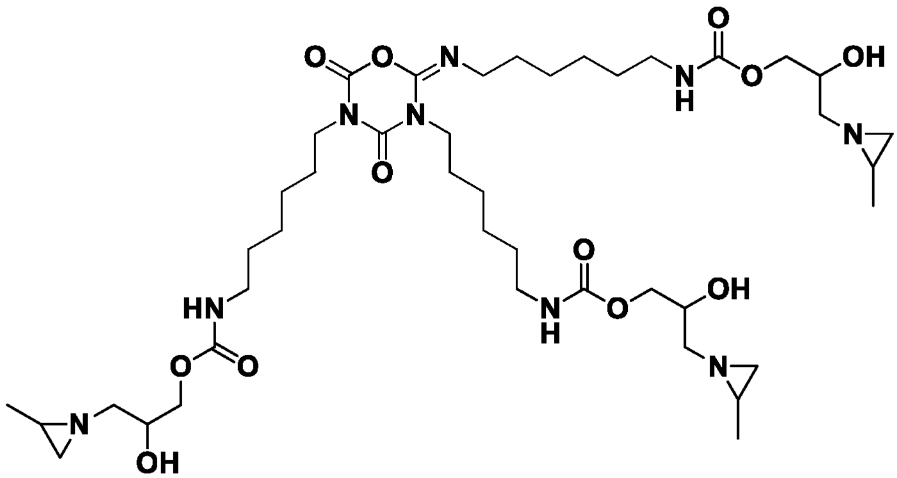

10. The AZ-component according to any one of the preceding claims 1-3 and 5, wherein the AZ-component is selected from the group consisting of AZ2-compound and wherein the AZ2-compound is the compound of the following formula

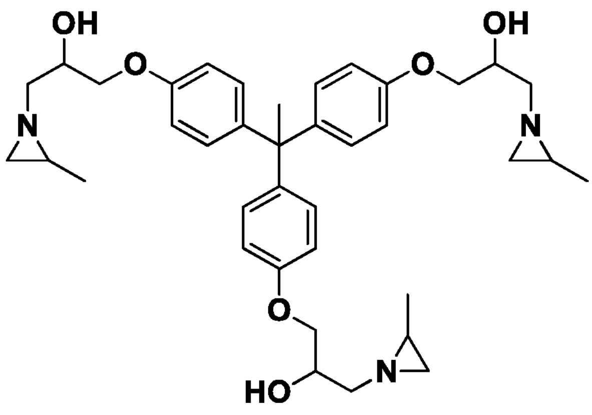

11. The AZ component according to any one of the preceding claims 1-3 and 5, wherein the AZ-component is selected

from the group consisting of AZ1-compound, AZ2-compound and mixtures thereof, and wherein the AZ1-compound is the compound of the following formula,

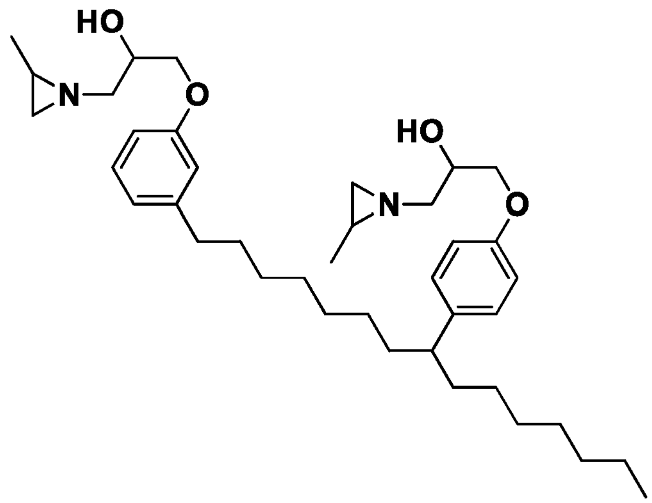

and wherein the AZ2-compound is selected from the group consisting of compounds of the following formulae,

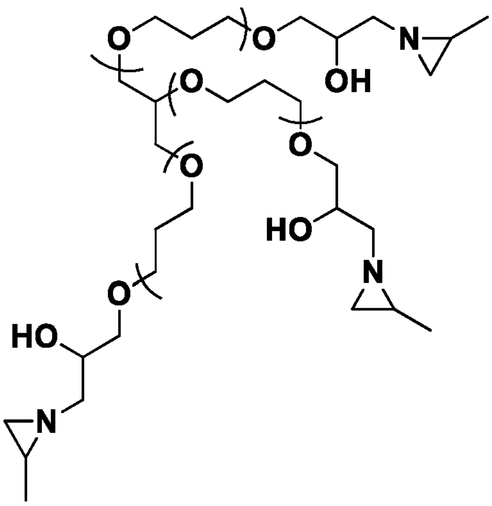

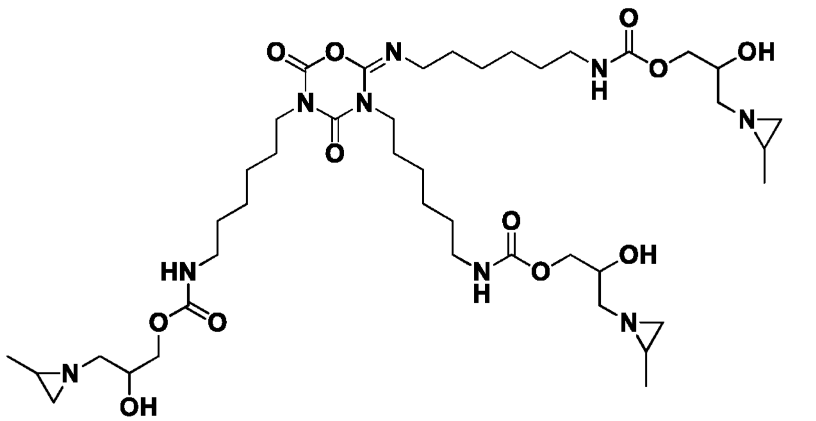

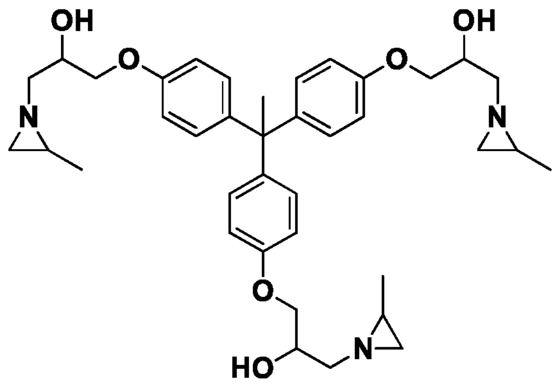

12. A liquid composition, comprising:

i) a liquid medium which is selected from the group consisting of organic solvents, water and a mixture thereof, in an amount of at most 90, wt% on the total weight of the liquid composition; and
ii) an AZ-component according to any one of the claims 1-11; and

wherein the total amount of all the components that make up the liquid composition totals 100 wt%.

13. The liquid composition according to the claim 12, wherein the liquid composition has a pH determined according to the ISO 976:2013 and according to the description, of at least 7.5 and at most 14.0, with the proviso that the liquid composition comprises water in an amount of at least 20, preferably at least 25, more preferably at least 30, most preferably at least 35 wt% on the total weight of the liquid composition.

14. The liquid composition according to any one of the claims 12-13, wherein the liquid composition comprises a component T selected from the group consisting of: i) organic compounds having a molecular weight determined via MALDI-TOF MS according to the description, lower than 600 Da and comprising at least one aziridine ring, and ii) mixtures thereof, in an amount determined via liquid chromatography coupled with mass spectroscopy (LC-MS) according to the description, of at most 5, preferably at most 0.5, for example at most 0.1, for example at most 0.05 wt% on the total weight of the AZ-compound.

15. The liquid composition according to any one of the claims 12-14, further comprising:
iii) a polymer which has an acid value determined according to the ASTM D1639-90(1996)e1 of at least 5 and at most 300 mg KOH/g and wherein the polymer may optionally comprise ionic functional groups.

16. A kit-of-parts comprising parts A and B which are physically separated from each other, wherein:

i) the part A comprises a liquid composition according to any one of the claims 12-15, and
ii) the part B comprises a polymer which has an acid value determined according to the ASTM D1639-90(1996)e1 in the range from 5 to 300, preferably from 8 to 200, more preferably from 10 to 150 mg KOH/g and wherein the polymer may optionally comprise ionic functional groups, and

wherein the part A does not comprise the polymer of the part B, and the part B does not comprise the liquid composition of the part A.

17. A cured form of an AZ-component according to any one of the claims 1-11, or of a liquid composition according to any one of the claims 12-15.

18. An article comprising: i) an AZ-component according to any one of the claims 1-11, or ii) a liquid composition according to any one of claims 12-15, and/or iii) a cured form according to the claim 17.

19. Use of any one or any combination of the following:

i) an AZ-component according to any one of the claims 1-11;
ii) a liquid composition according to any one of the claims 12-15;

iii) a kit-of-parts according to the claim 16;
iv) a cured form according to the claim 17;
v) an article according to the claim 18;

in coatings, paints, inks, varnishes, lubricants, adhesives, additive manufacturing, 3D-printing, textiles, waxes, fuels, photography, plastics, medical devices, and in the preparation of medical compositions.

**Patentansprüche**

1. (Aziridinylhydroxy)-funktionelle organische Komponente (AZ-Komponente), ausgewählt aus der Gruppe bestehend aus i) bis vi): i) (Aziridinylhydroxy)-funktionelle organische Verbindung AZ1 der Formel A1, aufweisend nur zwei Aziridinringe (AZ1-Verbindung), ii) (Aziridinylhydroxy)-funktionelle organische Verbindung AZ2 der Formel A2, aufweisend nur drei Aziridinringe (AZ2-Verbindung), iii) (Aziridinylhydroxy)-funktionelle organische Verbindung AZ3 der Formel A3, aufweisend nur vier Aziridinringe (AZ3-Verbindung), iv) (Aziridinylhydroxy)-funktionelle organische Verbindung AZ4 der Formel A4, aufweisend nur fünf Aziridinringe (AZ4-Verbindung), v) (Aziridinylhydroxy)-funktionelle organische Verbindung AZ5 der Formel A5, aufweisend nur sechs Aziridinringe (AZ5-Verbindung), und vi) Mischungen davon,

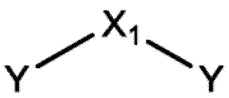

Formel A1

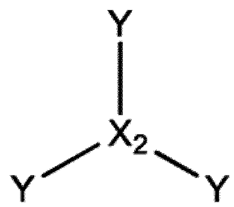

Formel A2

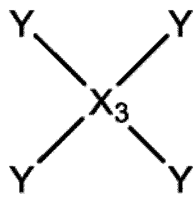

Formel A3

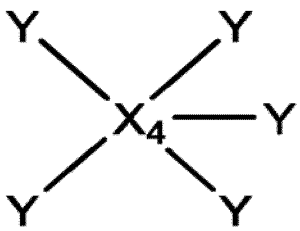

Formel A4

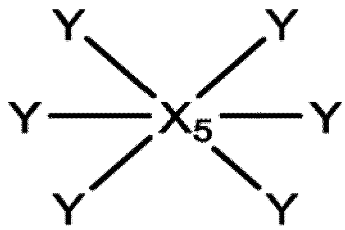

Formel A5

wobei

es sich bei $X_1$ um ein bivalentes aliphatisches organisches Radikal oder um ein bivalentes aromatisches organisches Radikal handelt, vorzugsweise wobei es sich bei $X_1$ um ein bivalentes aliphatisches organisches Radikal handelt;
es sich bei $X_2$ um ein trivalentes aliphatisches organisches Radikal oder um ein trivalentes aromatisches organisches Radikal handelt, vorzugsweise wobei es sich bei $X_2$ um ein trivalentes aliphatisches organisches Radikal handelt;
es sich bei $X_3$ um ein quadrivalentes aliphatisches organisches Radikal oder um ein quadrivalentes aromatisches organisches Radikal handelt, vorzugsweise wobei es sich bei $X_3$ um ein quadrivalentes aliphatisches organisches Radikal handelt;
es sich bei $X_4$ um ein pentavalentes aliphatisches organisches Radikal oder um ein pentavalentes aromatisches organisches Radikal handelt, vorzugsweise wobei es sich bei $X_4$ um ein pentavalentes aliphatisches organisches Radikal handelt;
es sich bei $X_5$ um ein hexavalentes aliphatisches organisches Radikal oder um ein hexavalentes aromatisches organisches Radikal handelt, vorzugsweise wobei es sich bei $X_5$ um ein hexavalentes aliphatisches organisches Radikal handelt;
und wobei jedes der $X_1$ bis $X_5$ aus einer Ansammlung von Atomen besteht, die kovalent in einer Konfiguration

verbunden sind, die lineare und/oder verzweigte und/oder Ringstrukturen umfasst -vorzugsweise bestehend aus-,
wobei die Ansammlung von Atomen ausgewählt ist aus der Gruppe bestehend aus i) bis x):
i) Kohlenstoff- und Wasserstoffatomen, ii) Kohlenstoff-, Wasserstoff- und Sauerstoffatomen, iii) Kohlenstoff-, Wasserstoff- und Stickstoffatomen, iv) Kohlenstoff-, Wasserstoff- und Schwefelatomen, v) Kohlenstoff-, Wasserstoff-, Sauerstoff- und Stickstoffatomen, vi) Kohlenstoff-, Wasserstoff- Stickstoff- und Schwefelatomen, vii) Kohlenstoff-, Wasserstoff-, Sauerstoff- und Schwefelatomen, viii) Kohlenstoff-, Wasserstoff-, Sauerstoff-, Stickstoff- und Schwefelatomen, ix) Kohlenstoff-, Wasserstoff- und Siliciumatomen, und x) Kohlenstoff-, Wasserstoff-, Sauerstoff- und Siliciumatomen und xi) beliebigen Kombinationen von ix) und/oder x) mit einem beliebigen oder allen der iii) bis viii),
und wobei jedes der $X_1$ bis $X_5$ Kohlenstoffatome und Wasserstoffatome aufweist,
und wobei jedes der $X_1$ bis $X_5$ gegebenenfalls Sauerstoffatome und/oder Stickstoffatome und/oder Schwefelatome und/oder Siliciumatome aufweist, und wobei das $X_1$, und/oder das $X_2$ und/oder das $X_3$ und/oder das $X_4$ und/oder das $X_5$ gegebenenfalls eine ionisch funktionelle Gruppe umfasst,
wobei
es sich bei Y um ein monovalentes organisches Radikal handelt, ausgewählt aus der Gruppe bestehend aus: i) monovalentes (Aziridinylhydroxyisopropyl) organisches Radikal der Formel B1, ii) monovalentes (Aziridinylhydroxycyclohexan) organisches Radikal der Formel B2, und iii) monovalentes (Aziridinylhydroxycyclohexan) organisches Radikal der Formel B3, es sich bei Y vorzugsweise um ein monovalentes (Aziridinylhydroxyisopropyl) organisches Radikal der Formel B1 handelt,

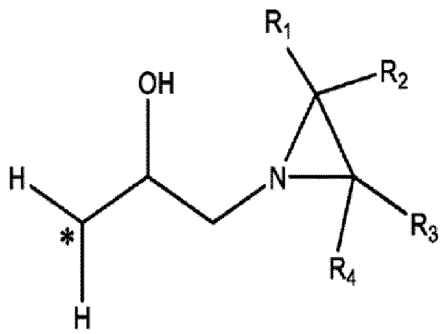

Formel B1

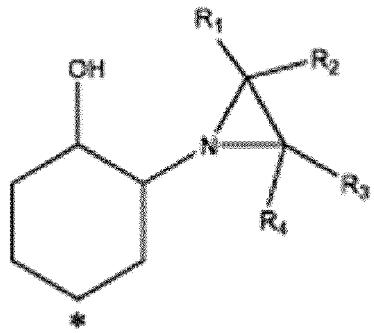

Formel B2

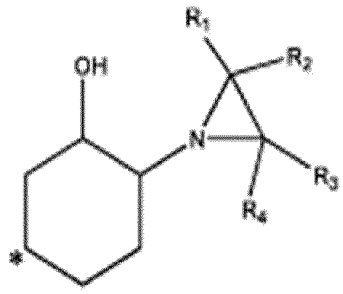

Formel B3

und wobei

$R_1$ ausgewählt ist aus der Gruppe bestehend aus Wasserstoff und Methyl; und
$R_2$ ausgewählt ist aus der Gruppe bestehend aus Wasserstoff, Methyl und $C_2$-$C_5$-Alkyl; und
$R_3$ ausgewählt ist aus der Gruppe bestehend aus Methyl und $C_2$-$C_4$-Alkyl; und
$R_4$ ausgewählt ist aus der Gruppe bestehend aus Wasserstoff, Methyl und $C_2$-$C_4$-Alkyl,
und wobei

das Y in jeder der Verbindungen A1 bis A5 gleich oder unterschiedlich voneinander sein kann, und wobei jede der kovalenten Einfachbindungen zwischen dem Y und jedem einzelnen von $X_1$ bis $X_5$ ausgewählt ist aus der Gruppe bestehend aus Kohlenstoff-Kohlenstoff-Einfachbindung, und Kohlenstoff-Sauerstoff-Einfachbindung, vorzugsweise es sich bei jeder der kovalenten Einfachbindungen zwischen dem Y und jedem einzelnen von $X_1$ bis $X_5$ um Kohlenstoff-Sauerstoff-Einfachbindung handelt,
und wobei
jede der AZ1- bis AZ5-Verbindungen ein Molekulargewicht, das mittels MALDI-TOF MS gemäß der Beschreibung bestimmt wurde, von zumindest 600 und höchstens 10000, vorzugsweise zumindest 600 und höchstens 8000, bevorzugter zumindest 600 und höchstens 6000, am meisten bevorzugt zumindest 600 und höchstens 5000, besonders zumindest 600 und höchstens 4000, ganz besonders zumindest 600 und höchstens 3500, insbesondere zumindest 600 und höchstens 3200, zum Beispiel zumindest 600 und höchstens 3000, zum Beispiel zumindest 600 und höchstens 2500 Da aufweist,
und wobei
das $X_1$ und das $X_2$ und das $X_3$ und das $X_4$ und das $X_5$ nicht eine oder eine beliebige Kombination der folgenden Struktureinheiten BP1, BP2, BP3 und BS enthalten

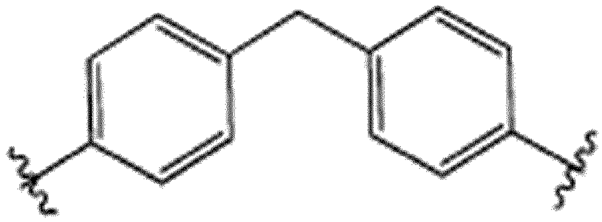

Einheit BP1

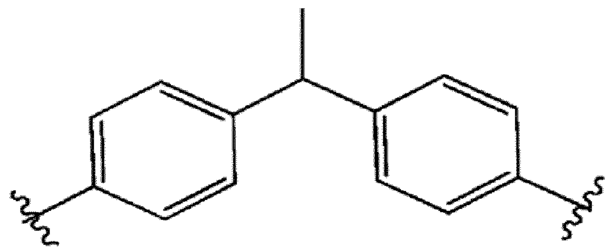

Einheit BP2

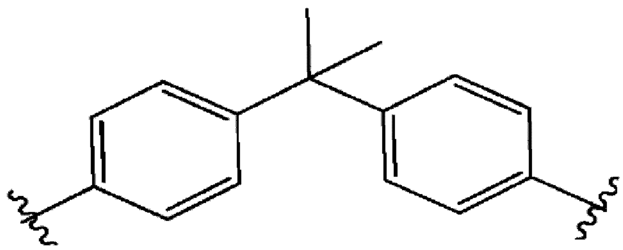

Einheit BP3

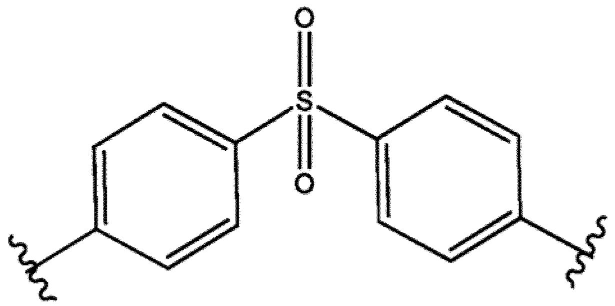

Einheit BS.

2. AZ-Komponente nach Anspruch 1, wobei das $X_1$ und/oder das $X_2$ und/oder das $X_3$ und/oder das $X_4$ und/oder das $X_5$ zumindest eine Struktureinheit oder eine Kombination von Struktureinheiten aufweist, die ausgewählt sind aus der Gruppe bestehend aus Einheit 1, Einheit 2, Einheit 3, Einheit 4, Einheit 5, Einheit 6, Einheit 7, Einheit 8, Einheit 9, Einheit 10, und Einheit 11, wie die Einheiten 1 bis 11 untenstehend abgebildet:

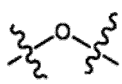 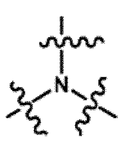 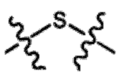 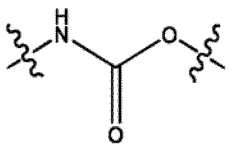 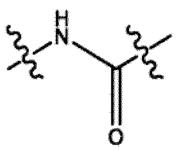

Einheit 1 Einheit 2 Einheit 3 Einheit 4 Einheit 5

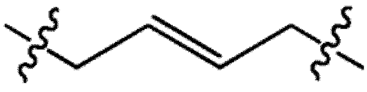

Einheit 6

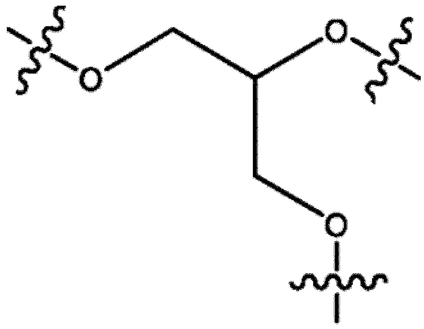

Einheit 7

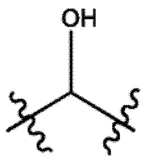

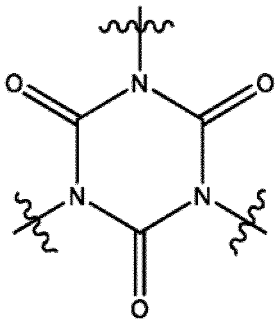

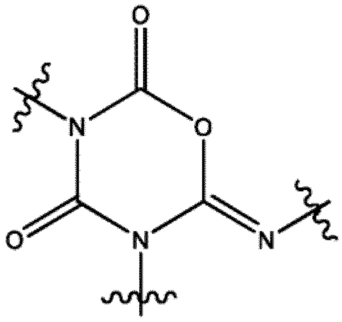

Einheit 8 Einheit 9 Einheit 10

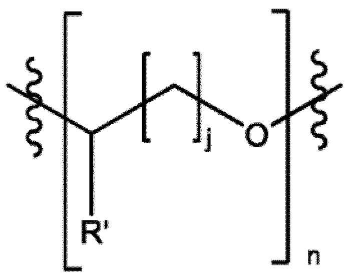

Einheit 11

wobei

R' ausgewählt ist aus der Gruppe bestehend aus Wasserstoff und Methyl; und
j eine ganze Zahl im Bereich von 1 bis 5, vorzugsweise von 1 bis 3 ist; und
n eine ganze Zahl im Bereich von und einschließlich 2 bis zu und einschließlich 50 ist.

**3.** AZ-Komponente nach einem der vorhergehenden Ansprüche, wobei die Gesamtzahl von Kohlenstoffatomen in $R_1$ und $R_2$ und $R_3$ und $R_4$ höchstens 9, vorzugsweise höchstens 4, bevorzugter höchstens 2, zum Beispiel höchstens 1 beträgt.

**4.** AZ-Komponente nach einem der vorhergehenden Ansprüche, wobei es sich bei dem $X_1$ um das bivalente aliphatische organische Radikal von Formel A1a' handelt

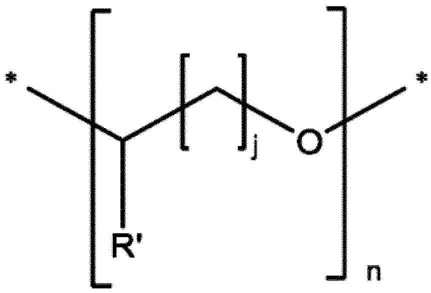

Formel A1a'

wobei

R' ausgewählt ist aus der Gruppe bestehend aus Wasserstoff und Methyl; und
j eine ganze Zahl im Bereich von 1 bis 5, vorzugsweise von 1 bis 3 ist; und
n eine ganze Zahl im Bereich von und einschließlich 2 bis zu und einschließlich 50 ist.

**5.** AZ-Komponente nach einem der Ansprüche 1 bis 4, wobei jedes der $X_1$ bis $X_5$ nur kovalente Einfachbindungen oder sowohl kovalente Einfach- als auch Doppelbindungen enthält, und wobei die kovalenten Einfachbindungen ausgewählt sind aus der Gruppe bestehend aus Kohlenstoff-Kohlenstoff-Einfachbindung, Kohlenstoff-Wasserstoff-Einfachbindung, Kohlenstoff-Stickstoff-Einfachbindung, Kohlenstoff-Schwefel-Einfachbindung, Kohlenstoff-Silicium-Einfachbindung, Silicium-Sauerstoff-Einfachbindung, Stickstoff-Wasserstoff-Einfachbindung, Schwefel-Sauerstoff-Einfachbindung, Kohlenstoff-Sauerstoff-Einfachbindung, wobei der Sauerstoff an einen Wasserstoff gebunden ist, um eine Hydroxylgruppe zu bilden, Silicum-Sauerstoff-Silicium-Einfachbindungen, und wobei die kovalenten Doppelbindungen ausgewählt sind aus der Gruppe bestehend aus Kohlenstoff-Kohlenstoff-Doppelbindung, Kohlenstoff-Stickstoff-Doppelbindung, Schwefel-Sauerstoff-Doppelbindung, Kohlenstoff-Sauerstoff-Doppelbindung, wobei der Kohlenstoff ein Mitglied einer Ringstruktur ist, und Kohlenstoff-Sauerstoff-Doppelbindung, wobei der Kohlenstoff mittels Kohlenstoff-Kohlenstoff-Einfachbindung an zwei andere Kohlenstoffe gebunden ist, Kohlenstoff-Sauerstoff-Doppelbindung, wobei der Kohlenstoff an anderes Sauerstoff mittels Einfachbindung und an ein Stickstoff mittels Einfachbindung gebunden ist, Kohlenstoff-Sauerstoff-Doppelbindung, wobei der Kohlenstoff an zwei Stickstoffe mittels Kohlenstoff-Stickstoff-Einfachbindung gebunden ist, Kohlenstoff-Sauerstoff-Doppelbindung, wobei der Kohlenstoff an zwei andere Sauerstoffe mittels Einfachbindungen gebunden ist; vorzugsweise sind die kovalenten Doppelbindungen ausgewählt aus der Gruppe bestehend aus Kohlenstoff-Kohlenstoff-Doppelbindung, Kohlenstoff-Stickstoff-Doppelbindung, Schwefel-Sauerstoff-Doppelbindung, Kohlenstoff-Sauerstoff-Doppelbindung, wobei der Kohlenstoff ein Mitglied einer Ringstruktur ist, und Kohlenstoff-Sauerstoff-Doppelbindung, wobei der Kohlenstoff an zwei anderen Kohlenstoffen mittels Kohlenstoff-Kohlenstoff-Einfachbindung gebunden ist, Kohlenstoff-Sauerstoff-Doppelbindung, wobei der Kohlenstoff an anderes Sauerstoff mittels Einfachbindung und an ein Stickstoff mittels Einfachbindung gebunden ist, Kohlenstoff-Sauerstoff-Doppelbindung, wobei der Kohlenstoff an zwei Stickstoffe mittels Kohlenstoff-Stickstoff-Einfachbindungen gebunden ist.

**6.** AZ-Komponente nach einem der vorhergehenden Ansprüche 1-3 und 5, wobei die AZ-Komponente ausgewählt ist aus der Gruppe bestehend aus i) bis v): i) (Aziridinylhydroxy)-funktionelle organische Verbindung AZ1 der Formel A1 (AZ1-Verbindung), ii) (Aziridinylhydroxy)-funktionelle organische Verbindung AZ2 der Formel A2 (AZ2-Verbindung), iii) (Aziridinylhydroxy)-funktionelle organische Verbindung AZ3 der Formel A3 (AZ3-Verbindung), iv) (Aziridinylhydroxy)-funktionelle organische Verbindung AZ5 der Formel A5 (AZ5-Verbindung), und vi) Mischungen davon, und
wobei

die AZ1-Verbindung ausgewählt ist aus der Gruppe bestehend aus Verbindungen, aufweisend die Formel A1a, und Verbindungen, aufweisend die Formel A1b, wie jede dieser Formeln A1a-A1d untenstehend beschrieben wird

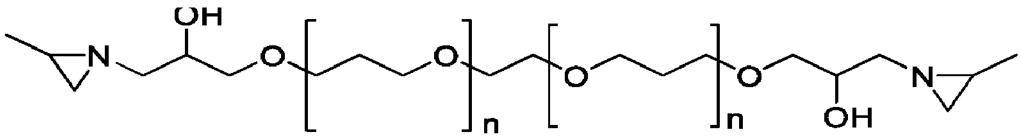

Formel A1a

wobei jedes der n in Formel A1a unabhängig ausgewählt ist und jedes der n in Formel A1a eine ganze Zahl im Bereich von und einschließlich 2 bis zu und einschließlich 20 ist; und

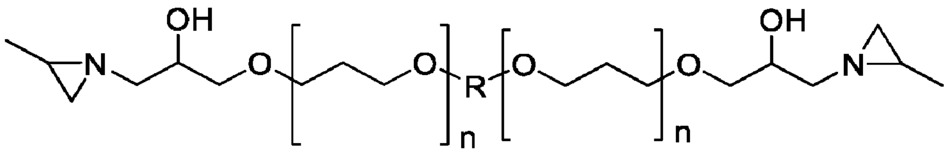

Formel A1b

wobei das R in Formel A1b ein gesättigtes $C_3$-$C_{10}$-Hydrocarbylen ist, und wobei jedes der n in Formel A1b unabhängig ausgewählt ist und jedes der n in Formel A1a eine ganze Zahl im Bereich von und einschließlich 2 bis zu und einschließlich 20 ist; und

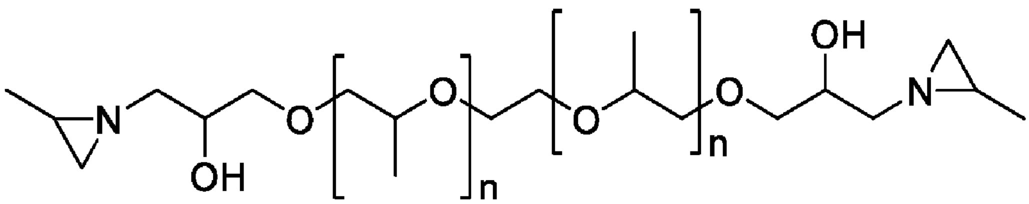

Formel A1c

wobei jedes der n in Formel A1c unabhängig ausgewählt ist und jedes der n in Formel A1b eine ganze Zahl im Bereich von und einschließlich 2 bis zu und einschließlich 20 ist; und

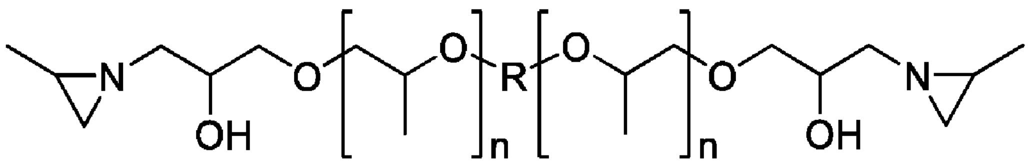

Formel A1d

wobei das R in Formel A1d ein gesättigtes $C_3$-$C_{10}$-Hydrocarbylen ist, und wobei jedes der n in Formel A1d unabhängig ausgewählt ist und jedes der n in Formel A1b eine ganze Zahl im Bereich von und einschließlich 2 bis zu und einschließlich 20 ist; und

wobei

die AZ2-Verbindung ausgewählt ist aus der Gruppe bestehend aus Verbindungen, aufweisend die Formel A2a, Verbindungen, aufweisend die Formel A2b, Verbindungen, aufweisend die Formel A2c, Verbindungen, aufweisend die Formel A2d, Verbindungen, aufweisend die Formel A2e, wie jede dieser Formeln A2a-A2e untenstehend beschrieben wird

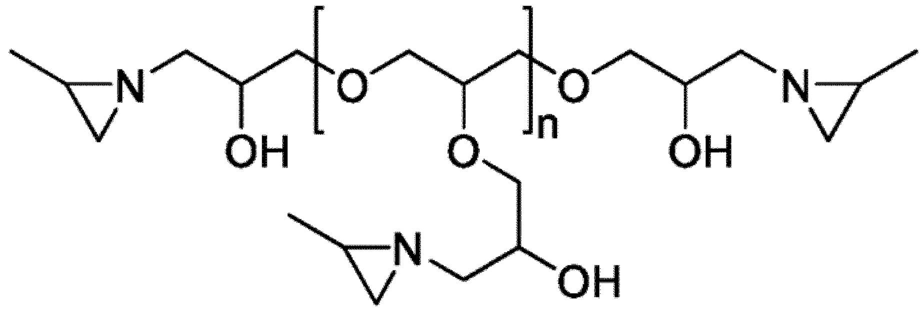

Formel A2a

wobei das n in Formel A2a eine ganze Zahl im Bereich von und einschließlich 2 bis zu und einschließlich 20 ist; und

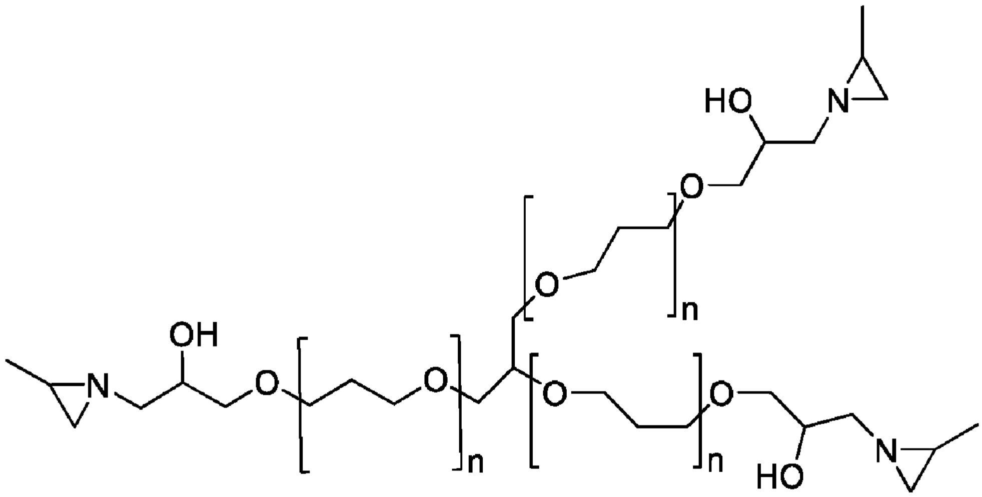

Formel A2b

wobei jedes der n in Formel A2b unabhängig ausgewählt ist und jedes der n in Formel A2b eine ganze Zahl im Bereich von und einschließlich 2 bis zu und einschließlich 20 ist;
und

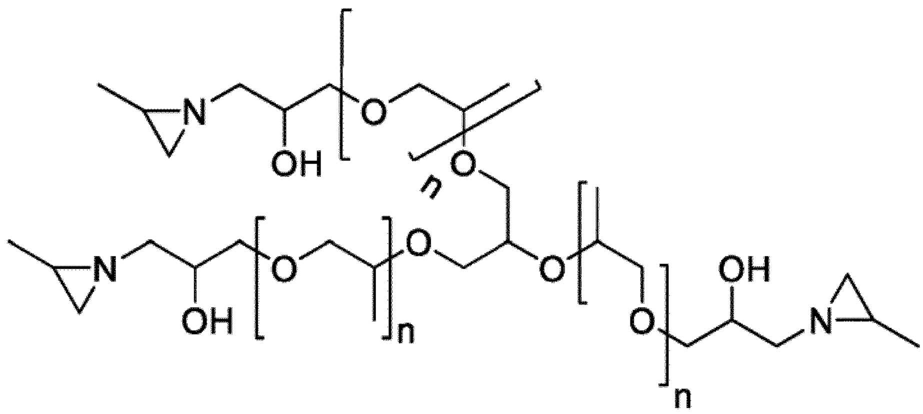

Formel A2c

wobei jedes der n in Formel A2c unabhängig ausgewählt ist und jedes der n in Formel A2c eine ganze Zahl im Bereich von und einschließlich 2 bis zu und einschließlich 20 ist;

und

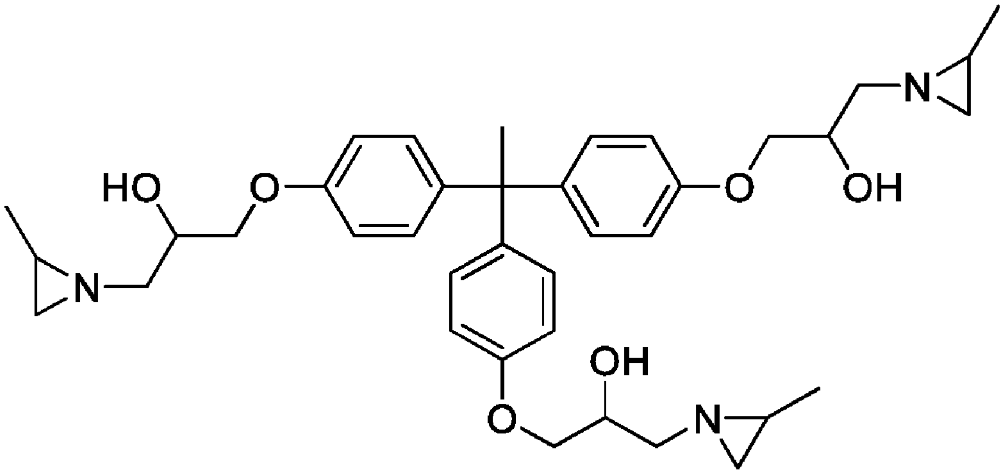

Formel A2d

und

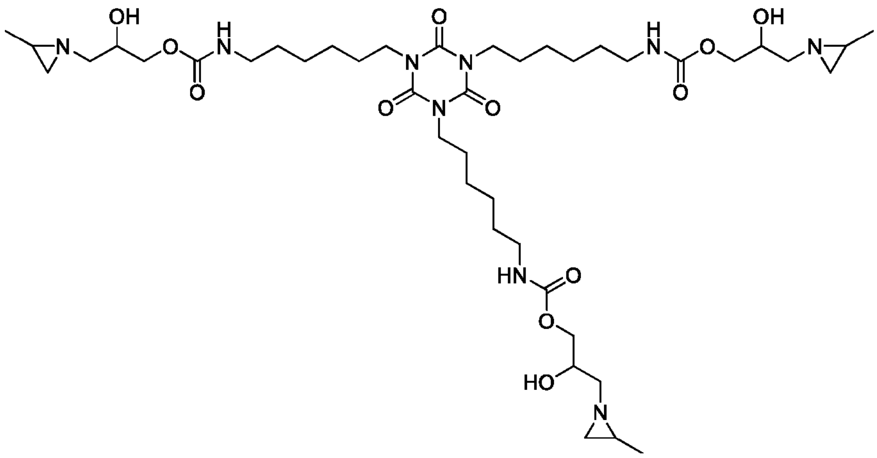

Formel A2e

und wobei die AZ3-Verbindung ausgewählt ist aus der Gruppe bestehend aus Verbindungen, aufweisend die Formel A3a

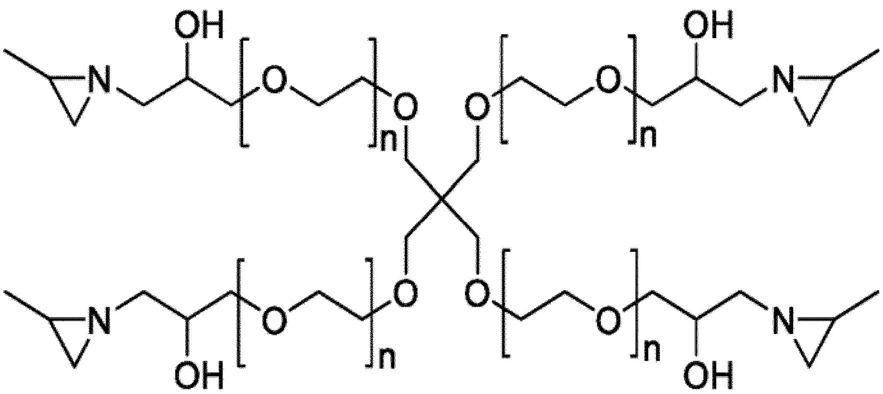

Formel A3a

wobei jedes der n in Formel A3a unabhängig ausgewählt ist und jedes der n in Formel A3a eine ganze Zahl im Bereich von und einschließlich 2 bis zu und einschließlich 20 ist;
und wobei
die AZ5-Verbindung ausgewählt ist aus der Gruppe bestehend aus Verbindungen, aufweisend die Formel A5a

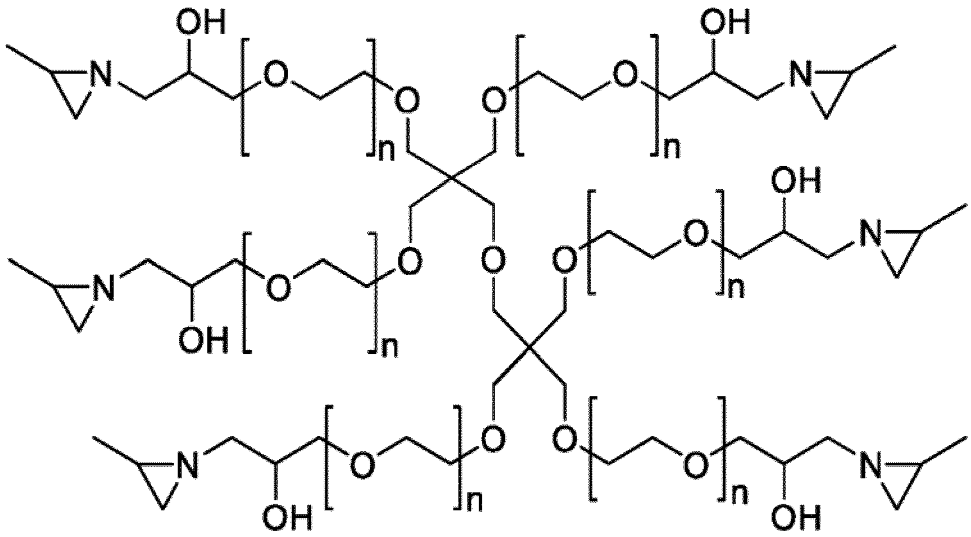

Formel A5a

wobei jedes der n in Formel A5a unabhängig ausgewählt ist und jedes der n in Formel A5a eine ganze Zahl im Bereich von und einschließlich 2 bis zu und einschließlich 40 ist.

**7.** AZ-Komponente nach einem der vorhergehenden Ansprüche 1-3 und 5, wobei die AZ-Komponente ausgewählt ist aus der Gruppe bestehend aus AZ1-Verbindung und wobei es sich bei der AZ1-Verbindung um eine Verbindung der folgenden Formel handelt:

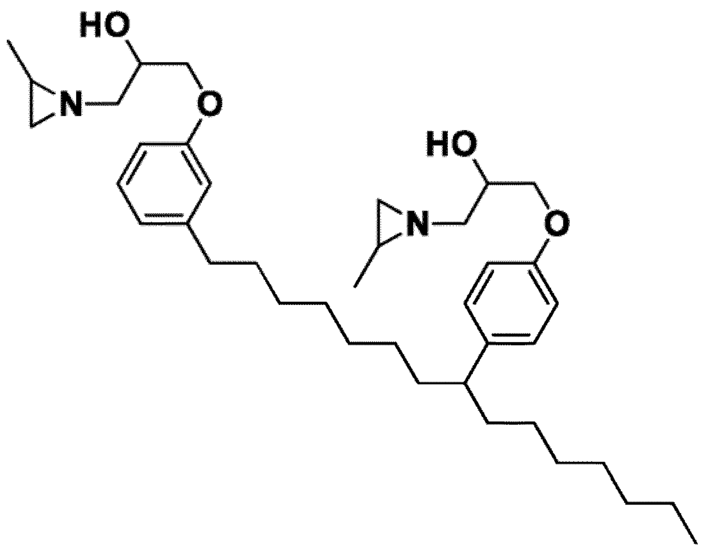

**8.** AZ-Komponente nach einem der vorhergehenden Ansprüche 1-3 und 5, wobei die AZ-Komponente ausgewählt ist aus der Gruppe bestehend aus AZ2-Verbindung und wobei es sich bei der AZ2-Verbindung um eine Verbindung der folgenden Formel handelt:

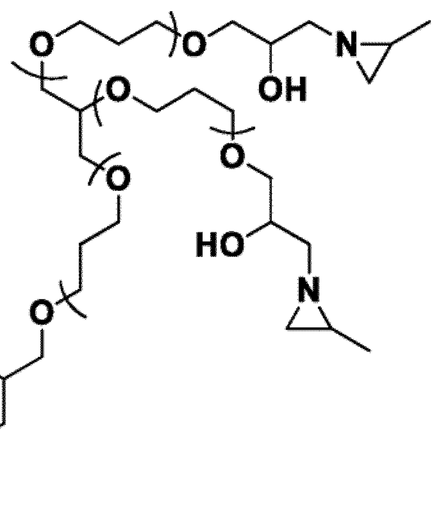

9. AZ-Komponente nach einem der vorhergehenden Ansprüche 1-3 und 5, wobei die AZ-Komponente ausgewählt ist aus der Gruppe bestehend aus AZ2-Verbindung und wobei es sich bei der AZ2-Verbindung um eine Verbindung der folgenden Formel handelt:

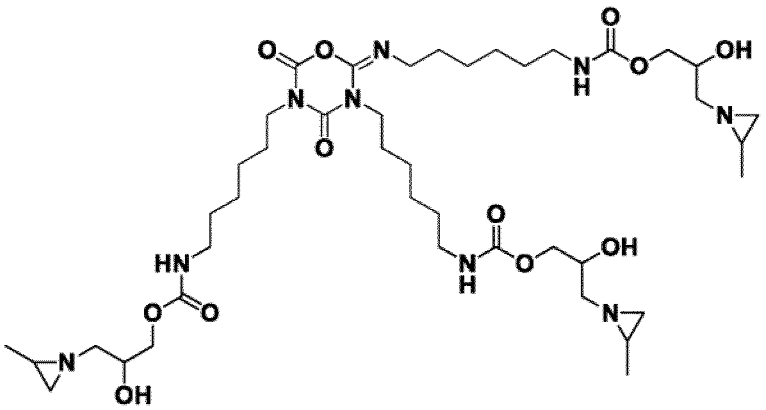

10. AZ-Komponente nach einem der vorhergehenden Ansprüche 1-3 und 5, wobei die AZ-Komponente ausgewählt ist aus der Gruppe bestehend aus AZ2-Verbindung und wobei es sich bei der AZ2-Verbindung um eine Verbindung der folgenden Formel handelt:

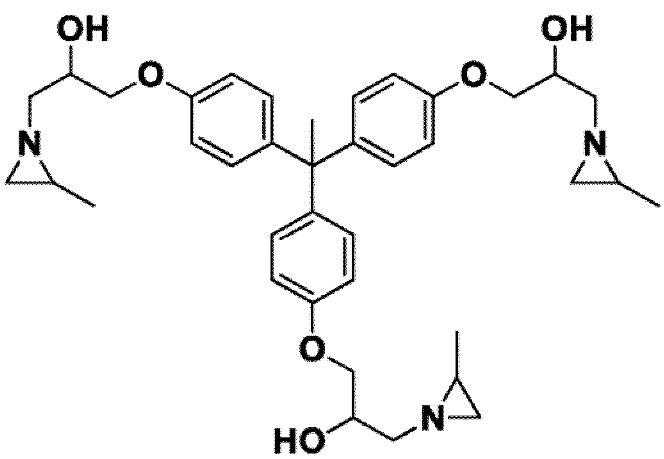

11. AZ-Komponente nach einem der vorhergehenden Ansprüche 1-3 und 5, wobei die AZ-Komponente ausgewählt ist aus der Gruppe bestehend aus AZ1-Verbindung, AZ2-Verbindung und Mischungen davon, und wobei es sich bei

der AZ1-Verbindung um eine Verbindung der folgenden Formel handelt:

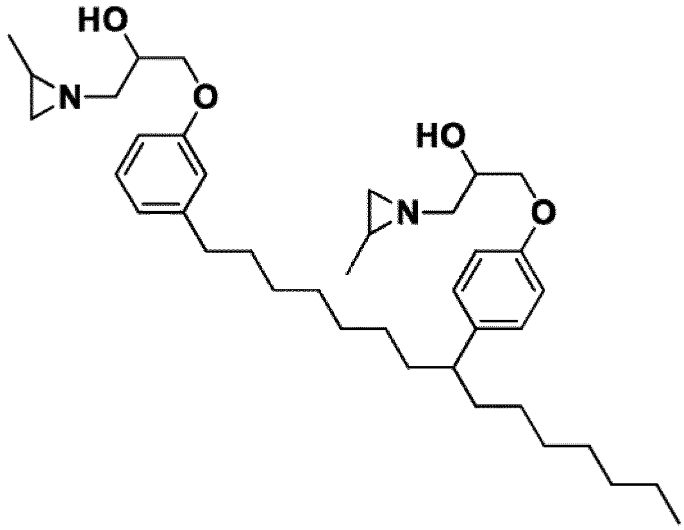

und wobei die AZ2-Verbindung ausgewählt ist aus der Gruppe bestehend aus Verbindungen der folgenden Formeln

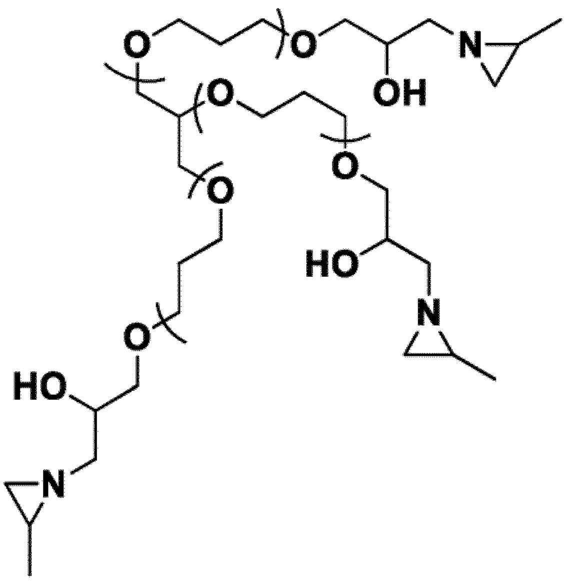

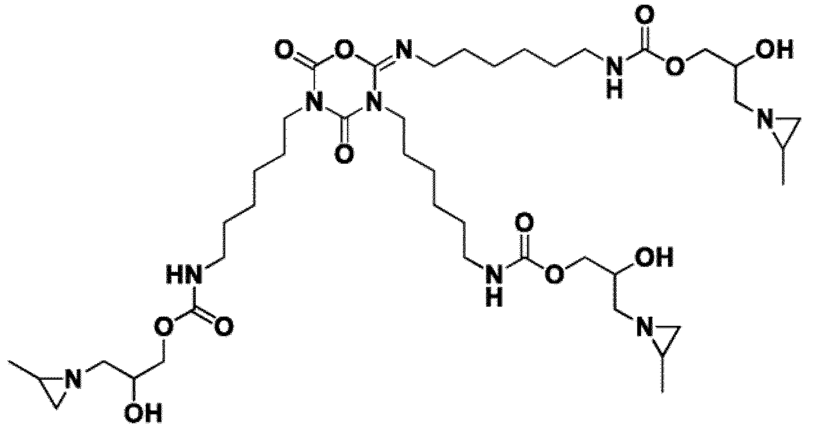

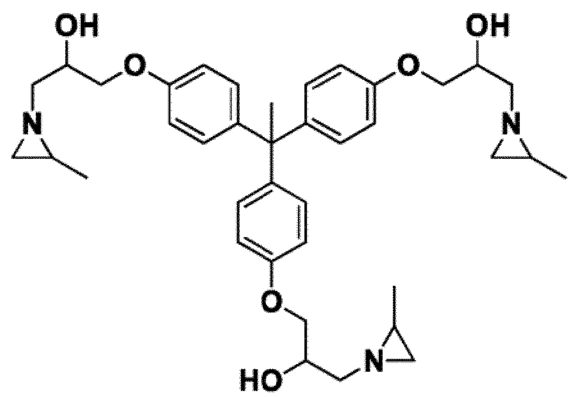

**12.** Flüssige Zusammensetzung, umfassend

i) ein flüssiges Medium, das ausgewählt ist aus der Gruppe bestehend aus organischen Lösungsmitteln, Wasser und einer Mischung davon, in einer Menge von höchstens 90 Gew.-% von dem Gesamtgewicht der flüssigen Zusammensetzung; und
ii) eine AZ-Komponente nach einem der Ansprüche 1-11; und

wobei die Gesamtmenge von all den Komponenten, die die flüssige Zusammensetzung ausmachen, 100 Gew.-% beträgt.

**13.** Flüssige Zusammensetzung nach Anspruch 12, wobei die flüssige Zusammensetzung einen pH-Wert aufweist, der gemäß ISO 976:2013 und gemäß der Beschreibung bestimmt wurde, von zumindest 7,5 und höchstens 14,0, mit der Maßgabe, dass die flüssige Zusammensetzung Wasser in einer Menge von zumindest 20, vorzugsweise zumindest 25, bevorzugter zumindest 30, am meisten bevorzugt zumindest 35 Gew.-% von dem Gesamtgewicht der flüssigen Zusammensetzung umfasst.

**14.** Flüssige Zusammensetzung nach einem der Ansprüche 12-13, wobei die flüssige Zusammensetzung eine Komponente T umfasst, ausgewählt aus der Gruppe bestehend aus: i) organischen Verbindungen, aufweisend ein Molekulargewicht, das mittels MALDI-TOF MS gemäß der Beschreibung bestimmt wurde, von weniger als 600 Da und umfassend zumindest einen Aziridinring, und ii) Mischungen davon, in einer Menge, die mittels Flüssigchromatographie, gekoppelt mit Massenspektroscopie (LC-MS), gemäß der Beschreibung bestimmt wurde, von höchstens 5, vorzugsweise höchstens 0,5, zum Beispiel höchstens 0,1, zum Beispiel höchstens 0,05 Gew.-% von dem Gesamtgewicht der AZ-Verbindung.

**15.** Flüssige Zusammensetzung nach einem der Ansprüche 12-14, ferner umfassend:
iii) ein Polymer, das eine Säurezahl, die gemäß ASTM D1639-90(1996)e1 bestimmt wurde, von zumindest 5 und höchstens 300 mg KOH/g aufweist und wobei das Polymer gegebenenfalls ionische funktionelle Gruppen umfasst.

**16.** Teilesatz, umfassend Teile A und B, die physisch voneinander getrennt sind, wobei:

i) das Teil A eine flüssige Zusammensetzung nach einem der Ansprüche 12-15 umfasst, und
ii) das Teil B ein Polymer umfasst, das eine Säurezahl, die gemäß ASTM D1639-90(1996)e1 bestimmt wurde, im Bereich von 5 bis 300, vorzugsweise von 8 bis 200, bevorzugter von 10 bis 150 mg KOH/g aufweist und wobei das Polymer gegebenenfalls ionische funktionelle Gruppen umfasst, und

wobei das Teil A nicht das Polymer von dem Teil B umfasst, und das Teil B nicht die flüssige Zusammensetzung von Teil A umfasst.

**17.** Gehärtete Form einer AZ-Komponente nach einem der Ansprüche 1-11, oder einer flüssigen Zusammensetzung nach einem der Ansprüche 12-15.

**18.** Artikel, umfassend: i) eine AZ-Komponente nach einem der Ansprüche 1-11, oder ii) eine flüssige Zusammensetzung nach einem der Ansprüche 12-15, und/oder iii) eine gehärtete Form nach Anspruch 17.

**19.** Verwendung von einem beliebigen oder einer beliebigen Kombination des Folgenden:

i) eine AZ-Komponente nach einem der Ansprüche 1-11;
ii) eine flüssige Zusammensetzung nach einem der Ansprüche 12-15;
iii) ein Teilesatz nach Anspruch 16;
iv) eine gehärtete Form nach Anspruch 17;
v) ein Artikel nach Anspruch 18;

in Beschichtungen, Farben, Tinten, Lacken, Schmierstoffen, Klebstoffen, additiver Fertigung, 3D-Druck, Textilien, Wachsen, Kraftstoffen, Fotografie, Kunststoffen, medizinischen Vorrichtungen und bei der Herstellung von medizinischen Zusammensetzungen.

## Revendications

**1.** Composant organique à fonction aziridinyle et hydroxy (composant AZ) choisi dans le groupe constitué par i) à vi) : i) un composé organique à fonction aziridinyle et hydroxy AZ1 de formule A1 ayant seulement deux cycles aziridine (composé AZ1), ii) un composé organique à fonction aziridinyle et hydroxy AZ2 de formule A2 ayant seulement trois cycles aziridine (composé AZ2), iii) un composé organique à fonction aziridinyle et hydroxy AZ3 de formule A3 ayant seulement quatre cycles aziridine (composé AZ3), iv) un composé organique à fonction aziridinyle et hydroxy AZ4 de formule A4 ayant seulement cinq cycles aziridine (composé AZ4), v) un composé organique à fonction aziridinyle et hydroxy AZ5 de formule A5 ayant seulement six cycles aziridine (composé AZ5) et vi) les mélanges de ceux-ci,

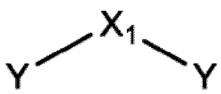
Formule A1

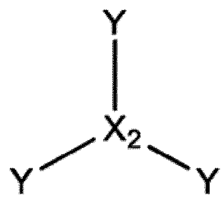
Formule A2

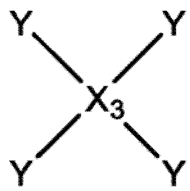
Formule A3

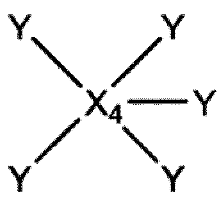
Formule A4

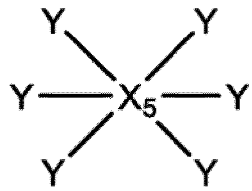
Formule A5

dans lequel

$X_1$ est un radical organique aliphatique divalent ou un radical organique aromatique divalent, de préférence $X_1$ est un radical organique aliphatique divalent ;
$X_2$ est un radical organique aliphatique trivalent ou un radical organique aromatique trivalent, de préférence $X_2$ est un radical organique aliphatique trivalent ;
$X_3$ est un radical organique aliphatique tétravalent ou un radical organique aromatique tétravalent, de préférence $X_3$ est un radical organique aliphatique tétravalent ;
$X_4$ est un radical organique aliphatique pentavalent ou un radical organique aromatique pentavalent, de préférence $X_4$ est un radical organique aliphatique pentavalent ;
$X_5$ est un radical organique aliphatique hexavalent ou un radical organique aromatique hexavalent, de préférence $X_5$ est un radical organique aliphatique hexavalent ;
et dans lequel chacun des $X_1$ à $X_5$ est constitué d'un ensemble d'atomes liés de manière covalente en une configuration qui comprend - de préférence qui est constituée de celles-ci - des structures linéaires et/ou ramifiées et/ou cycliques, lequel ensemble d'atomes est choisi dans le groupe constitué par i) à x) : i) des atomes de

carbone et d'hydrogène, ii) des atomes de carbone, d'hydrogène et d'oxygène, iii) des atomes de carbone, d'hydrogène et d'azote, iv) des atomes de carbone, d'hydrogène et de soufre, v) des atomes de carbone, d'hydrogène, d'oxygène et d'azote, vi) des atomes de carbone, d'hydrogène, d'azote et de soufre, vii) des atomes de carbone, d'hydrogène, d'oxygène et de soufre, viii) des atomes de carbone, d'hydrogène, d'oxygène, d'azote et de soufre, ix) des atomes de carbone, d'hydrogène et de silicium et x) des atomes de carbone, d'hydrogène, d'oxygène et de silicium et xi) une quelconque combination de ix) et/ou x) avec l'un quelconque ou la totalité des iii) à viii),
et dans lequel chacun des $X_1$ à $X_5$ a des atomes de carbone et des atomes d'hydrogène,
et dans lequel chacun des $X_1$ à $X_5$ a éventuellement des atomes d'oxygène et/ou des atomes d'azote et/ou des atomes de soufre et/ou des atomes de silicium,
et dans lequel le $X_1$ et/ou le $X_2$ et/ou le $X_3$ et/ou le $X_4$ et/ou le $X_5$ peuvent éventuellement comprendre un groupe fonctionnel ionique,
dans lequel
Y est un radical organique monovalent choisi dans le groupe constitué par : i) un radical organique aziridinylhydroxyisopropyle monovalent de formule B1, ii) un radical organique d'aziridinylhydroxycyclohexane monovalent de formule B2 et iii) un radical organique d'aziridinylhydroxycyclohexane monovalent de formule B3, de préférence Y est un radical organique aziridinylhydroxyisopropyle monovalent de formule B1,

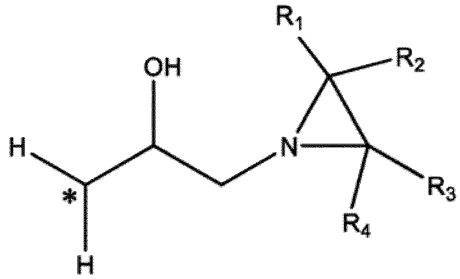

Formule B1

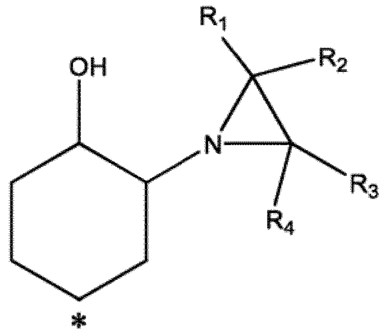

Formule B2

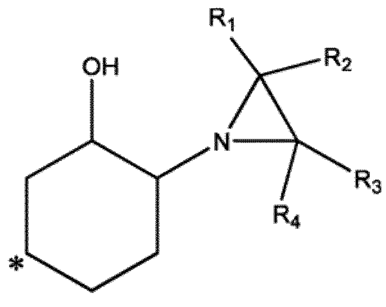

Formule B3

et

$R_1$ étant choisi dans le groupe constitué par l'atome d'hydrogène et un groupe méthyle ; et
$R_2$ étant choisi dans le groupe constitué par l'atome d'hydrogène, un groupe méthyle et un groupe alkyle en $C_2$-$C_5$ ; et
$R_3$ étant choisi dans le groupe constitué par les groupes méthyle et alkyle en $C_2$-$C_4$ ; et
$R_4$ étant choisi dans le groupe constitué par l'atome d'hydrogène, un groupe méthyle et un groupe alkyle en $C_2$-$C_4$ ;

et dans lequel
les Y dans chacun des composés A1 à A5 peuvent être identiques ou différents les uns des autres et dans lequel chacune des liaisons covalentes simples entre le Y et chacun des $X_1$ à $X_5$ est choisie dans le groupe constitué par une liaison simple carbone-carbone et une liaison simple carbone-oxygène, de préférence chacune des liaisons covalentes simples entre le Y et chacun des $X_1$ à $X_5$ est une liaison simple carbone-oxygène,
et dans lequel
chacun des composés AZ1 à AZ5 a une masse moléculaire déterminée par le biais de MALDI-TOF MS selon la description, d'au moins 600 et d'au maximum 10000, de préférence d'au moins 600 et d'au maximum 8000, plus préférablement d'au moins 600 et d'au maximum 6000, le plus préférablement d'au moins 600 et d'au maximum 5000, en particulier d'au moins 600 et d'au maximum 4000, plus particulièrement d'au moins 600 et d'au maximum 3500, le plus particulièrement d'au moins 600 et d'au maximum 3200, par exemple d'au moins 600 et d'au maximum 3000, par exemple d'au moins 600 et d'au maximum 2500 Da,
et dans lequel
le $X_1$ et le $X_2$ et le $X_3$ et le $X_4$ et le $X_5$ ne contiennent pas l'une quelconque ou une quelconque combinaison des unités de structure BP1, BP2, BP3 et BS suivantes

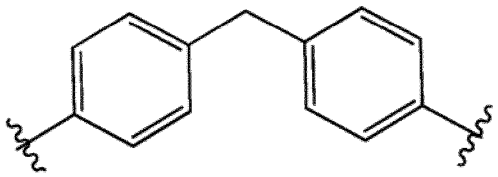

unité BP1

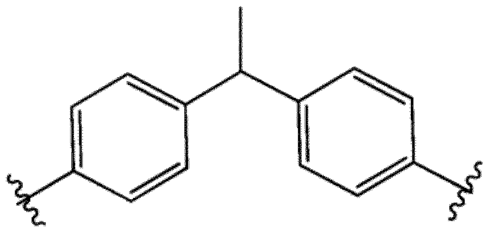

unité BP2

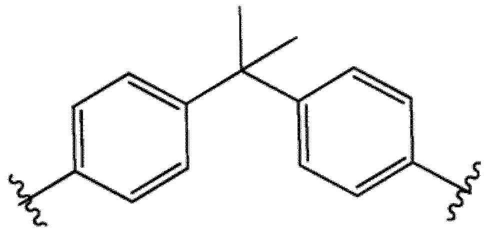

unité BP3

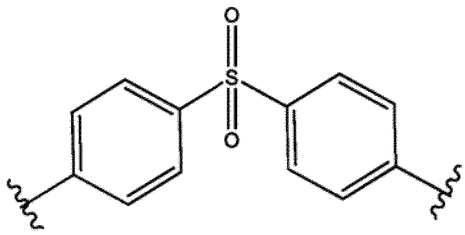

unité BS.

2. Composant AZ selon la revendication 1, dans lequel le $X_1$ et/ou le $X_2$ et/ou le $X_3$ et/ou le $X_4$ et/ou le $X_5$ comprennent au moins une unité de structure ou une combinaison d'unités de structure choisies dans le groupe constitué par l'unité 1, l'unité 2, l'unité 3, l'unité 4, l'unité 5, l'unité 6, l'unité 7, l'unité 8, l'unité 9, l'unité 10 et l'unité 11, les unités 1 à 11 étant représentées ci-dessous :

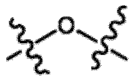

unité 1

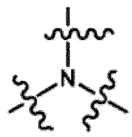

unité 2

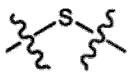

unité 3

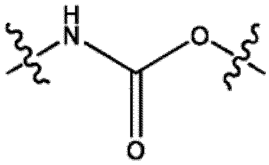

unité 4

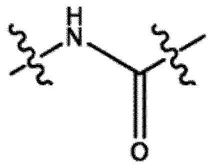

unité 5

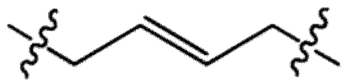

unité 6

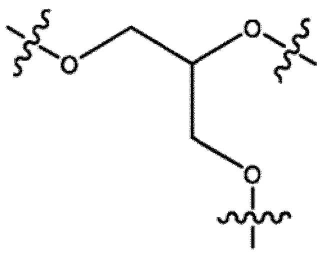

unité 7

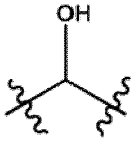

unité 8

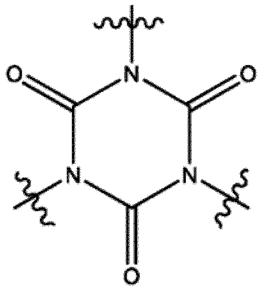

unité 9

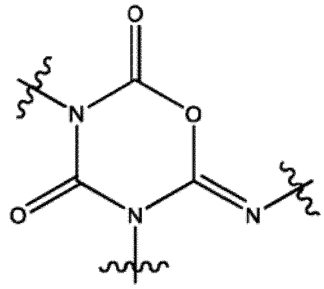

unité 10

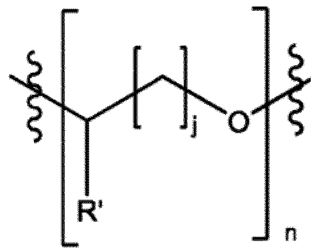

unité 11,

R' étant choisi dans le groupe constitué par l'atome d'hydrogène et un groupe méthyle ; et j étant un nombre entier allant de 1 à 5, de préférence de 1 à 3 ; et

n étant un nombre entier allant de 2 inclus jusqu'à 50 inclus.

**3.** Composant AZ selon l'une quelconque des revendications précédentes, dans lequel le nombre d'agrégats d'atomes de carbone dans $R_1$ et $R_2$ et $R_3$ et $R_4$ est d'au maximum 9, de préférence d'au maximum 4, plus préférablement d'au maximum 2, par exemple d'au maximum 1.

**4.** Composant AZ selon l'une quelconque des revendications précédentes, dans lequel le $X_1$ est le radical organique aliphatique divalent de formule A1a'

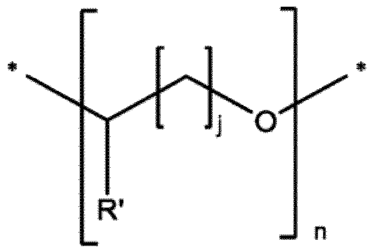

Formule A1a′

R' étant choisi dans le groupe constitué par l'atome d'hydrogène et un groupe méthyle ; et j étant un nombre entier allant de 1 à 5, de préférence de 1 à 3 ; et

n étant un nombre entier allant de 2 inclus jusqu'à 50 inclus.

**5.** Composant AZ selon l'une quelconque des revendications 1 à 4 dans lequel chacun des $X_1$ à $X_5$ contient uniquement des liaisons covalentes simples, ou à la fois des liaisons covalentes simples et doubles, et les liaisons covalentes simples étant choisies dans le groupe constitué par une liaison simple carbone-carbone, une liaison simple carbone-hydrogène, une liaison simple carbone-azote, une liaison simple carbone-soufre, une liaison simple carbone-silicium, une liaison simple silicium-oxygène, une liaison simple azote-hydrogène, une liaison simple soufre-oxygène, une liaison simple carbone-oxygène, l'atome d'oxygène étant lié à un atome d'hydrogène formant un groupe hydroxyle, les liaisons simples silicium-oxygène-silicium, et les doubles liaisons covalentes étant choisies dans le groupe constitué par une double liaison carbone-carbone, une double liaison carbone-azote, une double liaison soufre-oxygène, une double liaison carbone-oxygène, l'atome de carbone étant un élément d'une structure cyclique, et une double liaison carbone-oxygène, l'atome de carbone étant lié à deux autres atomes de carbone par le biais de liaisons simples carbone-carbone, une double liaison carbone-oxygène, l'atome de carbone étant lié à un autre atome d'oxygène par le biais d'une liaison simple et à un atome d'azote par le biais d'une liaison simple, une double liaison carbone-oxygène, l'atome de carbone étant lié à deux atomes d'azote par le biais de liaisons simples carbone-azote, une double liaison carbone-oxygène, l'atome de carbone étant lié à deux autres atomes d'oxygène par le biais de liaisons simples ; de préférence les doubles liaisons covalentes étant choisies dans le groupe constitué par une double liaison carbone-carbone, une double liaison carbone-azote, une double liaison soufre-oxygène, une double liaison carbone-oxygène, l'atome de carbone étant un élément d'une structure cyclique, et une double liaison carbone-oxygène, l'atome de carbone étant lié à deux autres atomes de carbone par le biais de liaisons simples carbone-carbone, une double liaison carbone-oxygène, l'atome de carbone étant lié à un autre atome d'oxygène par le biais d'une liaison simple et à un atome d'azote par le biais d'une liaison simple, une double liaison carbone-oxygène, l'atome de carbone étant lié à deux atomes d'azote par le biais de liaisons simples carbone-azote.

**6.** Composant AZ selon l'une quelconque des revendications 1-3 et 5 précédentes, le composant AZ étant choisi dans le groupe constitué par i) à v): i) un composé organique à fonction aziridinyle et hydroxy AZ1 de formule A1 (composé AZ1), ii) un composé organique à fonction aziridinyle et hydroxy AZ2 de formule A2 (composé AZ2), iii) un composé organique à fonction aziridinyle et hydroxy AZ3 de formule A3 (composé AZ3), iv) un composé organique à fonction aziridinyle et hydroxy AZ5 de formule A5 (composé AZ5) et vi) les mélanges de ceux-ci, et

le composé AZ1 étant choisi dans le groupe constitué par les composés répondant à la formule A1a et les composés répondant à la formule A1b, chacune de ces formules A1a-A1d étant décrite ci-dessous

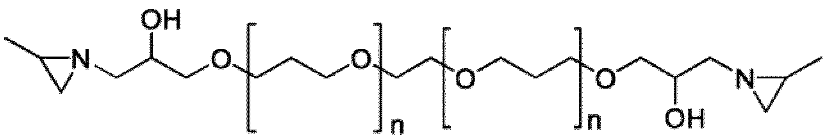

Formule A1a

chacun des n dans la formule A1a étant indépendamment choisi et chacun des n dans la formule A1a étant un nombre entier allant de 2 inclus jusqu'à 20 inclus ; et

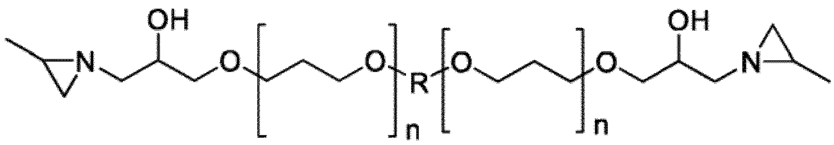

Formule A1b

le R dans la formule A1b étant un groupe hydrocarbylène en $C_3$-$C_{10}$ saturé et chacun des n dans la formule A1b étant indépendamment choisi et chacun des n dans la formule A1a étant un nombre entier allant de 2 inclus jusqu'à 20 inclus ; et

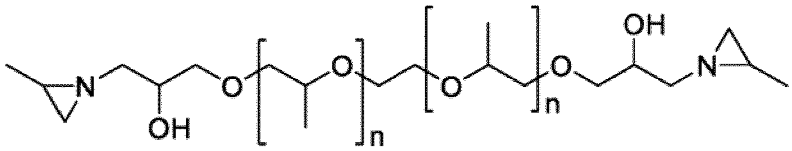

Formule A1c

chacun des n dans la formule A1c étant indépendamment choisi et chacun des n dans la formule A1b étant un nombre entier allant de 2 inclus jusqu'à 20 inclus ; et

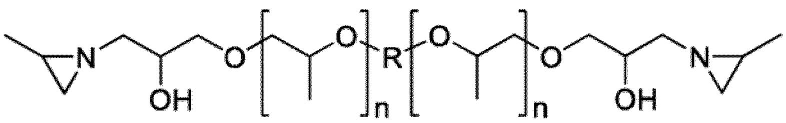

Formule A1d

le R dans la formule A1d étant un groupe hydrocarbylène en $C_3$-$C_{10}$ saturé et chacun des n dans la formule A1d étant indépendamment choisi et chacun des n dans la formule A1b étant un nombre entier allant de 2 inclus jusqu'à 20 inclus ;
et
le composé AZ2 étant choisi dans le groupe constitué par les composés répondant à la formule A2a, les composés répondant à la formule A2b, les composés répondant à la formule A2c, les composés répondant à la formule A2d, les composés répondant à la formule A2e, chacune de ces formules A2a-A2e étant décrite ci-dessous

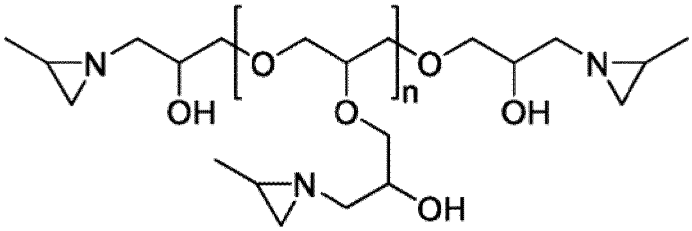

Formule A2a

n dans la formule A2a étant un nombre entier allant de 2 inclus jusqu'à 20 inclus ; et

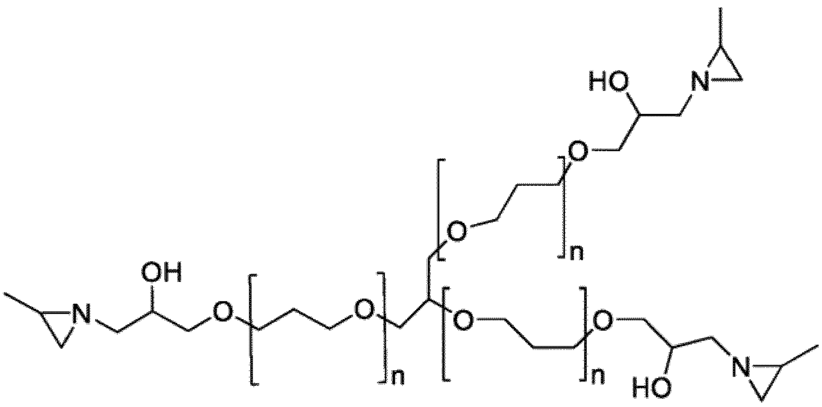

Formule A2b

chacun des n dans la formule A2b étant indépendamment choisi et chacun des n dans la formule A2b étant un nombre entier allant de 2 inclus jusqu'à 20 inclus ; et

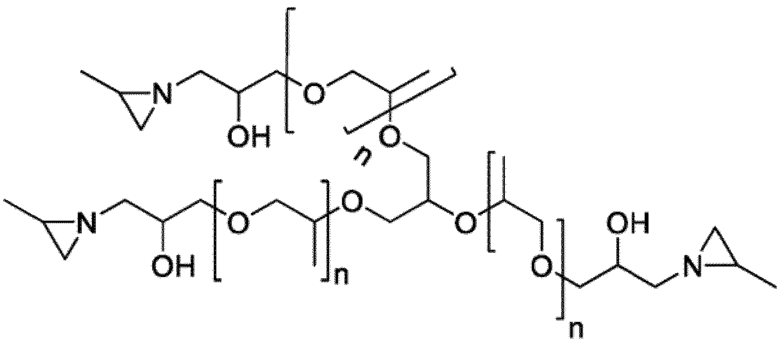

Formule A2c

chacun des n dans la formule A2c étant indépendamment choisi et chacun des n dans la formule A2c étant un nombre entier allant de 2 inclus jusqu'à 20 inclus ; et

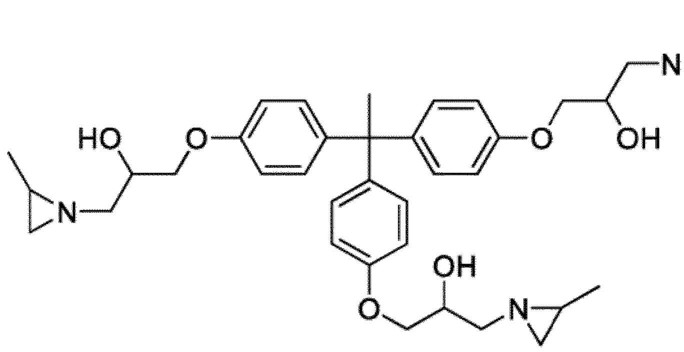

Formule A2d

et

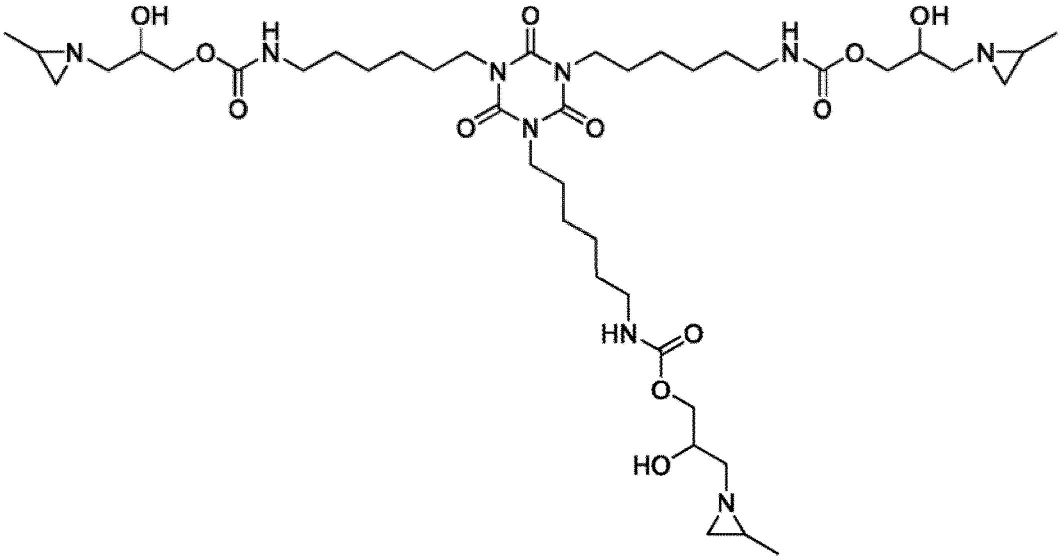

Formule A2e

et

le composé AZ3 étant choisi dans le groupe constitué par les composés répondant à la formule A3a

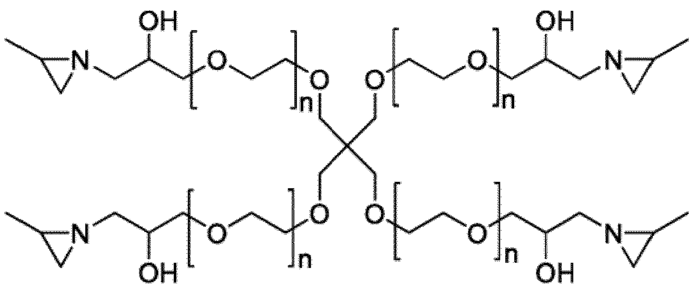

Formule A3a

chacun des n dans la formule A3a étant indépendamment choisi et chacun des n dans la formule A3a étant un nombre entier allant de 2 inclus jusqu'à 20 inclus ;

et

le composé AZ5 étant choisi dans le groupe constitué par les composés répondant à la formule A5a

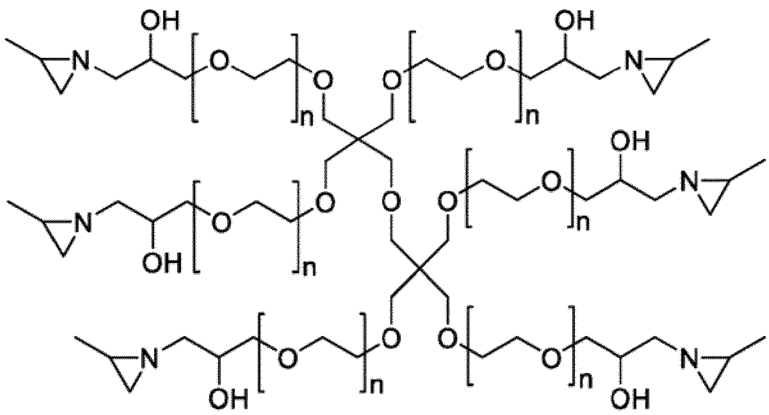

Formule A5a

chacun des n dans la formule A5a étant indépendamment choisi et chacun des n dans la formule A5a étant un nombre entier allant de 2 inclus jusqu'à 40 inclus.

7. Composant AZ selon l'une quelconque des revendications 1-3 et 5 précédentes, le composant AZ étant choisi dans le groupe constitué par un composé AZ1 et le composé AZ1 étant le composé de formule suivante

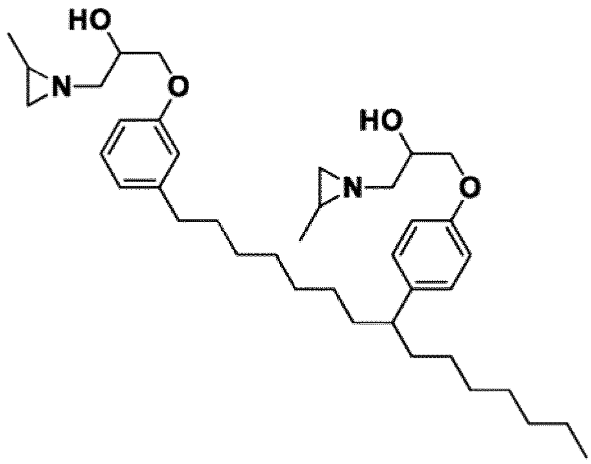

.

8. Composant AZ selon l'une quelconque des revendications 1-3 et 5 précédentes, le composant AZ étant choisi dans le groupe constitué par un composé AZ2 et le composé AZ2 étant le composé de formule suivante

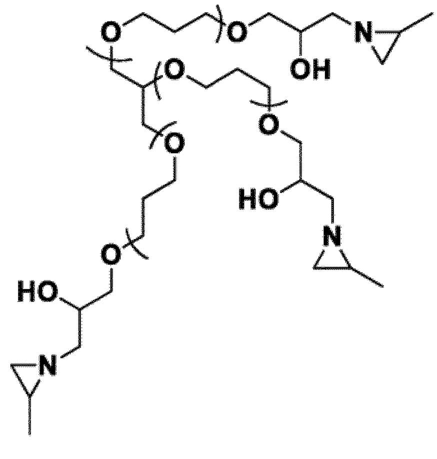

.

**9.** Composant AZ selon l'une quelconque des revendications 1-3 et 5 précédentes, le composant AZ étant choisi dans le groupe constitué par un composé AZ2 et le composé AZ2 étant le composé de formule suivante

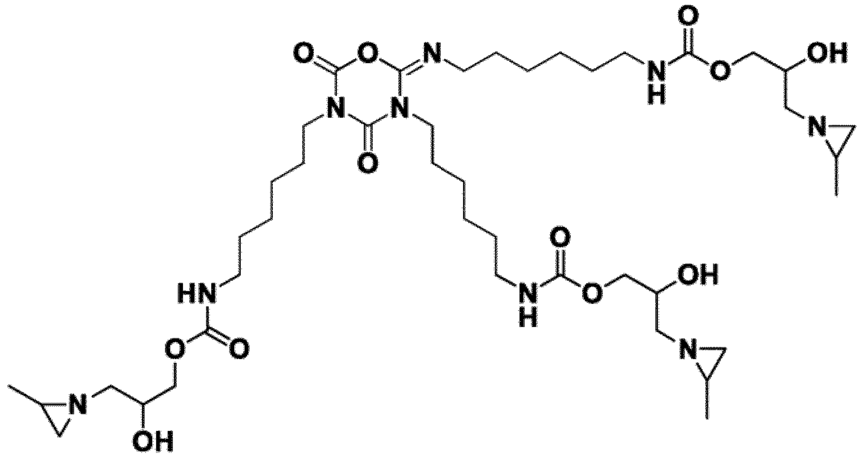

.

**10.** Composant AZ selon l'une quelconque des revendications 1-3 et 5 précédentes, le composant AZ étant choisi dans le groupe constitué par un composé AZ2 et le composé AZ2 étant le composé de formule suivante

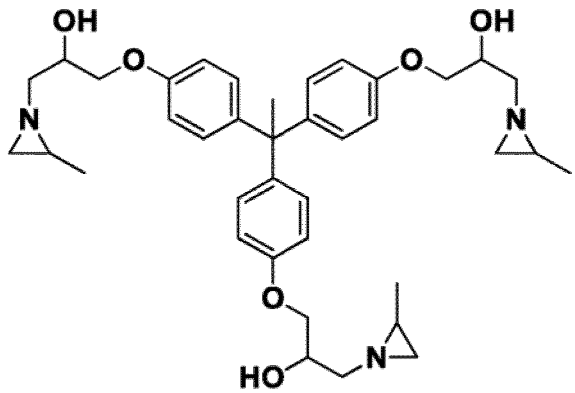

.

**11.** Composant AZ selon l'une quelconque des revendications 1-3 et 5 précédentes, le composant AZ étant choisi dans le groupe constitué par un composé AZ1, un composé AZ2 et les mélanges de ceux-ci et le composé AZ1 étant le composé de formule suivant

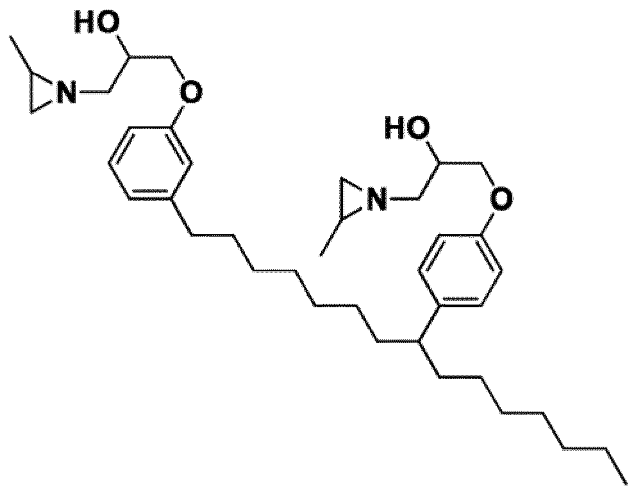

et le composé AZ2 étant choisi dans le groupe constitué par les composés de formule suivante

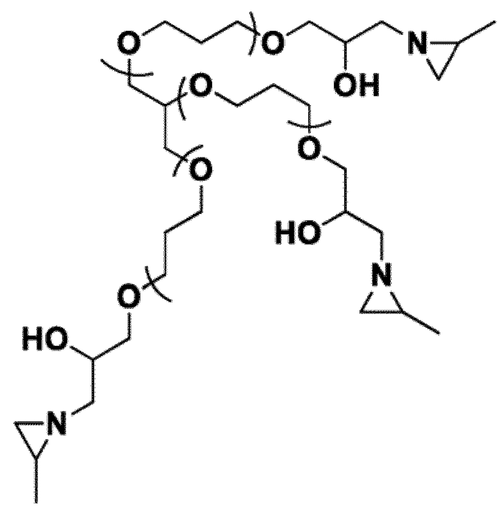

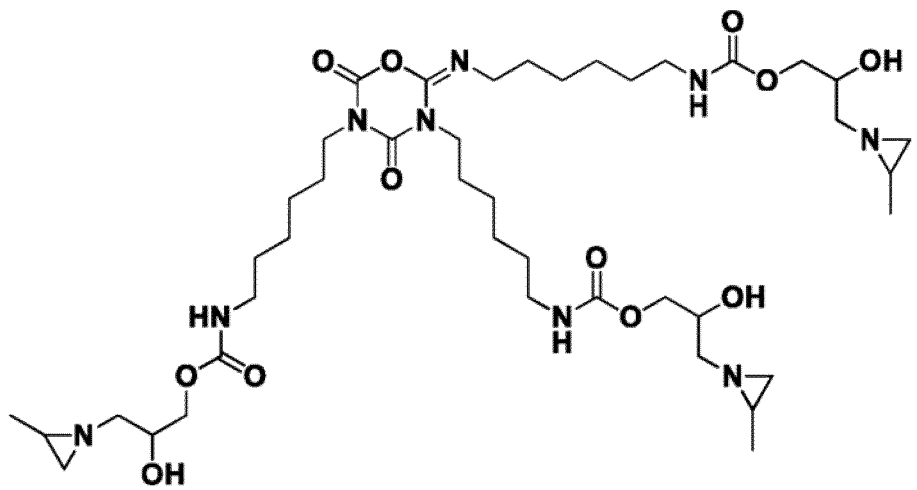

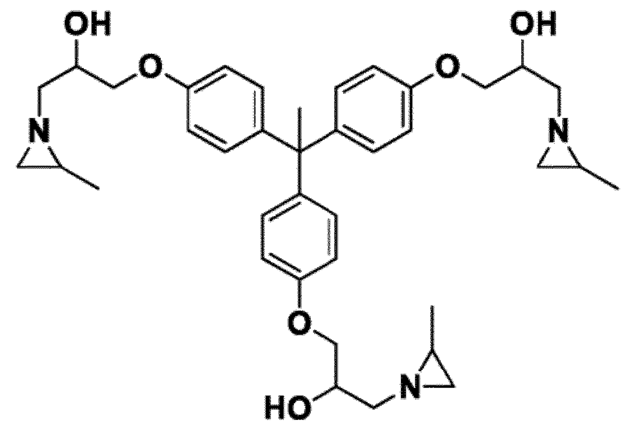

.

**12.** Composition liquide, comprenant :

i) un milieu liquide qui est choisi dans le groupe constitué par les solvants organiques, l'eau et un mélange de ceux-ci, en une quantité d'au maximum 90 % en poids par rapport au poids total de la composition liquide ; et
ii) un composant AZ selon l'une quelconque des revendications 1-11 ; et

la quantité totale de tous les composants qui constituent la composition liquide totalisant 100 % en poids.

**13.** Composition liquide selon la revendication 12, la composition liquide ayant un pH déterminé selon la norme ISO 976:2013 et selon la description, d'au moins 7,5 et d'au maximum 14,0, à condition que la composition liquide comprenne de l'eau en une quantité d'au moins 20, de préférence d'au moins 25, plus préférablement d'au moins 30, le plus préférablement d'au moins 35 % en poids par rapport au poids total de la composition liquide.

**14.** Composition liquide selon l'une quelconque des revendications 12-13, la composition liquide comprenant un composant T choisi dans le groupe constitué par : i) les composés organiques ayant une masse moléculaire déterminée par le biais de MALDI-TOF MS selon la description, inférieure à 600 Da et comprenant au moins un cycle aziridine et ii) les mélanges de ceux-ci, en une quantité déterminée par le biais d'une chromatographie en phase liquide couplée à la spectroscopie de masse (LC-MS) selon la description, d'au maximum 5, de préférence d'au maximum 0,5, par exemple d'au maximum 0,1, par exemple d'au maximum 0,05 % en poids par rapport au poids total du composé AZ.

**15.** Composition liquide selon l'une quelconque des revendications 12-14, comprenant outre :
iii) un polymère qui a un indice d'acide déterminé selon la norme ASTM D1639-90(1996)e1 d'au moins 5 et d'au maximum 300 mg de KOH/g et le polymère pouvant éventuellement comprendre des groupes fonctionnels ioniques.

**16.** Kit en plusieurs parties comprenant les parties A et B qui sont physiquement séparées l'une de l'autre, dans lequel :

i) la partie A comprend une composition liquide selon l'une quelconque des revendications 12-15 et
ii) la partie B comprend un polymère qui a un indice d'acide déterminé selon la norme ASTM D1639-90 (1996) e1 dans la plage allant de 5 à 300, de préférence de 8 à 200, plus préférablement de 10 à 150 mg de KOH/g et le polymère pouvant éventuellement comprendre des groupes fonctionnels ioniques, et

dans lequel la partie A ne comprend pas le polymère de la partie B et la partie B ne comprend pas la composition liquide de la partie A.

**17.** Forme durcie d'un composant AZ selon l'une quelconque des revendications 1-11 ou d'une composition liquide selon l'une quelconque des revendications 12-15.

**18.** Article comprenant : i) un composant AZ selon l'une quelconque des revendications 1-11 ou ii) une composition liquide selon l'une quelconque des revendications 12-15 et/ou iii) une forme durcie selon la revendication 17.

**19.** Utilisation de l'un quelconque ou d'une quelconque combinaison des suivants :

i) un composant AZ selon l'une quelconque des revendications 1-11 ;
ii) une composition liquide selon l'une quelconque des revendications 12-15 ;
iii) un kit en plusieurs parties selon la revendication 16 ;
iv) une forme durcie selon la revendication 17 ;
v) un article selon la revendication 18 ;

dans des revêtements, des peintures, des encres, des vernis, des lubrifiants, des adhésifs, la fabrication additive, l'impression 3D, des textiles, des cires, des combustibles, la photographie, des plastiques, des dispositifs médicaux et dans la préparation de compositions médicales.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2007298006 A1
- WO 2006115547 A2
- US 3329674 A
- EP 1865014 A1
- WO 2015066868 A1
- EP 0758662 A2
- US 9695481 B2
- EP 2616484 B1


### Non-patent literature cited in the description


- *CHEMICAL ABSTRACTS,* 64265-57-2
- *CHEMICAL ABSTRACTS,* 57116-45-7
- *CHEMICAL ABSTRACTS,* 63738-22-7
- *CHEMICAL ABSTRACTS,* 87093-13-8
- *CHEMICAL ABSTRACTS,* 1675-54-3
- *CHEMICAL ABSTRACTS,* 5026-74-4
- *CHEMICAL ABSTRACTS,* 300364-84-5
- *CHEMICAL ABSTRACTS,* 2923-16-2
- **HENDRIKS et al.** *Toxicol. Sci.,* 2015, vol. 150, 190-203